# EUROPEAN PATENT APPLICATION

(11) **EP 1 946 757 A1**
(43) Date of publication of application: **23.07.2008**
(21) Application number: 06811953.6
(22) Date of filing: 18.10.2006
(51) Int. Cl.: A61K 31/36, A61K 31/09, A61K 36/00, A61K 35/00, A61P 43/00, C07D 317/70

(54) **ATR INHIBITOR**

(30) Priority: 21.10.2005 JP 2005307534
(71) Applicant: Niigata TLO Corporation, Niigata-shi, Niigata 950-2181 (JP)
(72) Inventor: NISHIDA, Hiroshi, Niigata Uni. of Pharmacy and Applied Life Sciences, Niigata-shi Niigata 956-8603 (JP); HAMAMORI, Yasuo, Baylor College of Medicine, Houston Texas 77030 (US); KONISHI, T., Niigata Uni. of Pharmacy and Applied Life Sciences, Niigata-shi Niigata 956-8603 (JP)
(74) Representative: Bufton, Karen A.J.
(86) International application number: PCT/JP2006/320756
(87) International publication number: WO 2007/046426

(57) **Abstract**

The present invention is to provide ATR inhibitor containing tricyclic compound including Schizandrins and Gomisins as an active constituent, useful for an inhibitor of ATR protein kinase.

## Description

### Field of Invention

The present invention relates to an ATR inhibitor to inhibit the activity of ATR (ataxia-telangiectasia mutated and rad3-related), more particulary, an ATR inhibitor of a tricyclic compound as an active constituent.

### Related Art

It is known that ATM (ataxia-telangiectasia mutated) and ATR(ataxia-telangiectasia mutated and rad3-related) protein kinase (hereinafter, referred also to as "ATM" and "ATR") are playing a crucial role in cellular DNA damage (Non-Patent-Related Document 1). Ultra violet (UV) radiation and ionizing radiation (IR) induce DNA damage and exert the checkpoint signal cascades followed by cell cycle arrest as G1/S, intra-S and G2/M checkpoint in order to repair the DNA damage, or squeeze the cells into apoptosis. The cells derived from AT patient, ATM deficient, are hypersensitive to IR because it exhibits the phenotype of G1/S and G2/M checkpoint abrogation. Therefore, ATM and its downstream pathways are important for the maintenance of chromatin. Moreover, transient or stable deficiency of ATR causes to prominent instability of chromosome or early embryonic lethal (Non-Patent-Related Document 2). Interestingly, it is known that the phosphorylation of p53 protein (hereinafter, referred to as "p53") or NBS1 with ATR delays in the absence of ATM. It suggests that ATR is a cooperative partner of ATM in terms of DNA damage response. Indeed, ATR is playing a crucial role for protecting from DNA damage. These are also indicating that the regulation of ATM or ATR protein activities is not only required for the maintenance of genome stability, but also applicable for anti-cancer therapy such as chemotherapy and radiation therapy.

Non-Patent-Related Document 3 discloses that caffeine (3,7-dihydro-1,3,7-trimethyl-1H-purine-2,6-dione)-assisted chemotherapy minimizes tumber excision for non-metastatic osteosarcoma. It is perceived that caffeine inhibits ATM and ATR protein kinase activities in the response of DNA damage induced by chemotherapy. It is also known that caffeine inhibits the activity of ATM rather than that of ATR.

Critical roles of ATR and ATM have been investigated in recent studies such as the transfection siRNA into the cell or overexpression of a protein lacking the kinase active site of the recombinant kinase in the cell (Non-Patent-Related Documents 4 to 6). However, the less are reported the function of ATR in comparison with that of ATM because the stable knockout mice of ATR are lethal. In addition, in the current situation of specific inhibitors for ATR being unknown, the inhibition of ATM and ATR protein kinase activity by chemicals are widely investigated by using caffeine and wortmannin, an inhibitor of PI3 kinase. Therefore, the discovery of specific inhibitor for ATR will lead to biochemical knowledge in the DNA damage response and to many advantages to the clinical study of anti-cancer therapy. Therefore, it is desired to obtain compounds to inhibit ATR activities.

Among those, ATM and ATR phosphorylate p53 at Serine 15 during the response to the DNA damage in the cells. p53 plays a pivotal role in the G1/S checkpoint induced by DNA damage. Recent findings indicate that the serine at 15 of p53 is directly phosphorylated by ATR or ATM to arrest the cell cycle at G1 phase during the exposure of UV light. Thus, the phosphorylation of serine 15 in p53 is the reasonable index to estimate G1/S checkpoint function originated from ATM or ATR after DNA damage is induced by UV. In addition, such a phosphorylation reflects ATR activity in the damage response toward UV. As well as G1/S checkpoint, G2/M checkpoint is regulated by both ATR and ATM in DNA damaged cells. G2/M checkpoint is very important and well conserved in most of cancer cells either in p53 deficient or mutated tumors. Notably, the cells lacking p53 function were also sensitive to DNA damage by the abrogation of G2/M checkpoint. Indeed, ATR related G2/M checkpoint is essential for genome maintenance of proliferating cells. These results suggest that inhibition of the whole bunch related G2/M checkpoint wherein such an inhibition includes the inhibition of ATR protein kinase activity will be a candidate for the anti-cancer therapy.

On the other hand, Schizandrins and Gomisins are dibenzocyclooctadine derivatives derived from Schisandrae Fructus, and are compounds commonly used as Chinese medicine. As one of efficacy of these compounds, Non-Patent-Related Document 8 discloses that Schizandrin B induces caspase-3 dependent apoptosis in SMMC-7221 hepatoma cells. Now, other signal modulation or the other effects on various types of cells including tumors by this compound are remaining for further study. It is naturally believed that there are many possibilities in Schisandrae Fructus traditional Chinese medicine, including Schizandrins and Gomisins, based on the thousands year experiences. Over 70% of anti-cancer drugs are derived from natural products or its mimics with expectation of fewer side effects. We sought to apply Schizandrins and Gomisins on the signal transduction pathway of DNA damage response in terms of sensitizer of anti-cancer therapy.
Patent Document 1
Japanese Patent Application Publication No. 206751/1995 Patent Document 2
Japanese Patent Application Publication No. 192133/1994 Patent Document 3
Japanese Patent Application Publication No. 048592/1990 Patent Document 4
Japanese Patent Application Publication No. 123184/1993 Patent Document 5
US Application Publication No. 2005/0282910 Non-Patent-Related Document 1
Shiloh Y. et al., "ATM and ATR: networking cellular responses to DNA damage", Curr. Opin. Genet. Dev., 2001, vol. 11, p.71-7
Non-Patent-Related Document 2
Cortez D. et al., "ATR and ATRIP: partners in checkpoint signaling", Science, 2001, vol. 294, p.1713-6
Non-Patent-Related Document 3
Tsuchiya H. et al., "Caffeine-assisted chemotherapy and minimized tumor excision for nonmetastatic osteosarcoma.", Anticancer Res., 1998, vol. 18, p.657-66
Non-Patent-Related Document 4
Shackelford R. E. et al., "The Ataxia telangiectasia gene product is required for oxidative stress-induced G1 and G2 checkpoint function in human fibroblasts.", J. Biol. Chem., 2001, vol. 276, p.21951-9
Non-Patent-Related Document 5
Wright J. A. et al., "Protein kinase mutants of human ATR increase sensitivity to UV and ionizing radiation and abrogate cell cycle checkpoint control.", Proc. Natl. Acad. Sci. USA., 1998, vol. 95, p.7445-50
Non-Patent-Related Document 6
Shigeta T. et al., "Defective control of apoptosis and mitotic spindle checkpoint in heterozygous carriers of ATM mutations.", Cancer Res., 1999, vol. 59, p.2602-7 Non-Patent-Related Document 7
Sarkaria J. N. et al., "Inhibition of ATM and ATR kinase activities by the radiosensitizing agent, caffeine.", Cancer Res., 1999, vol. 59, p.4375-82
Non-Patent-Related Document 8
Wu Y. F. et al., "Down-modulation of heat shock protein 70 and up-modulation of Caspase-3 during schisandrin B-induced apoptosis in human hepatoma SMMC-7221 cells", World J. Gastroenterol., 2004, vol. 10, p.2944-48
Non-Patent-Related Document 9
Tibbetts et al., Gene & Development, 2000, vol. 14, p.2989-3002
Non-Patent-Related Document 10
Canman et al., Science, 1998, vol. 281, p.1677-9 Non-Patent-Related Document 11
Rouet et al., Proc. Natl. Acad. Sci. USA, 1994, vol.91, p.6064-8
Non-Patent-Related Document 12
Chem. Pharm. Bull., 2003, vol. 51, No. 11, p.1233-1236 Non-Patent-Related Document 13
Acta Pharmacologica Sinica, 1998, vol. 19, No. 4, p.313-316

### Disclosure of Invention

### Problems to be solved by the Invention

Hence, the present invention is to provide ATR inhibitor containing tricyclic compound including Schizandrins and Gomisins as an active constituent, useful for an inhibitor of ATR protein kinase.

### Means to solve the problems

The ATR inhibitor according to the present invention is characterized in that:
ATR inhibitor containing a compound having a following formula (1) as an active constituent.
   [ka11]

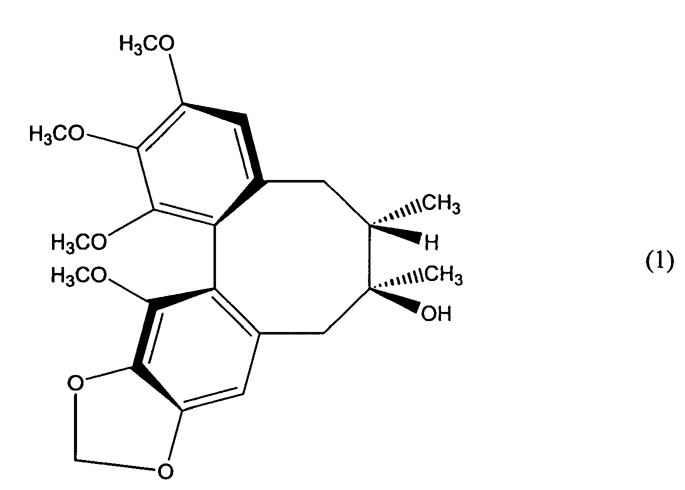

The ATR inhibitor according to the present invention is characterized in that:
ATR inhibitor containing a compound having a following formula (2) as an active constituent.
   [ka12]

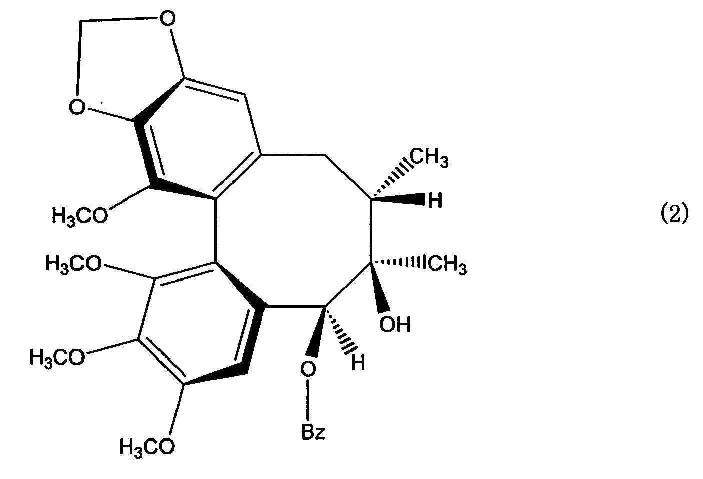

The ATR inhibitor according to the present invention is characterized in that:
ATR inhibitor containing a compound having a following formula (3) as an active constituent.
   [ka13]

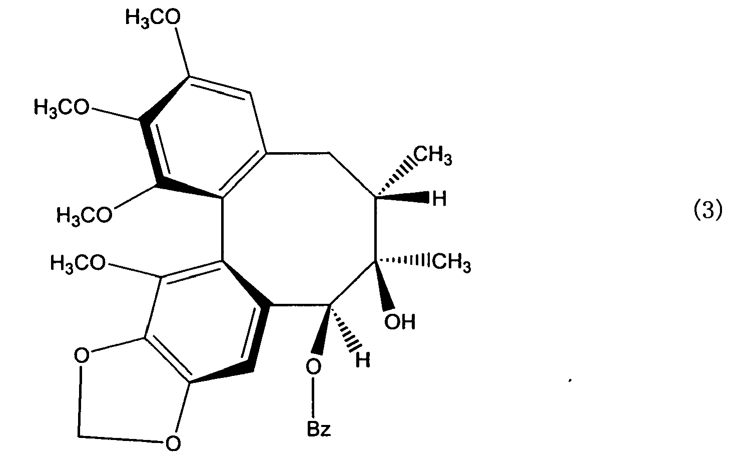

The ATR inhibitor according to the present invention is characterized that:
ATR inhibitor containing a compound having a following formula (4) as an active constituent.
   [ka14]

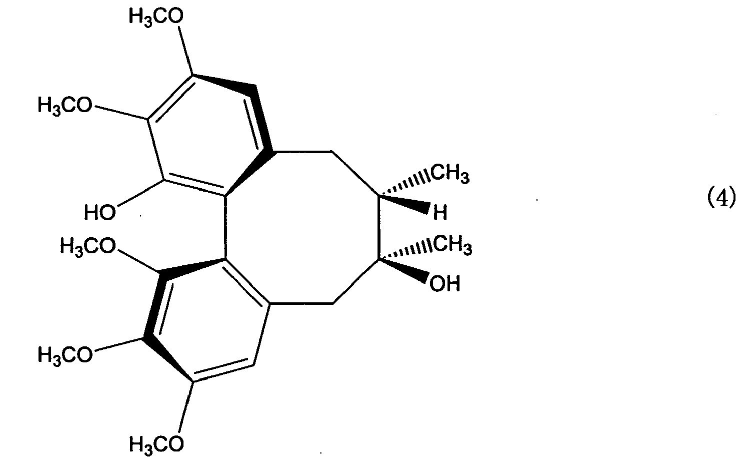

The ATR inhibitor according to the present invention is characterized in that:
ATR inhibitor containing a compound having a following formula (5) as an active constituent.
   [ka15]

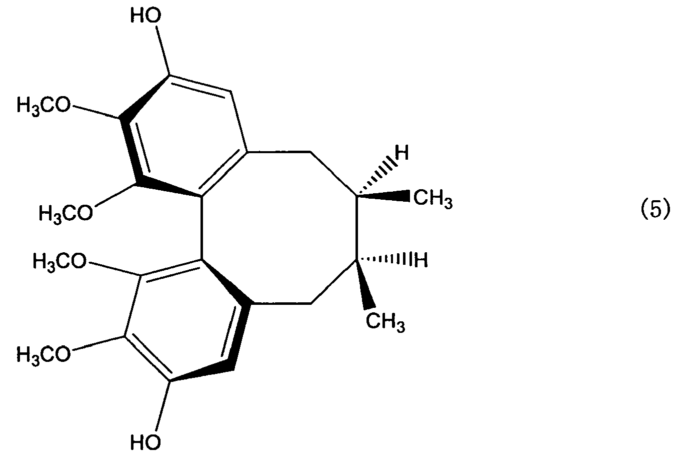

The ATR inhibitor according to the present invention is characterized in that:
ATR inhibitor containing a compound having a following formula (6) as an active constituent.
   [ka16]

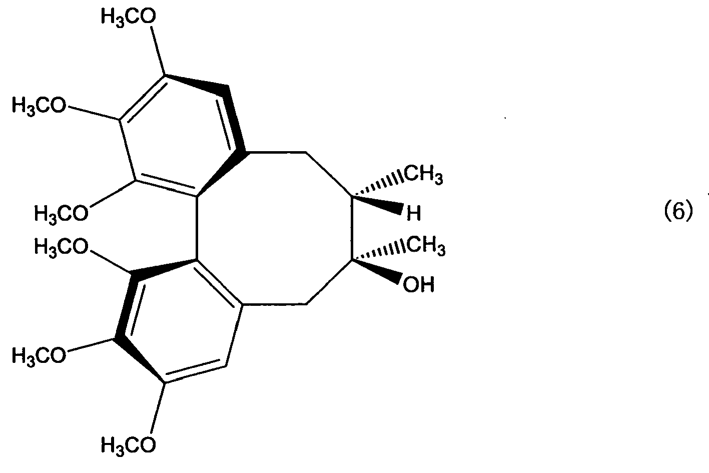

The ATR inhibitor according to the present invention is characterized in that:
ATR inhibitor containing a compound having a following formula (7) as an active constituent.
   [ka17]

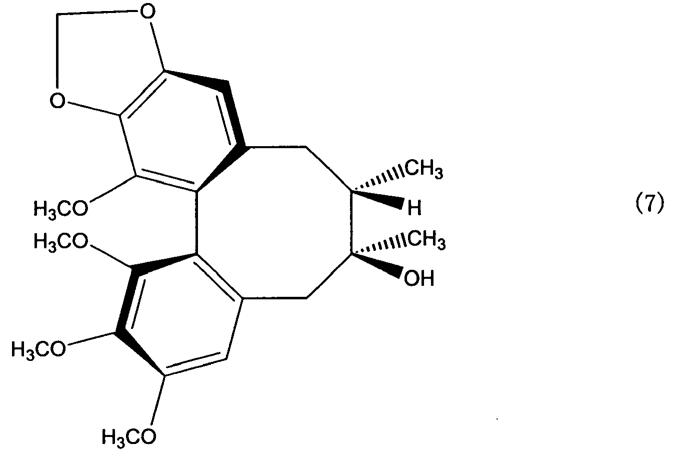

The ATR inhibitor according to the present invention is characterized in that:
ATR inhibitor containing a compound having a following formula (8) as an active constituent.
   [ka18]

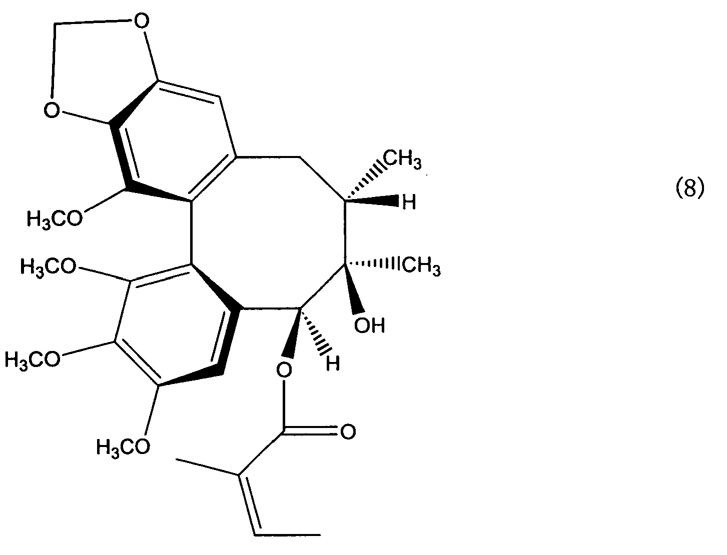

The ATR inhibitor according to the present invention is characterized in that:
ATR inhibitor containing a compound having a following formula (9) as an active constituent.
   [ka19]

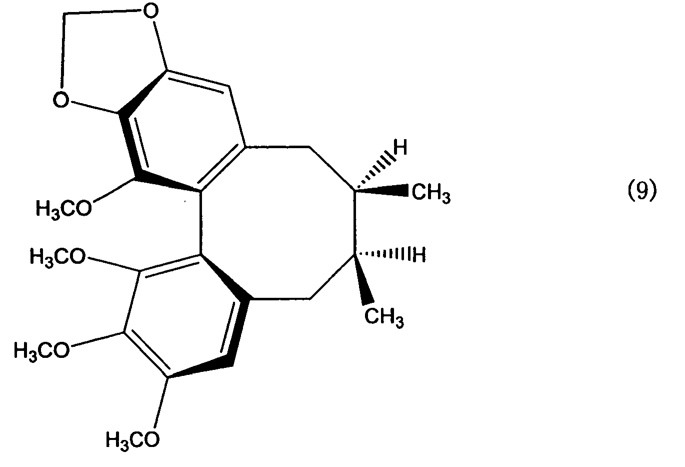

The ATR inhibitor according to the present invention is characterized in that:
ATR inhibitor containing a compound having a following formula (10) as an active constituent.
   [ka20]

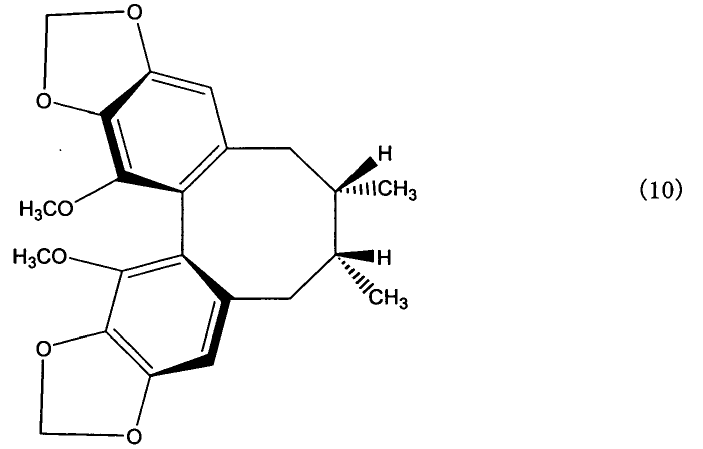

Among these, it is preferable that the ATR inhibitor according to the present invention contains compounds of the Formulas (1) to (5) as an active constituent.

. In the ATR inhibitor according to the present invention, it is preferable that the ATR activity is a protein kinase activity.

In the ATR inhibitor according to the present invention, it is preferable that the protein kinase activity is an activity of phosphorylating cell-cycle-related proteins.

In the ATR inhibitor according to the present invention, it is preferable that the cell-cycle-related proteins are p53.

In the ATR inhibitor according to the present invention, it is preferable that the cell-cycle-related proteins are selected from the group consisting of Brca1 and Chk1.

### Effect of Invention

According to the present invention, it is possible to specifically inhibit the ATR activity both of in vitro and in vivo.

### Brief explanation of Drawing

Figure 1 is a graph showing the effect of Schizandrin B on the surviving rate of A549 cells irradiated with UV;
Figure 2 is figures showing the effect of Schizandrin B on the cell cycle of A549 cells irradiated with UV, wherein (A) is a figure showing the content of the phosphorylated Histone H3 in the total cells, and (B) shows ratio of the dividing cells to the total cells based on the content of the phosphorylated histone H3;
Figure 3 is a graph showing the effect of Schizandrin B on the rate of dividing cells in A549 cells irradiated with ionizing radiation;
Figure 4 is a graph showing the effect of Schizandrin B on the phosphorylation of p53 in A549 cells irradiated with UV or ionizing radiation;
Figure 5-1 is a figure showing the dose-depended effect of Schizandrin B on the phosphorylation of p53 in A549 cells irradiated with UV;
Figure 5-2 is a figure showing the effect of ATR inhibitor according to the present invention on the phosphorylation of p53 in A549 cells irradiated with UV;
Figure 6 is a figure showing the effect of Schizandrin B on the cell cycle of A549 cells;
Figure 7 is a graph showing the effect of Schizandrin B on the protein kinase activities of ATR and ATM, wherein (A) corresponds to ATR, and (B) corresponds to ATM;
Figure 8 is a figure showing the effect of Schizandrin B on the phosphorylation of proteins in AT2KY cells irradiated with UV;
Figure 9 is a figure showing the effect of Schizandrin B on the phosphorylation of proteins in A549 cells irradiated with UV, wherein the expression of ATR or ATM is inhibited in the cells;
Figure 10 is a figure showing the effect of Schizandrin B on the phosphorylation of MAPK in A549 cells.

### Best Mode for carrying out the Invention

The ATR inhibitor according to the present invention can be compounds commercially available including synthetic compounds and natural compounds, and can be manufactured through the well-known synthetic method as disclosed in, e.g. Patent Document 1. In addition, it can be extracted and/or purified from the natural Chinese medicine including Fructus Schisandrae originated from Schisandra Chinensis according to the well-known method as disclosed in, e.g. Patent Document 2.

In particular, Schizandrin B (SchB) of formula (1) can be manufactured according to Patent Document 2. That is, Fructus Schisandrae is subjected to extraction with hydrocarbon solvent such as n-hexane or alcoholic solvent such as methanol under heat for a predetermined period. Then, the extract is applied on the column chromatography to obtain a solution containing a mixture of water-insoluble material and water-soluble material, and so obtained solution is concentrated to dryness, followed by adding a mixture of potassium hydroxide and methanol to saponify, thereby obtaining a raw material. Methanol is added to the raw material to solve under stir, followed by dissolving it in aqueous solution of potassium hydroxide. Then, the methanol is removed from obtained solution to precipitate the water-insoluble material of the target compound, thereby obtaining Schizandrin B.

In addition, Gomisin C (GC) can be manufactured according to the method as disclosed in Patent Document 3. That is, after the extraction with a solvent having low polarity such as petroleum ether, it is subjected to the partition extraction using water-insoluble solvent having low polarity such as petroleum ether and n-heptane, and water-soluble solvent having high polarity such as methanol, followed by, if necessary, to treatment such as column chromatography and high-performance liquid chromatography to obtain the target compound.

In addition, Gomisin G (GG) of the formula (3) can be manufactured from the dry stuck of Shizandra arisanensis according to the Non-Patent-Related Document 12 in which it is extracted with ethyl acetate, followed by, if necessary, purifying the target compound with column chromatography.

In addition, Gomisin H (GH) of the formula (4) can be derived from Schizandra chinensis according to Patent Document 5.

In addition, Gomisin J (GJ) of the formula (5) can be manufactured from Schisandrae Fructus according to Patent Document 1, in which deoxyschizandrin extracted from Schisandrae Fructus is acylated to obtain the compound of a formula (B) as mentioned below, then dealkylated (R₁ group of the formula), and deacylated (COR group of the formula) to obtain the target compound.

[ka21] 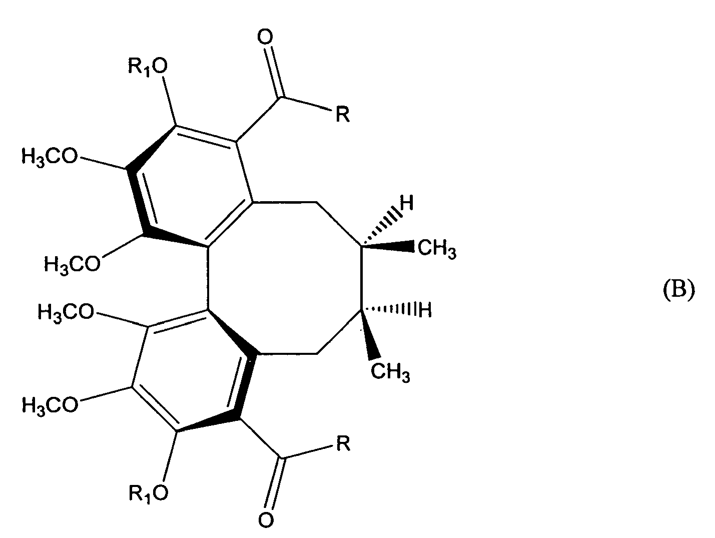 (In the formula, R₁ indicates lower alkyl, and R indicates hydrogen atom or lower alkyl.)

In addition, Schizandrin (Sch) of the formula (6) can be manufactured according to the method as disclosed in Patent Document 3. That is, after the extraction with a solvent having low polarity such as petroleum ether, it is subjected to the partition extraction using water-insoluble solvent having low polarity such as petroleum ether and n-heptane, and water-soluble solvent having high polarity such as methanol, followed by, if necessary, to treatment such as column chromatography and high-performance liquid chromatography to obtain the target compound.

In addition, Gomisin A (GA) can be manufacture according to the method of the Patent Document 3, or can be manufactured according to Patent Document 4, in which five-membered ring of the compound of a formula (A) as mentioned below is reduced to dihydroxylate, followed by subjecting to the oxidation and methanesulphonyl reaction to obtain dl-Schizandrin (III), and then is epimerically extracted to obtain the target compound.

[ka22]

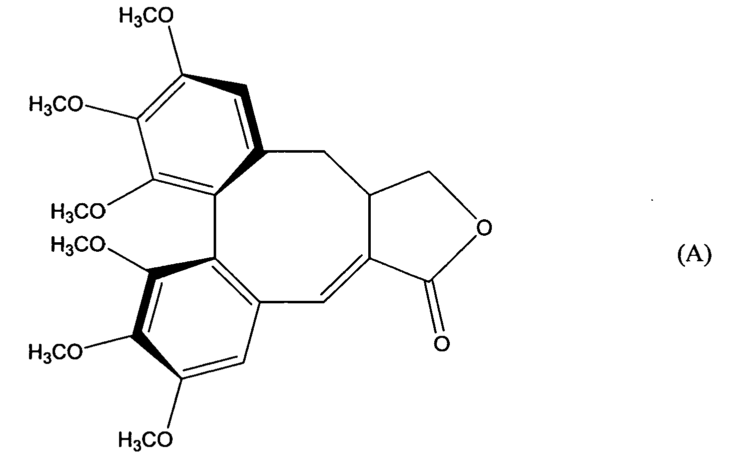

In addition, Gomisin B (GB) of the formula (8) can be manufacture from the dry stuck of Shizandra arisanensis according to the Non-Patent-Related Document 12 in which it is extracted with ethyl acetate, followed by, if necessary, purifying the target compound with column chromatography.

In addition, Gomisin N (GN) of the formula (9) can be derived from Schizandra chinensis according to Patent Document 5.

In addition, Schizandrin C (SC) of the formula (10) can be manufacture from Schisandrae Fructus according to Non-Patent-Related Document 13 in which the extracts of Schisandrae Fructus with petroleum ether are purified with silicagel column chromatography.

### Embodiment

### (Embodiment 1)

### Effect of Schizandrin B on the surviving rate of A549 cells irradiated with UV

A549 cells were seeded as 500 cells/60 mm dish in DMEM medium supplemented with 10% fetal bovine serum (FBS), 100 µg/mL of streptomycin and 100 units/mL penicillin (hereinafter, referred to as "growth medium"), and incubated for overnight. Schizandrin B (which is prepared as 10 g/L DMSO solution of Schizandrin B, and is used to dilute it with the medium as a DMSO concentration of less than 5 g/L) was added in the medium as final concentration of 1 µM and 10 µM in the medium.

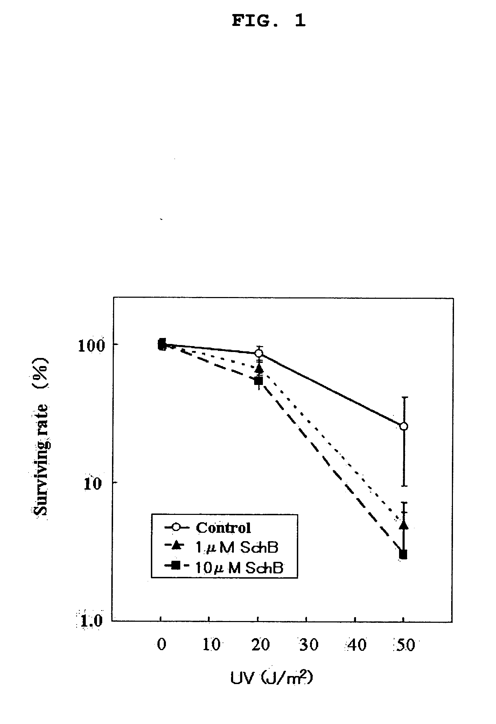

At 1 hour after the addition of Schizandrin B, the medium was removed, and were irradiated with 20 J/m² and 50 J/m² of UV (wavelength 254 nm, the same as follows) using Stratalinker (Stratagene). The medium containing the same concentration of Schizandrin B (hereinafter, referred to as "Schizandrin B-containing medium") was added. After the consecutive culture for 14 days, the cells were washed with phosphate-buffered-saline (PBS), and stained with 2% methylene blue. The surviving cell numbers were counted and calculated according to the clonogenic assay. The result is shown in Figure 1. In Figure 1, the horizontal axis indicates the radiation dose of irradiated UV, and the vertical axis indicates percentage of the surviving ratio of surviving cells of treated group with regard to that of non-treated group. In figures, symbol "○" indicates the group not treated with Schizandrin B, symbol "▲" indicates the group treated with 1 µM of Schizandrin B, and symbol "■" indicates the group treated with 10 µM of Schizandrin B. It should be noted that each date are means ± S.D. (n=3).

### (Embodiment 2-1)

### Effect of Schizandrin B on the cell cycle of A549 cells irradiated with UV

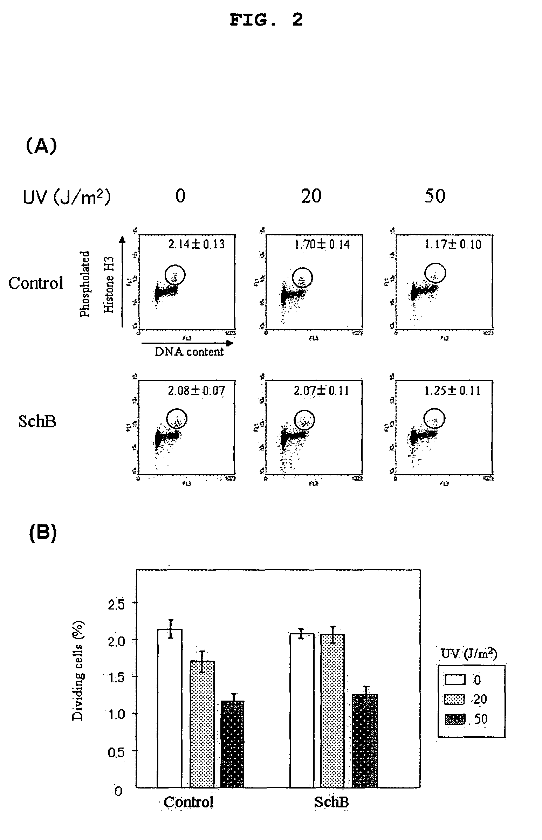

A549 cells were seeded as 3×10⁴ cells/cm² in the growth medium on 10 cm dish and incubated for overnight. Then, Schizandrin B was added to the medium as final concentration of 30 µM. At 1 hour after the addition of Schizandrin B, the medium was removed, and were irradiated with 20 J/m² and 50 J/m² of UV. After the UV irradiation, and then the cells were incubated in the Schizandrin B-containing medium having the same concentration as mentioned above for 1 hour. Then, the percentage of the cells containing phosphorylated histone H3 were measured in accordance with a Measurement of the phosphorylated histone H3 as mentioned below. The result is shown in Figure 2. The Figure 2(A) is a result of flowcytometry as obtained in the treatment with Schizandrin B. In the Figure 2(A), the encircled portion indicates the cell group in G2/M phase, numbers as mentioned above the portion indicates the ratio of the cells in G2/M phase to the total number of cells. In addition, the Figure 2(B) indicates a graph showing the ratio.

### (Embodiment 2-2)

Ratios of the cells in G2/M phase to the total number of cells were obtained in accordance with the Embodiment 2-1, except that, in the Embodiment 2-1, 20 J/m² of UV were irradiated instead of 20 J/m² and 50 J/m² of UV and the compounds of the formulas (1) to (10) and Angeloyl Gomisin A of a formula (C) (final concentration of 30 µM) as mentioned below were used instead of the final concentration of 30 µM of Schizandrin B. The result is shown in Table 1.

[ka23]

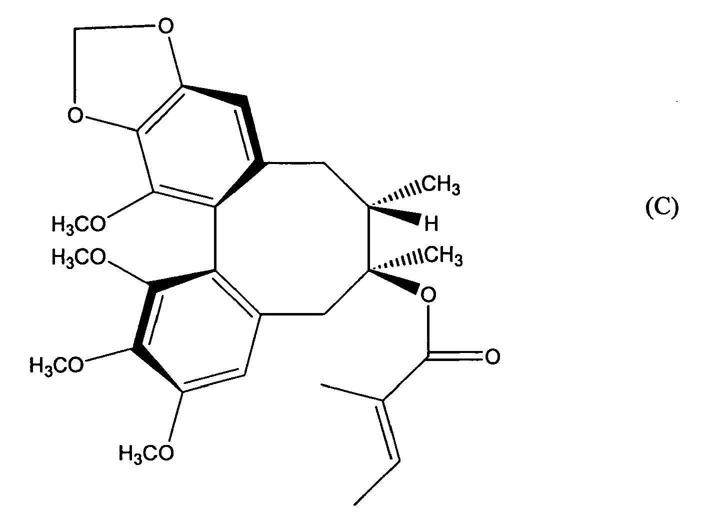

**[Table 1]**

| Compounds | Rate of Digided cells (%) |
|---|---|
| Control | 1.88±0.11 |
| UV irradiation | 0.52±0.10 |
| SchC | 0.68±0.11 |
| SchB | 1.09±0.11 |
| S | 0.68±0.04 |
| AGA | 1.04±0.06 |
| GA | 0.90±0.07 |
| GB | 0.85±0.12 |
| GC | 1.16±0.16 |
| GG | 1.13±0.05 |
| GH | 1.42±0.03 |
| GJ | 1.08±0.08 |
| GN | 0.88±0.12 |

### (Embodiment 3)

### Effect of Schizandrin B on the rate of dividing cells in A549 cells irradiated with UV

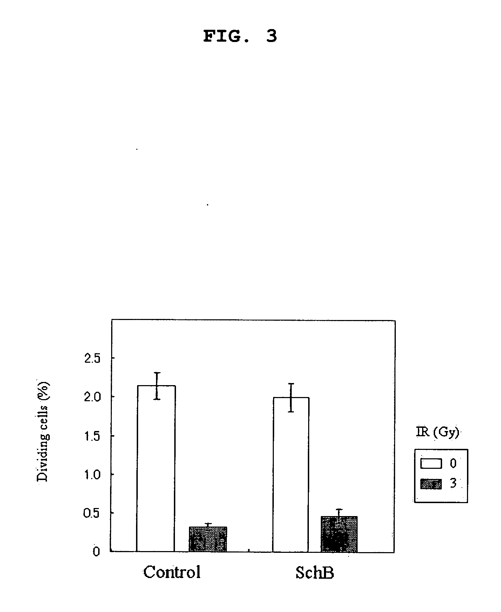

A549 cells were seeded as 3×10⁴ cells/cm² in the growth medium on 10 cm dish, incubated for overnight, and Schizandrin B was added in the medium as final concentration of 30 µM in the medium. After 1 hour incubation, the medium was removed, irradiated with 3 Gy of ionizing radiation, and incubated for 1 hour in the Schizandrin B-containing medium having the same concentration as mentioned above. The Measurement of the cells containing phosphorylated histone H3 as mentioned below was performed for the cells. The result is shown in Figure 3. Values in the Figure are the same as those of Figure 2(B).

### (Embodiment 4)

### Effect of Schizandrin B on the phosphorylation of p53 in A549 cells irradiated with UV or ionizing radiation

A549 cells were seeded as 3 × 10⁴ cells/cm² in the growth medium on 10 cm dish, incubated for overnight, and Schizandrin B was added in the medium as final concentration of 30 µM in the medium. After 1 hour incubation, the medium was removed, irradiated with 20 J/m² of UV, and incubated for 3 hours (hereinafter, referred to as "UV-cell group" with regard to the obtained cells).

In addition, as mentioned above, A549 cells were incubated for overnight, Schizandrin B was added as the final concentration of 30 µM in the medium, and incubated for 1 hour. The medium of the cells was removed, and the cells were irradiated with 10 Gy of ionizing radiation (γ ray), and incubated for 2 hours in the Schizandrin B-containing medium having the same concentration as mentioned above (hereinafter, referred to as "IR-cell group" with regard to the obtained cells).

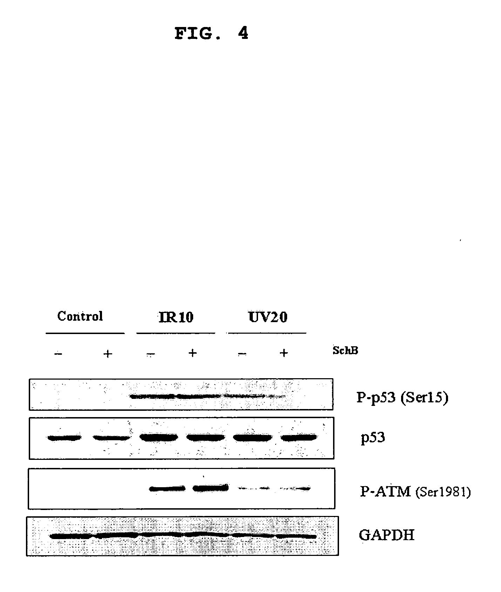

It should be noted that both groups of the UV-cell group and IR-cell group were cultured in the presence of 50 µM of N-acetyl-L-Leucyl-L-Leucyl-L-norleucinal (Sigma, MO, USA) (hereinafter, referred to as "LLnL") from 15 minutes prior to the irradiation to collection of the cells as mentioned below. Protein in the cells were extracted in accordance with a Preparation of proteins as mentioned below, immunoblot analysis was performed with regard to the phosphorylated p53 at 15 serine (P-p53 (Ser 15)), p53 protein (p53), phosphorylated ATM protein at 1981 serine (P-ATM (Ser1981)) and glyceraldehyde-3-phosphate dehydrogenase (GAPDH) in accordance with an Immunoblot analysis as mentioned below. The result is shown in Figure 4.

### (Embodiment 5-1)

### The dose-depended effect of Schizandrin B on the phosphorylation of p53 in A549 cells irradiated with UV

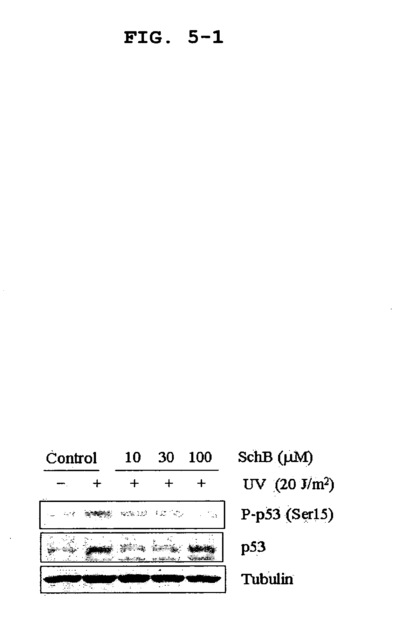

A549 cells were seeded as 3 × 10⁹ cells/cm² in the growth medium on 10 cm dish, incubated for overnight, and Schizandrin B was added in the medium as final concentration of 10, 30 and 100 µM in the medium. After 1 hour incubation, the medium was removed, and the cells were irradiated with 20 J/m² of UV. The cells were treated with LLnL as mentioned in Embodiment 4. Then, expression of p53 and phosphorylated p53 at 15 serine (P-p53 (Ser15) in the cells treated with the same concentration of Schizandrin B as mentioned above was examined. The result is shown in Figure 5-1.

### (Embodiment 5-2)

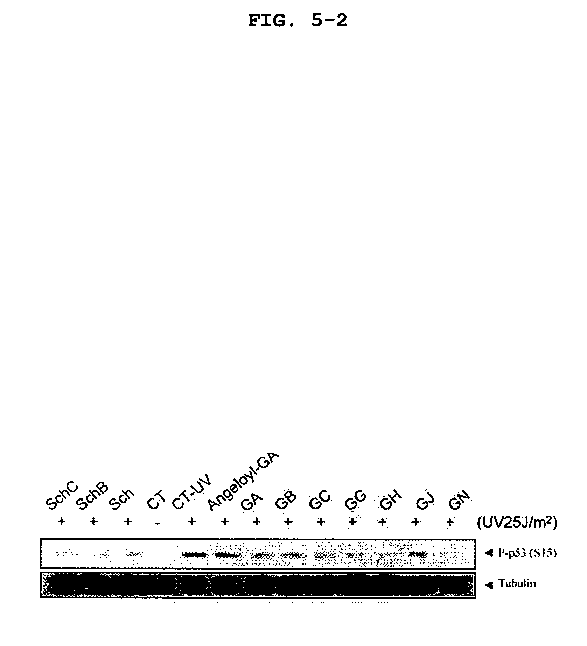

Expression of phosphorylated p53 at 15 serine (P-p53 (Ser15)) was examined in accordance with Embodiment 5-1, except that, in Embodiment 5-1, compounds of the Formulas (1) to (10) and the compound of the Formula (C) (final concentration of 30 µM) were added in the medium instead of the addition of final concentration of 10, 30 and 100 µM of Schizandrin B in the medium, and 25 J/m² of UV was irradiated instead of the 20 J/m² of UV irradiation. Electrophoresis image as obtained is shown in Figure 5-2, and relative ratio of the band intensity corresponding to P-p53 (Ser15) to that of the band as obtained in the irradiation with UV as 100% is shown in Table 2.

**[Table 2]**

| Compounds | Relative rate (%) |
|---|---|
| UV irradiation | 100.0 |
| Control | 15.4 |
| SchC | 42.6 |
| SchB | 37.9 |
| S | 42.7 |
| AGA | 92.2 |
| GA | 58.3 |
| GB | 60.9 |
| GC | 49.8 |
| GG | 51.3 |
| GH | 50.3 |
| GJ | 61.1 |
| GN | 38.4 |

### (Embodiment 6)

### Effect of Schizandrin B on the cell cycle of A549 cells

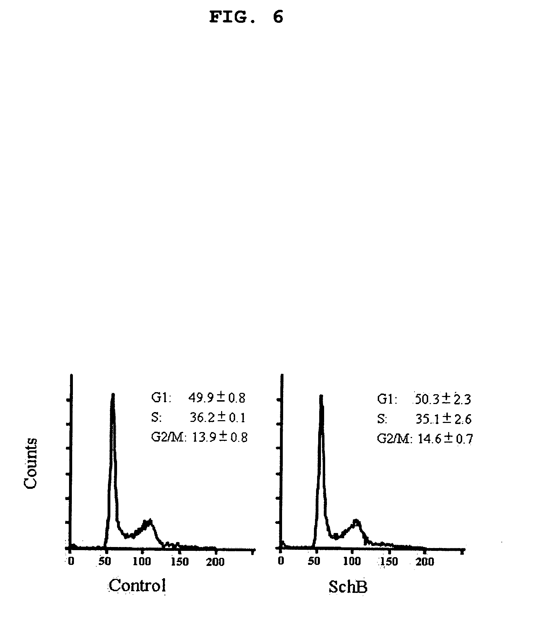

A549 cells were seeded as 3×10⁴ cells/cm² in the growth medium on 10 cm dish, incubated for overnight, and Schizandrin B was added in the medium as final concentration of 30 µM in the medium. After 4 hours of the addition, the cell cycle of the cells was examined in accordance with a FACS analysis as mentioned below. The result of the FACS analysis is shown in Figure 6.

### (Embodiment 7-1)

### Effect of Schizandrin B on the protein kinase activity of ATR and ATM

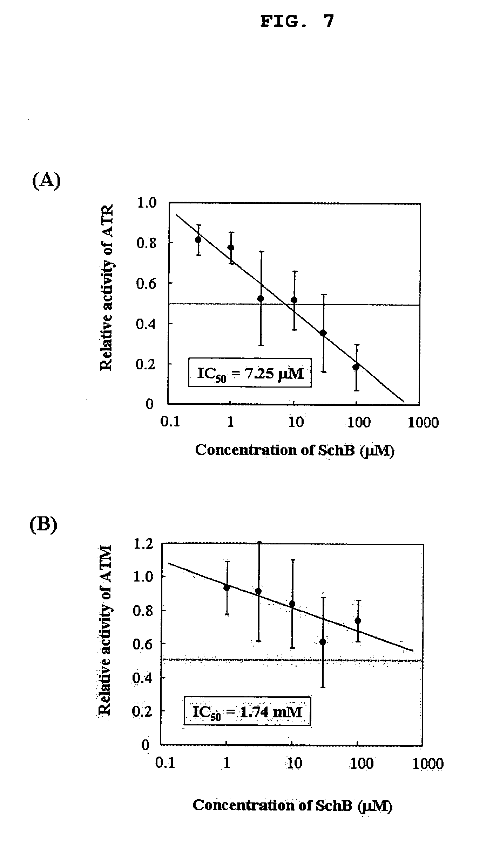

1 µg of recombinant PHAS-I protein (Alexis Biochemicals) as a substrate, and Schizandrin B were added to mixtures of ATR and ATM for the kinase reaction as prepared in accordance with a Preparation of mixtures of ATR and ATM for kinase reaction as follows. 300 µM of ATP containing a suitable concentration of ³²P-ATP was added to react it for 20 minutes at 30 °C. 4 volumes of SDS sample loading buffer (200 mM Tris-HCl (pH 6.8), 400 mM DTT and 8%SDS) were added to the reaction mixture to stop the reaction, and the obtained mixture is applied to 12% SDS-PAGE. After the gel as obtained was dried, γ ray corresponding to the band of ³²P-PHAS-I in the mixture was evaluated using autoradiography method. The result is shown in Figure 7. In the Figure, (A) and (B) indicate the relative activities of ATR and ATM , respectively, the horizontal axis indicates the concentration of Schizandrin B, and the vertical axis indicates the relative value of the count as obtained in Schizandrin B-treated group to that of no-addition of Schizandrin B group.

### (Embodiment 7-2)

### Effect of the ATR inhibitor according to the present invention on the kinase activity of ATR

IC₅₀s of the compounds for the phosphorylating activity of ATR were estimated based on the lines as obtained in accordance with Embodiment 7-1, except that, in Embodiment 7-1, the compounds of the Formulas (1) to (5) and the compound of the Formula (C) were used instead of Schizandrin B. The values are shown in Table 3.

**[Table 3]**

| Compounds | IC₅₀ (µM) |
|---|---|
| SchB | 7.25 |
| AGA | 110.78 |
| GC | 10.63 |
| GG | 32.00 |
| GH | 45.68 |
| GJ | 67.81 |

### (Embodiment 8)

### Effect of Schizandrin B on the phosphorylation of proteins in AT2KY cells irradiated with UV

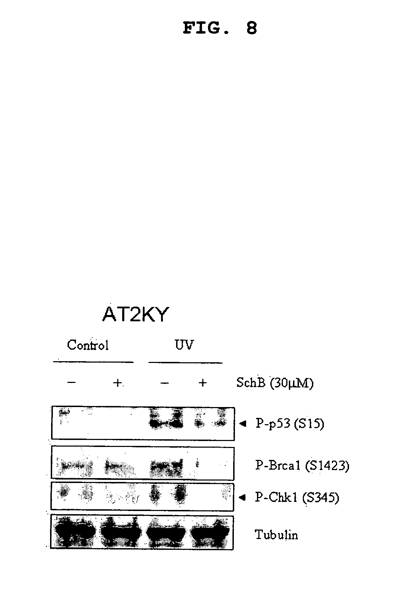

AT2KY cells (Health Science Research Resources Bank, Osaka, Japan) were seeded as 3×10⁴ cells/cm² in RPMI1640 supplemented with 15% FBS, 100 µg of streptomycin and 100 units/mL of penicillin on 10 cm dish, and incubated for overnight. Schizandrin B was added in the medium as final concentration of 30 µM in the medium. After 1 hour of the addition, the medium was removed, and 20 J/m² of UV was irradiated. Then, expression of phosphorylated p53 at 15 serine (P-p53 (Ser15)) phosphorylated Brca1 at 1423 Serine (P-Brcal (S1423)) and phosphorylated Chk1 at 345 serine in the cells treated with the same concentration of Schizandrin B as mentioned above for 4 hours were examined in accordance with the immunoblot analysis as mentioned below. The result is shown in Figure 8.

### (Embodiment 9)

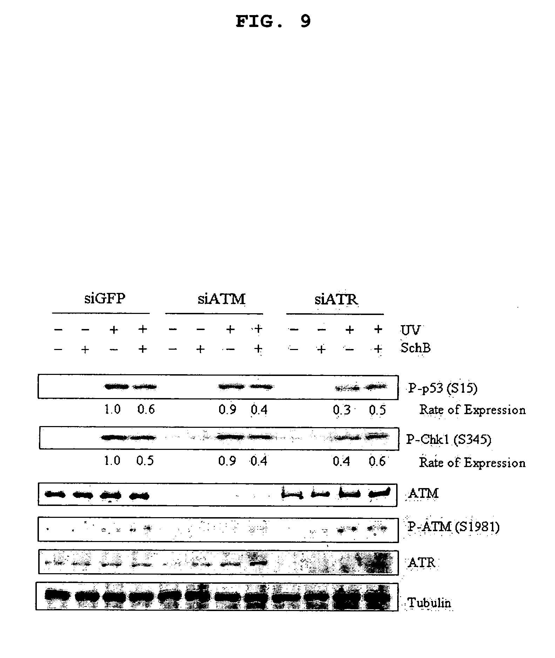

Effect of Schizandrin B on the phosphorylation of proteins in A549 cells irradiated with UV, wherein the expression of ATR or ATM is inhibited in the cells A549 cells were seed as 3×10⁴ cells/cm² in the growth medium on 10 cm dish, and incubated for overnight. The transfection of siRNAs (siGFP, siATM and siATR) as obtained in accordance with a Preparation of siRNA as mentioned below were performed in the cells using Oligofectamine (Invitrogen) and Opti-MEM (Invitrogen) with manufacture's guidance. In brief, after the incubation for overnight, the medium was replaced with a fresh growth medium for another 72 hours, the cells were treated with 30 µM of Schizandrin B for 1 hour, the medium was removed, and the cells were irradiated with 20 J/m² of UV. The phosphorylation of p53 at 15 serine (P-p53 (Ser15)), Chk1 at 345 Serine (P-Chk1 (S345)), and ATM protein at 1981 Serine (P-ATM(Ser1981)), and expression of ATM protein (ATM) and ATR protein (ATR) in the cells treated with the same concentration of Schizandrin B as mentioned above for 3 hours were examined by immunoblot analysis as mentioned below. The result is shown in Figure 9. It should be noted that, in Figure 9, the expression ratios as noted below the columns of P-p53(S15) and P-Chk1 (S345) indicate the relative band intensities to those as obtained in siGFP and UV of control group as 1.0.

### (Embodiment 10)

### Effect of Schizandrin B on the phosphorylation of MAPK in A549 cells

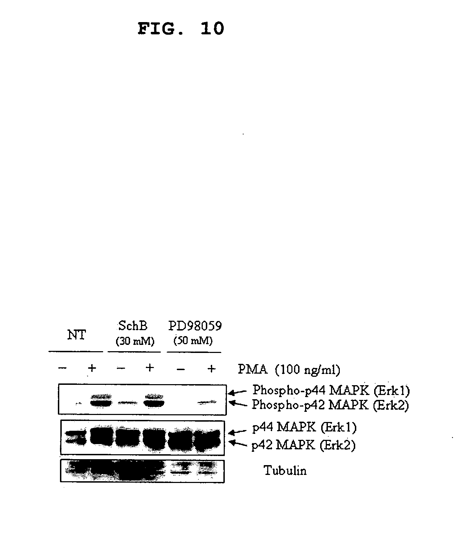

A549 cells were seeded as 3×10⁴ cells/cm² in the growth medium on 10 cm dish, incubated for overnight, and further incubated for 16 hours in the serum-free growth medium. Then, the cells were treated with 50 µM of PD098059 (Sigma) or 30 µM of Schizandrin B, and further treated with 100 ng/mL of phorbol 12-myristate 13-acetate (PMA; Sigma) for 5 minutes. The expressions of 42 kDa and 44 kD of mitogen activated protein kinase (p42 MAPK and p44 MAPK) and their phosphorylation were evaluated. The result is shown in Figure 10.

### (Measurement of the phosphorylated histone H3)

The cells were harvested and fixed with 70% ice-cooled ethanol after the washing with PBS. PBS containing 0.25% Triton X-100 was added, and the cells were stood on ice for 30 minutes. Cells were centrifuged (500 × g, 5 minutes) and replaced to PBS containing 1% bovine serum albumin (BSA) and 1 µg of anti-rabbit serum (specifically binding to the phosphorylated histone H3 at 13 Serine), and incubated for 4 hours at room temperature. After the subsequent washes with PBS containing 1% BSA, goat anti-rabbit IgG antibody conjugated with FITC which diluted with 100 times with PBS containing 1% BSA was added, and incubated for 30 minutes at dark place. These cells were counterstained with PI in accordance with the FACS analysis as mentioned below. The percentage of cells containing phosphorylated histone H3 was measured and evaluated with flowcytometry (Beckman-Coulter).

### (Immunoblot analysis)

50 µg of cellular proteins (as prepared in accordance with the Preparation of-proteins as mentioned below) were applied to SDS polyacrylamide gel electrophoresis (12.5%), the electrophored proteins were transferred into the nitrocellulose membrane in accordance with the standard method, and blocking reaction was performed for 1 hour at room temperature using Tris-buffered saline (TBS-T) containing 5% skim milk and 0.1% Triton X-100. So obtained membrane were incubated with the desired adequate antibodies as mentioned below for 16 hours at 4°C, and then incubated with secondary anti-serum (corresponding to the animal originated from the desired antibodies) conjugated with horseradish peroxidase for 1 hour at room temperature. Then, the target proteins were visualized with ECL reaction kit (Amersham) and chemiluminescence film (Amersham) to obtain a protein image.

### <Antibodies>

p53 antibody (Calbiochem)
Phosphorylated p53 antibody
(Cell signaling technology)
Phosphorylated ATM antibody (Rockland)
Phosphorylated Chk1 antibody
(Cell signaling technology)
ATR antibody (Bethyl)
Tubulin antibody (Sigma)
GAPDH antibody (Santa Cruz)
Phosphorylated Brca1 antibody (Upstate)
Anti-MAPK antibody and anti-phosphorylated MAPK antibody (Cell Signaling)

### (Preparation of proteins)

The cells were harvested with ice-cooled PBS from the culture dish, and were washed twice with cooled PBS. Then, it was centrifuged to obtain cell pellet. The pellet was solubilized with UTB buffer as mentioned below to prepare cellular protein. The content of the proteins was measured with Protein Assay kit (Bio-Rad).

| <UTB buffer> | |
|---|---|
| 8 mM | Urea |
| 150 mM | 2-mercaptoethanol |
| 50 mM | Tris (pH 7.5) |

### (FACS analysis)

After washing the cells with PBS, the cells were fixed with 70% ice-cooled ethanol. The pellet as colleted with centrifugation was washed with PBS, and further centrifuged. Cells were re-suspended with a solution containing 100 µM final concentration of propidium iodide (Sigma, hereinafter, referred to as "PI") and 40 µg/mL of RNase A (PI solution), and incubated for 30 minutes at room temperature. Then, the cell cycle analysis was performed with flowcytometry (Beckman-Coulter).

### (Preparation of mixtures of ATR and ATM for kinase reaction)

ATR conjugated with Flag as constructed with conventional method as disclosed in, e.g. Non-Patent-Related Document 9 was used for a mixture of ATR for kinase reaction. In detail, Bam H1-Swa I fragment of ATR gene (Sequence Number 4) (1 kb) was substituted with a gene having the ATR gene and Flag (KDDDDK) at N terminal and the gene was amplified with PCR. So obtained gene product was introduced into pcDNA3.1, and target product was obtained from the plasmid. In addition, ATM conjugated with Flag as manufactured in accordance with the well-known method as disclosed in e.g. non-patent related documents 10 and 11 and using Quikchange site-Directed Mutagenesis Kit (Stratagene) was used for a mixture of ATM for kinase reaction.

These ATR and ATM plasmids tagged with Flag (Sequence Numbers 4 and 5) were transfected in 293T cells (ATCC: CRL-11268) by Calcium phosphate method as followed. After 2 days, 5 mg of cellular proteins were prepared using IP buffer in accordance with the Preparation of proteins as mentioned below instead of the UTB buffer, and ATR and ATM were immuno-precipitated with 20 µg of anti-Flag M2 monoclonal antibody (Sigma) for 4 hours at 4°C, independently. The precipitates were incubated with beads conjugated with protein G cepharose (Amersham). The obtained immuno-complexes were washed twice with a TGN buffer, and further washed once with a Kinase buffer as mentioned below to obtain Mixtures of ATR and ATM for kinase reaction (Sequence Numbers 6 and 7).

| <IP buffer> | |
|---|---|
| 10 mM | Tris (pH7.5) |
| 1 mM | EDTA |
| 1 mM | EGTA |
| 150 mM | NaCl |
| 0.5% | NP-40 |
| 1% | Triton X-100 |
| 1 mM | Phenylmethane sulfonylfluoride (PMSF) |
| 2 µg/mL | Pepstatin |
| 2 µg/mL | Aprotinin |
| 1 mM | p,p' - |

dichlorodiphenyltrichloroethane(DDT)

| <TGN buffer> | |
|---|---|
| 50 mM | Tris (pH7.4) |
| 50 mM | Glycerophosphoric acid |
| 150 mM | NaCl |
| 1% | Tween20 |
| 10% | Glycerol |

| <Kinase buffer> | |
|---|---|
| 10 mM | Hepes (pH7.5) |
| 50 mM | Glycerophosphoric acid |
| 50 mM | NaCl |
| 10 mM | MgCl₂ |
| 10 mM | MnCl₂ |

### <Calcium phosphate method>

293T cells were seed as 2 × 10⁴ cells/cm² in the growth medium on 10 cm dish, and incubated for overnight. The above-mentioned Flag-ATM or Flag-ATR mixed with 25 mM final concentration of BES (N,N-bis(2-hydroxyethyl)-2-aminoethanesulfonic acid) and 25 mM of CaCl₂ were added to the cell cultures. After the incubation for overnight, the medium was replaced with a fresh growth medium and incubated for 2 days.

### (Preparation of siRNA)

The Double strand siRNAs for ATR, ATM and green fluorescence protein (GFP) (corresponding to Sequence Numbers 1, 2 and 3, respectively) were purchased from Qiagen (HP GenomeWide siRNA). The double strand siRNAs were referred to as siATR, siATM and siGFP.

### (Discussion)

ATM (ataxia telangiectsia mutated) and ATR (ataxia telangiectasia and Rad-3-related) are playing major role in cellular DNA damage responsive signal transduction of cell cycle. ATM and ATR phosphorylate the so-called checkpoint kinases and transcriptional factors such as Chk1, p53, NBS1, Brca1 and SMC1. In order to avoid carrying over the DNA damage to the next generation causing to carcinogenesis, these checkpoint signals are regulating the overall of cell cycle. The deficiency of ATM or ATR decreases the cell viability after DNA damage induced by UV, IR irradiation or damage inducible agents. This shows the requirements of ATM and ATR for the repair, maintenance and management of damaged DNA. In the present invention, the present applicant has demonstrated that Schizandrin B, an ingredient of Schisandra Chinensis, dose-dependently decreased the cell viability after UV irradiation. It indicates that Schizandrin B has an inhibitory effect on checkpoint signal transduction pathways in the process of DNA damage response in vivo.

Although Histone H3 is phosphorylated at 10 serine in unperturbed mitotic cells and it is an index for entering from G2 phase into M phase, DNA damages lead to decrease of the ratio of phosphorylated Histone H3 positive cells in the case that the G2/M checkpoint is effective. According to Figure 2, the ratio of the phosphorylated Histone H3 has decreased in the cells irradiated with 20 and 50 J/m² of UV. On the other hand, Schizandrin B of the formula (1) remarkably disrupted the G2/M checkpoint at 20 J/m² of UV irradiation, though it was not significant at 50 J/m² of UV. Indeed, in the low dose of UV irradiation, G2/M checkpoint was completely abolished by Schizandrin B during DNA damage response. In addition, G2/M checkpoint was completely abolished by the compounds of the formulas (1) to 10 and the compound of the formula (C). On the other hand, the dramatic inhibition of G2/M checkpoint did not occur in the cells irradiated with IR. It has been known that IR irradiation activates ATM and UV irradiation activates ATR, thereby going on the cell cycle checkpoints. These results suggest that Schizandrin B has an inhibitory effect on ATR activity rather than ATM in G2/M checkpoint during DNA damage induced low dose of UV irradiation. The same can apply to the ATR inhibitor according to the present invention such as the compounds of the formulas (1) to (10).

To clarify the inhibitory effect of the ATR inhibitor according to the present invention on ATR kinase activity in vivo, the p53 phosphorylation after DNA damage induced by IR or UV irradiation was examined. It is widely recognized that p53 is phosphorylated at multiple sites in vivo by several different kinds of protein kinases and that p53 is known as very important transcriptional factor in the cells. Considering the function of p53 in DNA damage response during cancer therapy, p53 is specifically phosphorylated at serine 15 by ATR and ATM when the cells are damaged by UV irradiation, γIR irradiation and methylating agent. In the present invention, p53 phosphorylation was decreased by Schizandrin B after 20 J/m² UV irradiation toward A549 cells, while it was not decreased after IR irradiation. The clear activation of ATM at serine 1981 was observed after IR irradiation, and slight activation after 20 J/m² of irradiation was observed. However, Schizandrin B did not influence on the ATM activity. In addition, any localized accumulation and bias of the cell cycle were not observed by Schizandrin B. Further, the inhibition of p53 phosphorylation was observed by the compounds of the formulas (1) to (10). These results indicate that the phosphorylation of p53 at serine 15 by 20 J/m² of UV irradiation is reduced only by ATR.

Non-Patent-Related Document 7 discloses an inhibitory effect of caffeine on ATM and ATR protein kinase activity of A549 adeno carcinoma cells. The present applicant has also examined the effect of Schizandrin B on ATM and ATR protein kinase activities in vitro with identical method of the document. Schizandrin B obviously inhibited the kinase activity of ATR rather than that of ATM, although the incubation of Schizandrin B with the kinase reaction buffer inhibited both ATM and ATR. Indeed, the IC₅₀ of ATR by Schizandrin B was about 240 fold against that of ATM. It has remarkably observed the inhibition of ATR protein kinase activity by the compounds of the formulas (1) to (5). These results of the cell viability and the kinase activity indicate that the ATR protein kinase activity was inhibited during the response of UV irradiation induced DNA damage of the cells.

To examine the effect of Schizandrin B on DNA damage checkpoints, fibroblasts derived from AT patient (AT2KY cells) and cells treated with siRNA were used. UV irradiation of AT2KY cells increased not only phosphorylated p53 but also phosphorylated Brca1 (Ser 1423) and Chk1 (Ser 345) as downstream molecules of ATM and ATR on the checkpoint pathway. Moreover, decreased phosphorylation of p53 and Chk1 by Schizandrin B was only seen in the cells treated with siRNA for control and ATM. In addition, the cells treated with siRNA did not show reduced phosphorylation of checkpoint proteins. These results indicate that Schizandrin B inhibited signal transduction pathways of DNA damage checkpoints in vivo through the inhibition of ATR protein kinase activity.

To verify the specificity of Schizandrin B on ATR inhibition in vivo, it was examined the effect of Schizandrin B on the phosphorylation of ERK, a mitogen activated protein kinase (MAPK) by its specific activation system with serum stimulation and PMA treatment. The phosphorylation of ERK was strongly enhanced by the referenced method. The treatment of Schizandrin B did not affect on ERK phosphorylation. Therefore, the efficacy of Schizandrin B in DNA damage response is highly specific to ATR activity.

In the present invention, the effects of Schizandrin B on DNA damage response induced by UV irradiation to the cells were demonstrated. Since the knocking down of ATR leads to lethal in embryo, it is speculated that ATR is playing crucial role in the developmental stages of mammals. This indicates that ATR is necessary for the checkpoint to the instability of chromatin and the deficiency of the checkpoint caused by the radical continuously created in the body, and further to replicative stress of the cellular DNA during development. On the other hand, ATR maintains the checkpoint in the cells of AT patient (cells genetically inactive of ATM), since ATR also works as a backup molecule of ATM. These indicate that ATR protein kinase is very important in the proliferating cells including tumor incidence. The finding as obtained in the present invention enables to make advantages of anti-cancer therapy such as radiation and chemotherapy since Schizandrin B is capable of abolishing the DNA damaged check points in patients through the inhibition of ATR kinase activity. Caffeine preferentially has 1/5 IC₅₀ for ATM than that of ATR. Therefore, it is speculated that single treatment of Schizandrin B, or the combined treatment of Schizandrin B with caffeine in the radiotherapy and anticancer drug therapy could increase the radio sensitivity in the human clinical cancer therapy.

Non-Patent-Related Document 3 reported the application of caffeine to clinical trial as an assistant of chemotherapy and radiochemotherapy. The document indicates that caffeine-assisted therapy reduced or minimized the size of osteosarcoma than individual treatment of chemotherapy or radiochemotherapy. These results indicate that caffeine inhibited ATM and ATR protein kinase activities, and resulting in the inhibition of the DNA damage checkpoint whichever in proliferating tumor cell cycle. These raise a possibility that Schizandrin B is applicable on clinical trial as Schizandrin B assistant chemotherapy or radiochemotherapy. For further investigation, it is required to estimate whether the elimination or minimization of tumor size can be streamlined by the combined effect of Schizandrin B, along with the clinical test of caffeine. Notably, Schizandrin B is an ingredient of natural plant extraction with expected no or less side effects in the patients. The combination of Schizandrin B with caffeine for chemo/radio therapy would be expected to reduce the amount of administration of chemo/radio therapy for patient with decreased side effects since these assistants are expected to increase the efficiency of chemo/radio therapy with lower dosage.

The present invention will be explained with reference to the preferable embodiments. It is obvious that modifications and alterations to these embodiments can be made without departing from the spirit and scope of the invention as defined in the claims, although it has been explained with the specific embodiments. That is, it should not be construed to limit the present invention to the details of the specific embodiments and attached drawing.

## Claims

1. ATR inhibitor containing a compound having a following formula (1) as an active constituent.
[kal]

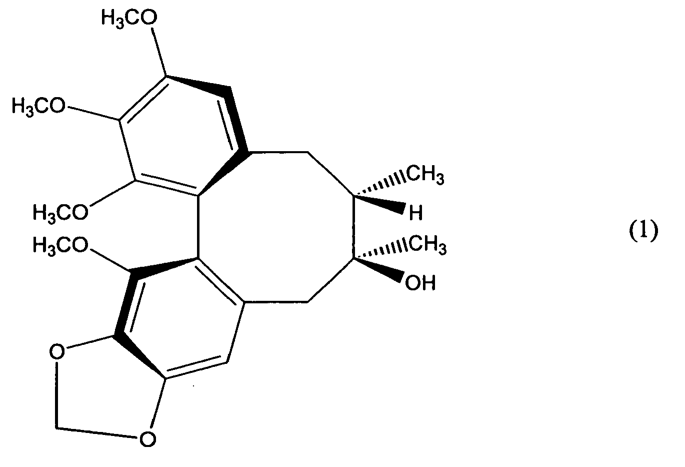

2. ATR inhibitor containing a compound having a following formula (2) as an active constituent.
[ka2]

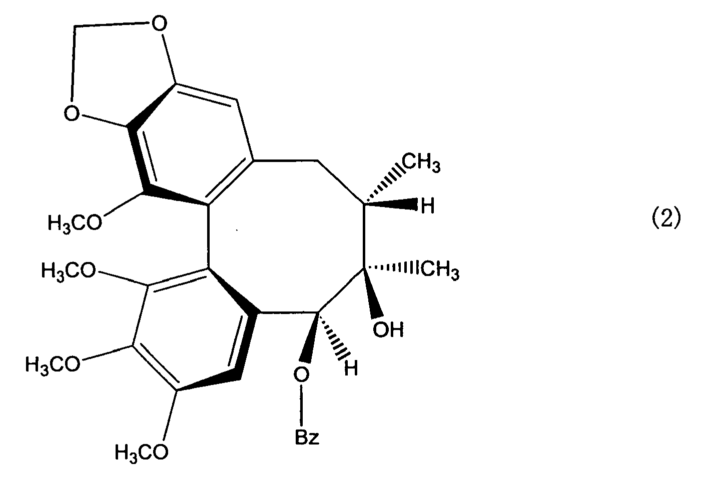

3. ATR inhibitor containing a compound having a following formula (3) as an active constituent.
[ka3]

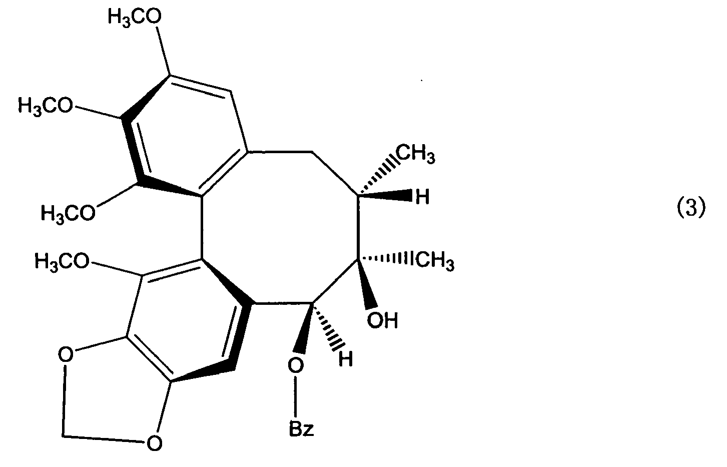

4. ATR inhibitor containing a compound having a following formula (4) as an active constituent.
[ka4]

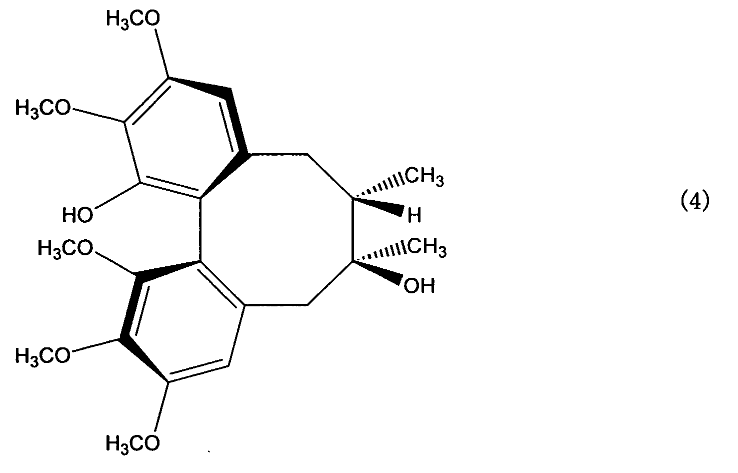

5. ATR inhibitor containing a compound having a following formula (5) as an active constituent.
[ka5]

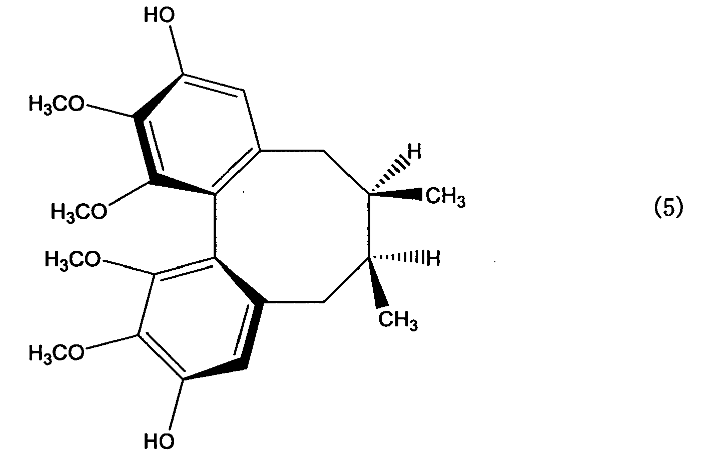

6. ATR inhibitor containing a compound having a following formula (6) as an active constituent.
[ka6]

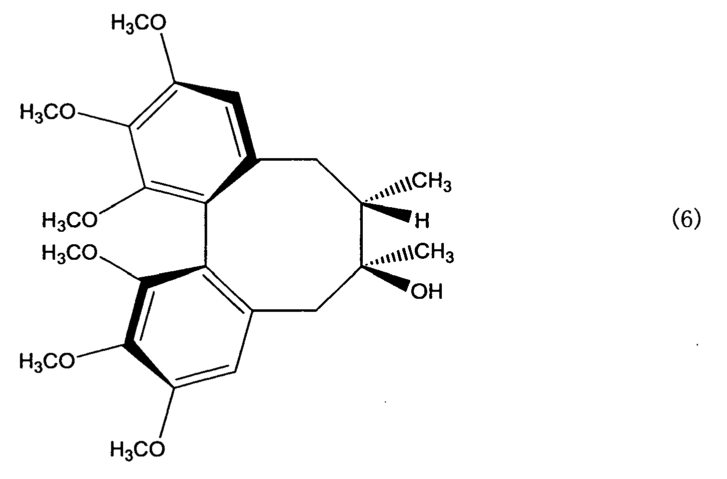

7. ATR inhibitor containing a compound having a following formula (7) as an active constituent.
[ka7]

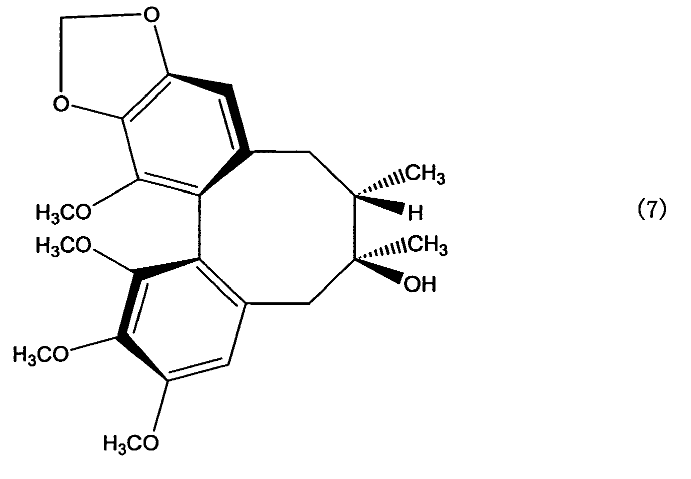

8. ATR inhibitor containing a compound having a following formula (8) as an active constituent.
[ka8]

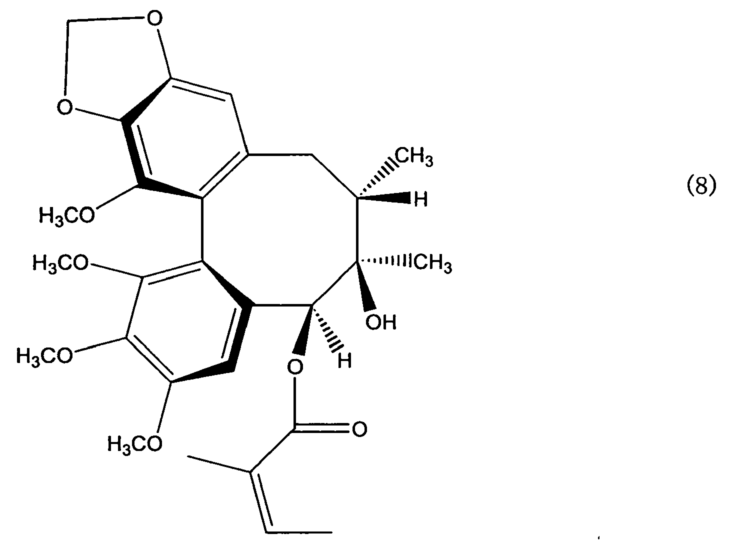

9. ATR inhibitor containing a compound having a following formula (9) as an active constituent.
[ka9]

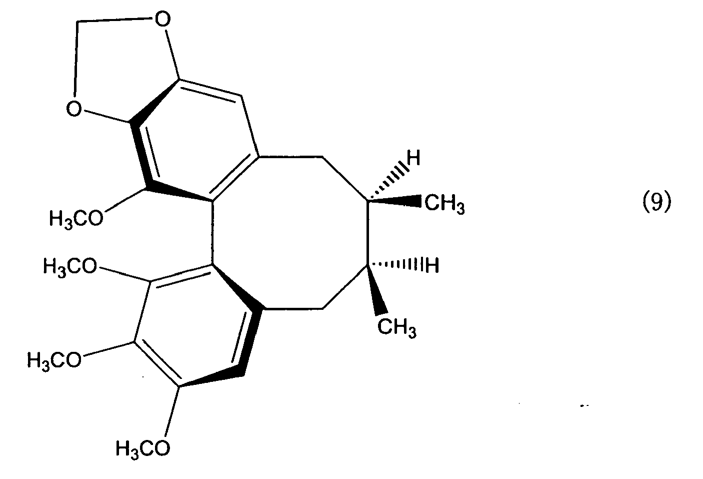

10. ATR inhibitor containing a compound having a following formula (10) as an active constituent.
[ka10]

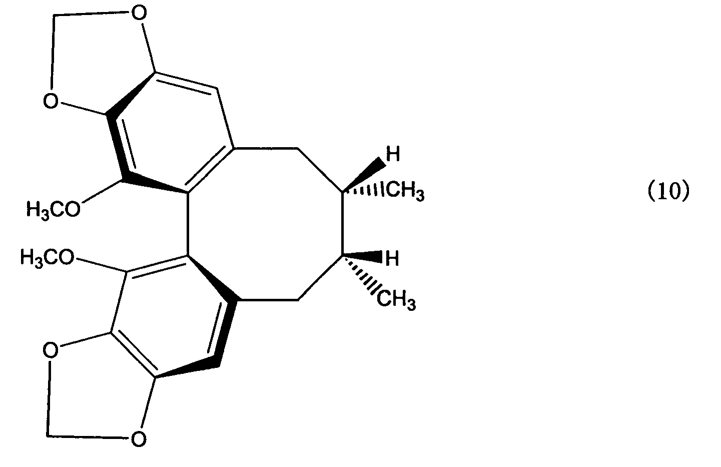

11. The ATR inhibitor as claimed in any one of Claims 1 to 10, wherein the ATR activity is a protein kinase activity.

12. The ATR inhibitor as claimed in Claim 11, wherein said protein kinase activity is an activity of phosphorylating cell-cycle-related proteins.

13. The ATR inhibitor as claimed in Claim 12, wherein said cell-cycle-related protein is p53.

14. The ATR inhibitor as claimed in Claim 12 or 13, wherein said cell-cycle-related proteins are selected from the group consisting of Brca1 and Chk1.


SEQUENCE LISTING

<110>  Niigata TLO Corporation

<120>  ATR inhibitor

<130>  KAB/FP6549646

<140>  EP 06811953.6
<141>  2006-10-18

<150>  PCT/JP2006/320756
<151>  2006-10-18

<150>  JP 2005-307534
<151>  2005-10-21

<160>  7

<170>  PatentIn version 3.3

<210>  1
<211>  21
<212>  DNA
<213>  Synthetic

<400>  1
aagcgcctga ttcgagatcc t                                              21

<210>  2
<211>  21
<212>  DNA
<213>  Synthetic

<400>  2
aagacggtgt gctcatgcgg c                                              21

<210>  3
<211>  24
<212>  DNA
<213>  Synthetic

<400>  3
cggcaagctg accctgaagt tcat                                           24

<210>  4
<211>  8265
<212>  DNA
<213>  Synthetic

<400>  4
gcctccacac ggctccgtcg ggcgccgcgc tcttccggca gcggtacgtt tggagacgcc      60

gggaacccgc gttggcgtgg ttgactagtg cctcgcagcc tcagcatggg ggaacatggc     120

ctggagctgg cttccatgat ccccgccctg cgggagctgg gcagtgccac accagaggaa     180

tataatacag ttgtacagaa gccaagacaa attctgtgtc aattcattga ccggatactt 240

acagatgtaa atgttgttgc tgtagaactt gtaaagaaaa ctgactctca gccaacctcc 300

gtgatgttgc ttgatttcat ccagcatatc atgaaatcct ccccacttat gtttgtaaat 360

gtgagtggaa gccatgagcg caaaggcagt tgtattgaat tcagtaattg gatcataacg 420

agacttctgc ggattgcagc aactccctcc tgtcatttgt tacacaagaa aatctgtgaa 480

gtcatctgtt cattattatt tctttttaaa agcaagagtc ctgctatttt tggggtactc 540

acaaaagaat tattacaact ttttgaagac ttggtttacc tccatagaag aaatgtgatg 600

ggtcatgctg tggaatggcc agtggtcatg agccgatttt taagtcaatt agatgaacac 660

atgggatatt tacaatcagc tcctttgcag ttgatgagta tgcaaaattt agaatttatt 720

gaagtcactt tattaatggt tcttactcgt attattgcaa ttgtgttttt tagaaggcaa 780

gaactcttac tttggcagat aggttgtgtt ctgctagagt atggtagtcc aaaaattaaa 840

tccctagcaa ttagcttttt aacagaactt tttcagcttg gaggactacc agcacaacca 900

gctagcactt ttttcagctc atttttggaa ttattaaaac accttgtaga aatggatact 960

gaccaattga aactctatga agagccatta tcaaagctga taaagacact atttcccttt 1020

gaagcagaag cttatagaaa tattgaacct gtctatttaa atatgctgct ggaaaaactc 1080

tgtgtcatgt ttgaagacgg tgtgctcatg cggcttaagt ctgatttgct aaaagcagct 1140

ttgtgccatt tactgcagta tttccttaaa tttgtgccag ctgggtatga atctgcttta 1200

caagtcagga aggtctatgt gagaaatatt tgtaaagctc ttttggatgt gcttggaatt 1260

gaggtagatg cagagtactt gttgggccca ctttatgcag ctttgaaaat ggaaagtatg 1320

gaaatcattg aggagattca atgccaaact caacaggaaa acctcagcag taatagtgat 1380

ggaatatcac ccaaaaggcg tcgtctcagc tcgtctctaa acccttctaa aagagcacca 1440

aaacagactg aggaaattaa acatgtggac atgaaccaaa agagcatatt atggagtgca 1500

ctgaaacaga aagctgaatc ccttcagatt tcccttgaat acagtggcct aaagaatcct 1560

gttattgaga tgttagaagg aattgctgtt gtcttacaac tgactgctct gtgtactgtt 1620

cattgttctc atcaaaacat gaactgccgt actttcaagg actgtcaaca taaatccaag 1680

aagaaacctt ctgtagtgat aacttggatg tcattggatt tttacacaaa agtgcttaag 1740

agctgtagaa gtttgttaga atctgttcag aaactggacc tggaggcaac cattgataag 1800

gtggtgaaaa tttatgatgc tttgatttat atgcaagtaa acagttcatt tgaagatcat 1860

atcctggaag atttatgtgg tatgctctca cttccatgga tttattccca ttctgatgat 1920

ggctgtttaa agttgaccac atttgccgct aatcttctaa cattaagctg taggatttca 1980

gatagctatt caccacaggc acaatcacga tgtgtgtttc ttctgactct gtttccaaga 2040

agaatattcc ttgagtggag aacagcagtt tacaactggg ccctgcagag ctcccatgaa 2100

gtaatccggg ctagttgtgt tagtggattt tttatcttat tgcagcagca gaattcttgt 2160

aacagagttc ccaagattct tatagataaa gtcaaagatg attctgacat tgtcaagaaa 2220

gaatttgctt ctatacttgg tcaacttgtc tgtactcttc acggcatgtt ttatctgaca 2280

agttctttaa cagaaccttt ctctgaacac ggacatgtgg acctcttctg taggaacttg 2340

aaagccactt ctcaacatga atgttcatct tctcaactaa aagcttctgt ctgcaagcca 2400

ttccttttcc tactgaaaaa aaaaatacct agtccagtaa aacttgcttt catagataat 2460

ctacatcatc tttgtaagca tcttgatttt agagaagatg aaacagatgt aaaagcagtt 2520

cttggaactt tattaaattt aatggaagat ccagacaaag atgttagagt ggcttttagt 2580

ggaaatatca agcacatatt ggaatccttg gactctgaag atggatttat aaaggagctt 2640

tttgtcttaa gaatgaagga agcatataca catgcccaaa tatcaagaaa taatgagctg 2700

aaggatacct tgattcttac aacaggggat attggaaggg ccgcaaaagg agatttggta 2760

ccatttgcac tcttacactt attgcattgt ttgttatcca agtcagcatc tgtctctgga 2820

gcagcataca cagaaattag agctctggtt gcagctaaaa gtgttaaact gcaaagtttt 2880

ttcagccagt ataagaaacc catctgtcag tttttggtag aatcccttca ctctagtcag 2940

atgacagcac ttccgaatac tccatgccag aatgctgacg tgcgaaaaca agatgtggct 3000

caccagagag aaatggcttt aaatacgttg tctgaaattg ccaacgtttt cgactttcct 3060

gatcttaatc gttttcttac taggacatta caagttctac tacctgatct tgctgccaaa 3120

gcaagccctg cagcttctgc tctcattcga actttaggaa aacaattaaa tgtcaatcgt 3180

agagagattt taataaacaa cttcaaatat attttttctc atttggtctg ttcttgttcc 3240

aaagatgaat tagaacgtgc ccttcattat ctgaagaatg aaacagaaat tgaactgggg 3300

agcctgttga gacaagattt ccaaggattg cataatgaat tattgctgcg tattggagaa 3360

cactatcaac aggtttttaa tggtttgtca atacttgcct catttgcatc cagtgatgat 3420

ccatatcagg gcccgagaga tatcatatca cctgaactga tggctgatta tttacaaccc 3480

aaattgttgg gcattttggc tttttttaac atgcagttac tgagctctag tgttggcatt 3540

gaagataaga aaatggcctt gaacagtttg atgtctttga tgaagttaat gggacccaaa 3600

catgtcagtt ctgtgagggt gaagatgatg accacactga gaactggcct tcgattcaag 3660

gatgattttc ctgaattgtg ttgcagagct tgggactgct ttgttcgctg cctggatcat 3720

gcttgtctgg gctcccttct cagtcatgta atagtagctt tgttacctct tatacacatc 3780

cagcctaaag aaactgcagc tatcttccac tacctcataa ttgaaaacag ggatgctgtg 3840

caagattttc ttcatgaaat atatttttta cctgatcatc cagaattaaa aaagataaaa 3900
gccgttctcc aggaatacag aaaggagacc tctgagagca ctgatcttca gacaactctt 3960
cagctctcta tgaaggccat tcaacatgaa aatgtcgatg ttcgtattca tgctcttaca 4020
agcttgaagg aaaccttgta taaaaatcag gaaaaactga taaagtatgc aacagacagt 4080
gaaacagtag aacctattat ctcacagttg gtgacagtgc ttttgaaagg ttgccaagat 4140
gcaaactctc aagctcggtt gctctgtggg gaatgtttag gggaattggg ggcgatagat 4200
ccaggtcgat tagatttctc aacaactgaa actcaaggaa aagattttac atttgtgact 4260
ggagtagaag attcaagctt tgcctatgga ttattgatgg agctaacaag agcttacctt 4320
gcgtatgctg ataatagccg agctcaagat tcagctgcct atgccattca ggagttgctt 4380
tctatttatg actgtagaga gatggagacc aacggcccag gtcaccaatt gtggaggaga 4440
tttcctgagc atgttcggga aatactagaa cctcatctaa ataccagata caagagttct 4500
cagaagtcaa ccgattggtc tggagtaaag aagccaattt acttaagtaa attgggtagt 4560
aactttgcag aatggtcagc atcttgggca ggttatctta ttacaaaggt tcgacatgat 4620
cttgccagta aaattttcac ctgctgtagc attatgatga agcatgattt caaagtgacc 4680
atctatcttc ttccacatat tctggtgtat gtcttactgg gttgtaatca agaagatcag 4740
caggaggttt atgcagaaat tatggcagtt ctaaagcatg acgatcagca taccataaat 4800
acccaagaca ttgcatctga tctgtgtcaa ctcagtacac agactgtgtt ctccatgctt 4860
gaccatctca cacagtgggc aaggcacaaa tttcaggcac tgaaagctga gaaatgtcca 4920
cacagcaaat caaacagaaa taaggtagac tcaatggtat ctactgtgga ttatgaagac 4980
tatcagagtg taacccgttt tctagacctc ataccccagg atactctggc agtagcttcc 5040
tttcgctcca aagcatacac acgagctgta atgcactttg aatcatttat tacagaaaag 5100
aagcaaaata ttcaggaaca tcttggattt ttacagaaat tgtatgctgc tatgcatgaa 5160
cctgatggag tggccggagt cagtgcaatt agaaaggcag aaccatctct aaaagaacag 5220
atccttgaac atgaaagcct tggcttgctg agggatgcca ctgcttgtta tgacagggct 5280
attcagctag aaccagacca gatcattcat tatcatggtg tagtaaagtc catgttaggt 5340
cttggtcagc tgtctactgt tatcactcag gtgaatggag tgcatgctaa caggtccgag 5400
tggacagatg aattaaacac gtacagagtg gaagcagctt ggaaattgtc acagtgggat 5460
ttggtggaaa actatttggc agcagatgga aaatctacaa catggagtgt cagactggga 5520
cagctattat tatcagccaa aaaaagagat atcacagctt tttatgactc actgaaacta 5580
gtgagagcag aacaaattgt acctctttca gctgcaagct ttgaaagagg ctcctaccaa 5640

cgaggatatg aatatattgt gagattgcac atgttatgtg agttggagca tagcatcaaa 5700
ccacttttcc agcattctcc aggtgacagt tctcaagaag attctctaaa ctgggtagct 5760
cgactagaaa tgacccagaa ttcctacaga gccaaggagc ctatcctggc tctccggagg 5820
gctttactaa gcctcaacaa aagaccagat tacaatgaaa tggttggaga atgctggctg 5880
cagagtgcca gggtagctag aaaggctggt caccaccaga cagcctacaa tgctctcctt 5940
aatgcagggg aatcacgact cgctgaactg tacgtggaaa gggcaaagtg gctctggtcc 6000
aagggtgatg ttcaccaggc actaattgtt cttcaaaaag gtgttgaatt atgttttcct 6060
gaaaatgaaa ccccacctga gggtaagaac atgttaatcc atggtcgagc tatgctacta 6120
gtgggccgat ttatggaaga aacagctaac tttgaaagca atgcaattat gaaaaaatat 6180
aaggatgtga ccgcgtgcct gccagaatgg gaggatgggc atttttacct tgccaagtac 6240
tatgacaaat tgatgcccat ggtcacagac aacaaaatgg aaaagcaagg tgatctcatc 6300
cggtatatag ttcttcattt tggcagatct ctacaatatg gaaatcagtt catatatcag 6360
tcaatgccac gaatgttaac tctatggctt gattatggta caaaggcata tgaatgggaa 6420
aaagctggcc gctccgatcg tgtacaaatg aggaatgatt tgggtaaaat aaacaaggtt 6480
atcacagagc atacaaacta tttagctcca tatcaatttt tgactgcttt ttcacaattg 6540
atctctcgaa tttgtcattc tcacgatgaa gtttttgttg tcttgatgga aataatagcc 6600
aaagtatttc tagcctatcc tcaacaagca atgtggatga tgacagctgt gtcaaagtca 6660
tcttatccca tgcgtgtgaa cagatgcaag gaaatcctca ataaagctat tcatatgaaa 6720
aaatccttag agaagtttgt tggagatgca actcgcctaa cagataagct tctagaattg 6780
tgcaataaac cggttgatgg aagtagttcc acattaagca tgagcactca ttttaaaatg 6840
cttaaaaagc tggtagaaga agcaacattt agtgaaatcc tcattcctct acaatcagtc 6900
atgataccta cacttccatc aattctgggt acccatgcta accatgctag ccatgaacca 6960
tttcctggac attgggccta tattgcaggg tttgatgata tggtggaaat tcttgcttct 7020
cttcagaaac caaagaagat ttctttaaaa ggctcagatg gaaagttcta catcatgatg 7080
tgtaagccaa aagatgacct gagaaaggat tgtagactaa tggaattcaa ttccttgatt 7140
aataagtgct taagaaaaga tgcagagtct cgtagaagag aacttcatat tcgaacatat 7200
gcagttattc cactaaatga tgaatgtggg attattgaat gggtgaacaa cactgctggt 7260
ttgagaccta ttctgaccaa actatataaa gaaaagggag tgtatatgac aggaaaagaa 7320
cttcgccagt gtatgctacc aaagtcagca gctttatctg aaaaactcaa agtattccga 7380
gaatttctcc tgcccaggca tcctcctatt tttcatgagt ggtttctgag aacattccct 7440
gatcctacat catggtacag tagtagatca gcttactgcc gttccactgc agtaatgtca 7500

atggttggtt atattctggg gcttggagac cgtcatggtg aaaatattct ctttgattct 7560

ttgactggtg aatgcgtaca tgtagatttc aattgtcttt tcaataaggg agaaaccttt 7620

gaagttccag aaattgtgcc atttcgcctg actcataata tggttaatgg aatgggtcct 7680

atgggaacag agggtctttt tcgaagagca tgtgaagtta caatgaggct gatgcgtgat 7740

cagcgagagc ctttaatgag tgtcttaaag acttttctac atgatcctct tgtggaatgg 7800

agtaaaccag tgaaagggca ttccaaagcg ccactgaatg aaactggaga agttgtcaat 7860

gaaaaggcca agacccatgt tcttgacatt gagcagcgac tacaaggtgt aatcaagact 7920

cgaaatagag tgacaggact gccgttatct attgaaggac atgtgcatta ccttatacaa 7980

gaagctactg atgaaaactt actatgccag atgtatcttg gttggactcc atatatgtga 8040

aatgaaatta tgtaaaagaa tatgttaata atctaaaagt aatgcatttg gtatgaatct 8100

gtggttgtat ctgttcaatt ctaaagtaca acataaattt acgttctcag caactgttat 8160

ttctctctga tcattaatta tatgtaaaat aatatacatt cagttattaa gaaataaact 8220

gctttcttaa taaaaaaaaa aaaaaaaaaa aaaaaaaaaa aaaaa 8265

<210> 5
<211> 13147
<212> DNA
<213> Homo sapiens

<400> 5
ccggagcccg agccgaaggg cgagccgcaa acgctaagtc gctggccatt ggtggacatg 60

gcgcaggcgc gtttgctccg acgggccgaa tgttttgggg cagtgttttg agcgcggaga 120

ccgcgtgata ctggatgcgc atgggcatac cgtgctctgc ggctgcttgg cgttgcttct 180

tcctccagaa gtgggcgctg ggcagtcacg cagggtttga accggaagcg ggagtaggta 240

gctgcgtggc taacggagaa aagaagccgt ggccgcggga ggaggcgaga ggagtcggga 300

tctgcgctgc agccaccgcc gcggttgata ctactttgac cttccgagtg cagtgacagt 360

gatgtgtgtt ctgaaattgt gaaccatgag tctagtactt aatgatctgc ttatctgctg 420

ccgtcaacta gaacatgata gagctacaga acgaaagaaa gaagttgaga aatttaagcg 480

cctgattcga gatcctgaaa caattaaaca tctagatcgg cattcagatt ccaaacaagg 540

aaaatatttg aattgggatg ctgtttttag atttttacag aaatatattc agaaagaaac 600

agaatgtctg agaatagcaa aaccaaatgt atcagcctca acacaagcct ccaggcagaa 660

aaagatgcag gaaatcagta gtttggtcaa atacttcatc aaatgtgcaa acagaagagc 720

acctaggcta aaatgtcaag aactcttaaa ttatatcatg gatacagtga aagattcatc 780

taatggtgct atttacggag ctgattgtag caacatacta ctcaaagaca ttctttctgt 840

gagaaaatac tggtgtgaaa tatctcagca acagtggtta gaattgttct ctgtgtactt 900
caggctctat ctgaaacctt cacaagatgt tcatagagtt ttagtggcta gaataattca 960
tgctgttacc aaaggatgct gttctcagac tgacggatta aattccaaat ttttggactt 1020
tttttccaag gctattcagt gtgcgagaca agaaaagagc tcttcaggtc taaatcatat 1080
cttagcagct cttactatct tcctcaagac tttggctgtc aactttcgaa ttcgagtgtg 1140
tgaattagga gatgaaattc ttcccacttt gctttatatt tggactcaac ataggcttaa 1200
tgattcttta aaagaagtca ttattgaatt atttcaactg caaatttata tccatcatcc 1260
gaaaggagcc aaaacccaag aaaaaggtgc ttatgaatca acaaaatgga gaagtatttt 1320
atacaactta tatgatctgc tagtgaatga gataagtcat ataggaagta gaggaaagta 1380
ttcttcagga tttcgtaata ttgccgtcaa agaaaatttg attgaattga tggcagatat 1440
ctgtcaccag gtttttaatg aagataccag atccttggag atttctcaat cttacactac 1500
tacacaaaga gaatctagtg attacagtgt cccttgcaaa aggaagaaaa tagaactagg 1560
ctgggaagta ataaaagatc accttcagaa gtcacagaat gattttgatc ttgtgccttg 1620
gctacagatt gcaacccaat taatatcaaa gtatcctgca agtttaccta actgtgagct 1680
gtctccatta ctgatgatac tatctcagct tctaccccaa cagcgacatg gggaacgtac 1740
accatatgtg ttacgatgcc ttacggaagt tgcattgtgt caagacaaga ggtcaaacct 1800
agaaagctca caaaagtcag atttattaaa actctggaat aaaatttggt gtattacctt 1860
tcgtggtata agttctgagc aaatacaagc tgaaaacttt ggcttacttg gagccataat 1920
tcagggtagt ttagttgagg ttgacagaga attctggaag ttatttactg ggtcagcctg 1980
cagaccttca tgtcctgcag tatgctgttt gactttggca ctgaccacca gtatagttcc 2040
aggaacggta aaaatgggaa tagagcaaaa tatgtgtgaa gtaaatagaa gcttttcttt 2100
aaaggaatca ataatgaaat ggctcttatt ctatcagtta gagggtgact tagaaaatag 2160
cacagaagtg cctccaattc ttcacagtaa ttttcctcat cttgtactgg agaaaattct 2220
tgtgagtctc actatgaaaa actgtaaagc tgcaatgaat ttttttccaa gcgtgccaga 2280
atgtgaacac caccaaaaag ataaagaaga actttcattc tcagaagtag aagaactatt 2340
tcttcagaca acttttgaca agatggactt tttaaccatt gtgagagaat gtggtataga 2400
aaagcaccag tccagtattg gcttctctgt ccaccagaat ctcaaggaat cactggatcg 2460
ctgtcttctg ggattatcag aacagcttct gaataattac tcatctgaga ttacaaattc 2520
agaaactctt gtccggtgtt cacgtctttt ggtgggtgtc cttggctgct actgttacat 2580
gggtgtaata gctgaagagg aagcatataa gtcagaatta ttccagaaag ccaagtctct 2640

aatgcaatgt gcaggagaaa gtatcactct gtttaaaaat aagacaaatg aggaattcag 2700

aattggttcc ttgagaaata tgatgcagct atgtacacgt tgcttgagca actgtaccaa 2760

gaagagtcca aataagattg catctggctt tttcctgcga ttgttaacat caaagctaat 2820

gaatgacatt gcagatattt gtaaaagttt agcatccttc atcaaaaagc catttgaccg 2880

tggagaagta gaatcaatgg aagatgatac taatggaaat ctaatggagg tggaggatca 2940

gtcatccatg aatctattta acgattaccc tgatagtagt gttagtgatg caaacgaacc 3000

tggagagagc caaagtacca taggtgccat taatccttta gctgaagaat atctgtcaaa 3060

gcaagatcta cttttcttag acatgctcaa gttcttgtgt ttgtgtgtaa ctactgctca 3120

gaccaatact gtgtccttta gggcagctga tattcggagg aaattgttaa tgttaattga 3180

ttctagcacg ctagaaccta ccaaatccct ccacctgcat atgtatctaa tgcttttaaa 3240

ggagcttcct ggagaagagt accccttgcc aatggaagat gttcttgaac ttctgaaacc 3300

actatccaat gtgtgttctt tgtatcgtcg tgaccaagat gtttgtaaaa ctattttaaa 3360

ccatgtcctt catgtagtga aaaacctagg tcaaagcaat atggactctg agaacacaag 3420

ggatgctcaa ggacagtttc ttacagtaat tggagcattt tggcatctaa caaaggagag 3480

gaaatatata ttctctgtaa gaatggccct agtaaattgc cttaaaactt tgcttgaggc 3540

tgatccttat tcaaaatggg ccattcttaa tgtaatggga aaagactttc ctgtaaatga 3600

agtatttaca caatttcttg ctgacaatca tcaccaagtt cgcatgttgg ctgcagagtc 3660

aatcaataga ttgttccagg acacgaaggg agattcttcc aggttactga aagcacttcc 3720

tttgaagctt cagcaaacag cttttgaaaa tgcatacttg aaagctcagg aaggaatgag 3780

agaaatgtcc catagtgctg agaaccctga aactttggat gaaatttata atagaaaatc 3840

tgttttactg acgttgatag ctgtggtttt atcctgtagc cctatctgcg aaaaacaggc 3900

tttgtttgcc ctgtgtaaat ctgtgaaaga gaatggatta gaacctcacc ttgtgaaaaa 3960

ggttttagag aaagtttctg aaacttttgg atatagacgt ttagaagact ttatggcatc 4020

tcatttagat tatctggttt tggaatggct aaatcttcaa gatactgaat acaacttatc 4080

ttcttttcct tttattttat taaactacac aaatattgag gatttctata gatcttgtta 4140

taaggttttg attccacatc tggtgattag aagtcatttt gatgaggtga agtccattgc 4200

taatcagatt caagaggact ggaaaagtct tctaacagac tgctttccaa agattcttgt 4260

aaatattctt ccttattttg cctatgaggg taccagagac agtgggatgg cacagcaaag 4320

agagactgct accaaggtct atgatatgct taaaagtgaa aacttattgg gaaaacagat 4380

tgatcactta ttcattagta atttaccaga gattgtggtg gagttattga tgacgttaca 4440

tgagccagca aattctagtg ccagtcagag cactgacctc tgtgactttt caggggattt 4500

ggatcctgct cctaatccac ctcattttcc atcgcatgtg attaaagcaa catttgccta 4560
tatcagcaat tgtcataaaa ccaagttaaa aagcatttta gaaattcttt ccaaaagccc 4620
tgattcctat cagaaaattc ttcttgccat atgtgagcaa gcagctgaaa caaataatgt 4680
ttataagaag cacagaattc ttaaaatata tcacctgttt gttagtttat tactgaaaga 4740
tataaaaagt ggcttaggag gagcttgggc ctttgttctt cgagacgtta tttatacttt 4800
gattcactat atcaaccaaa ggccttcttg tatcatggat gtgtcattac gtagcttctc 4860
cctttgttgt gacttattaa gtcaggtttg ccagacagcc gtgacttact gtaaggatgc 4920
tctagaaaac catcttcatg ttattgttgg tacacttata ccccttgtgt atgagcaggt 4980
ggaggttcag aaacaggtat tggacttgtt gaaatactta gtgatagata acaaggataa 5040
tgaaaacctc tatatcacga ttaagctttt agatcctttt cctgaccatg ttgtttttaa 5100
ggatttgcgt attactcagc aaaaaatcaa atacagtaga ggaccctttt cactcttgga 5160
ggaaattaac cattttctct cagtaagtgt ttatgatgca cttccattga caagacttga 5220
aggactaaag gatcttcgaa gacaactgga actacataaa gatcagatgg tggacattat 5280
gagagcttct caggataatc cgcaagatgg gattatggtg aaactagttg tcaatttgtt 5340
gcagttatcc aagatggcaa taaaccacac tggtgaaaaa gaagttctag aggctgttgg 5400
aagctgcttg ggagaagtgg gtcctataga tttctctacc atagctatac aacatagtaa 5460
agatgcatct tataccaagg cccttaagtt atttgaagat aaagaacttc agtggacctt 5520
cataatgctg acctacctga ataacacact ggtagaagat tgtgtcaaag ttcgatcagc 5580
agctgttacc tgtttgaaaa acattttagc cacaaagact ggacatagtt tctgggagat 5640
ttataagatg acaacagatc caatgctggc ctatctacag ccttttagaa catcaagaaa 5700
aaagttttta gaagtaccca gatttgacaa agaaaaccct tttgaaggcc tggatgatat 5760
aaatctgtgg attcctctaa gtgaaaatca tgacatttgg ataaagacac tgacttgtgc 5820
ttttttggac agtggaggca caaaatgtga aattcttcaa ttattaaagc caatgtgtga 5880
agtgaaaact gacttttgtc agactgtact tccatacttg attcatgata ttttactcca 5940
agatacaaat gaatcatgga gaaatctgct ttctacacat gttcagggat ttttcaccag 6000
ctgtcttcga cacttctcgc aaacgagccg atccacaacc cctgcaaact tggattcaga 6060
gtcagagcac tttttccgat gctgtttgga taaaaaatca caaagaacaa tgcttgctgt 6120
tgtggactac atgagaagac aaaagagacc ttcttcagga acaattttta atgatgcttt 6180
ctggctggat ttaaattatc tagaagttgc caaggtagct cagtcttgtg ctgctcactt 6240
tacagcttta ctctatgcag aaatctatgc agataagaaa agtatggatg atcaagagaa 6300

aagaagtctt gcatttgaag aaggaagcca gagtacaact atttctagct tgagtgaaaa 6360
aagtaaagaa gaaactggaa taagtttaca ggatcttctc ttagaaatct acagaagtat 6420
aggggagcca gatagtttgt atggctgtgg tggagggaag atgttacaac ccattactag 6480
actacgaaca tatgaacacg aagcaatgtg gggcaaagcc ctagtaacat atgacctcga 6540
aacagcaatc ccctcatcaa cacgccaggc aggaatcatt caggccttgc agaatttggg 6600
actctgccat attctttccg tctatttaaa aggattggat tatgaaaata aagactggtg 6660
tcctgaacta gaagaacttc attaccaagc agcatggagg aatatgcagt gggaccattg 6720
cacttccgtc agcaaagaag tagaaggaac cagttaccat gaatcattgt acaatgctct 6780
acaatctcta agagacagag aattctctac attttatgaa agtctcaaat atgccagagt 6840
aaaagaagtg gaagagatgt gtaagcgcag ccttgagtct gtgtattcgc tctatcccac 6900
acttagcagg ttgcaggcca ttggagagct ggaaagcatt ggggagcttt tctcaagatc 6960
agtcacacat agacaactct ctgaagtata tattaagtgg cagaaacact cccagcttct 7020
caaggacagt gattttagtt ttcaggagcc tatcatggct ctacgcacag tcattttgga 7080
gatcctgatg gaaaaggaaa tggacaactc acaaagagaa tgtattaagg acattctcac 7140
caaacacctt gtagaactct ctatactggc cagaactttc aagaacactc agctccctga 7200
aagggcaata tttcaaatta aacagtacaa ttcagttagc tgtggagtct ctgagtggca 7260
gctggaagaa gcacaagtat tctgggcaaa aaaggagcag agtcttgccc tgagtattct 7320
caagcaaatg atcaagaagt tggatgccag ctgtgcagcg aacaatccca gcctaaaact 7380
tacatacaca gaatgtctga gggtttgtgg caactggtta gcagaaacgt gcttagaaaa 7440
tcctgcggtc atcatgcaga cctatctaga aaaggcagta gaagttgctg gaaattatga 7500
tggagaaagt agtgatgagc taagaaatgg aaaaatgaag gcatttctct cattagcccg 7560
gttttcagat actcaatacc aaagaattga aaactacatg aaatcatcgg aatttgaaaa 7620
caagcaagct ctcctgaaaa gagccaaaga ggaagtaggt ctccttaggg aacataaaat 7680
tcagacaaac agatacacag taaaggttca gcgagagctg gagttggatg aattagccct 7740
gcgtgcactg aaagaggatc gtaaacgctt cttatgtaaa gcagttgaaa attatatcaa 7800
ctgcttatta agtggagaag aacatgatat gtgggtattc cgactttgtt ccctctggct 7860
tgaaaattct ggagtttctg aagtcaatgg catgatgaag agagacggaa tgaagattcc 7920
aacatataaa tttttgcctc ttatgtacca attggctgct agaatgggga ccaagatgat 7980
gggaggccta ggatttcatg aagtcctcaa taatctaatc tctagaattt caatggatca 8040
cccccatcac actttgttta ttatactggc cttagcaaat gcaaacagag atgaatttct 8100
gactaaacca gaggtagcca gaagaagcag aataactaaa aatgtgccta aacaaagctc 8160

tcagcttgat gaggatcgaa cagaggctgc aaatagaata atatgtacta tcagaagtag 8220
gagacctcag atggtcagaa gtgttgaggc actttgtgat gcttatatta tattagcaaa 8280
cttagatgcc actcagtgga agactcagag aaaaggcata aatattccag cagaccagcc 8340
aattactaaa cttaagaatt tagaagatgt tgttgtccct actatggaaa ttaaggtgga 8400
ccacacagga gaatatggaa atctggtgac tatacagtca tttaaagcag aatttcgctt 8460
agcaggaggt gtaaatttac caaaaataat agattgtgta ggttccgatg gcaaggagag 8520
gagacagctt gttaagggcc gtgatgacct gagacaagat gctgtcatgc aacaggtctt 8580
ccagatgtgt aatacattac tgcagagaaa cacggaaact aggaagagga aattaactat 8640
ctgtacttat aaggtggttc ccctctctca gcgaagtggt gttcttgaat ggtgcacagg 8700
aactgtcccc attggtgaat ttcttgttaa caatgaagat ggtgctcata aaagatacag 8760
gccaaatgat ttcagtgcct ttcagtgcca aaagaaaatg atggaggtgc aaaaaaagtc 8820
ttttgaagag aaatatgaag tcttcatgga tgtttgccaa aattttcaac cagttttccg 8880
ttacttctgc atggaaaaat tcttggatcc agctatttgg tttgagaagc gattggctta 8940
tacgcgcagt gtagctactt cttctattgt tggttacata cttggacttg gtgatagaca 9000
tgtacagaat atcttgataa atgagcagtc agcagaactt gtacatatag atctaggtgt 9060
tgcttttgaa cagggcaaaa tccttcctac tcctgagaca gttcctttta gactcaccag 9120
agatattgtg gatggcatgg gcattacggg tgttgaaggt gtcttcagaa gatgctgtga 9180
gaaaaccatg gaagtgatga gaaactctca ggaaactctg ttaaccattg tagaggtcct 9240
tctatatgat ccactctttg actggaccat gaatcctttg aaagctttgt atttacagca 9300
gaggccggaa gatgaaactg agcttcaccc tactctgaat gcagatgacc aagaatgcaa 9360
acgaaatctc agtgatattg accagagttt caacaaagta gctgaacgtg tcttaatgag 9420
actacaagag aaactgaaag gagtggaaga aggcactgtg ctcagtgttg gtggacaagt 9480
gaatttgctc atacagcagg ccatagaccc caaaaatctc agccgacttt tcccaggatg 9540
gaaagcttgg gtgtgatctt cagtatatga attacccttt cattcagcct ttagaaatta 9600
tattttagcc tttattttta acctgccaac atactttaag tagggattaa tatttaagtg 9660
aactattgtg ggtttttttg aatgttggtt ttaatacttg atttaatcac cactcaaaaa 9720
tgttttgatg gtcttaagga acatctctgc tttcactctt tagaaataat ggtcattcgg 9780
gctgggcgca gcggctcacg cctgtaatcc cagcactttg ggaggccgag gtgagcggat 9840
cacaaggtca ggagttcgag accagcctgg ccaagagacc agcctggcca gtatggtgaa 9900
accctgtctc tactaaaaat acaaaaatta gccgagcatg gtggcgggca cctgtaatcc 9960

cagctactcg agaggctgag gcaggagaat ctcttgaacc tgggaggtga aggttgctgt 10020
gggccaaaat catgccattg cactccagcc tgggtgacaa gagcgaaact ccatctcaaa 10080
aaaaaaaaaa aaaaaacaga aacgtatttg gatttttcct agtaagatca ctcagtgtta 10140
ctaaataatg aagttgttat ggagaacaaa tttcaaagac acagttagtg tagttactat 10200
ttttttaagt gtgtattaaa acttctcatt ctattctctt tatcttttaa gcccttctgt 10260
actgtccatg tatgttatct ttctgtgata acttcataga ttgccttcta gttcatgaat 10320
tctcttgtca gatgtatata atctctttta ccctatccat tgggcttctt ctttcagaaa 10380
ttgtttttca tttctaatta tgcatcattt ttcagatctc tgtttcttga tgtcattttt 10440
aatgtttttt taatgttttt tatgtcacta attattttaa atgtctgtac ttgatagaca 10500
ctgtaatagt tctattaaat ttagttcctg ctgtttatat ctgttgattt ttgtatttga 10560
taggctgttc atccagtttt gtctttttga aaagtgagtt tattttcagc aaggctttat 10620
ctatgggaat cttgagtgtc tgtttatgtc atattcccag ggctgttgct gcacacaagc 10680
ccattcttat tttaatttct tggctttagg gtttccatac ctgaagtgta gcataaatac 10740
tgataggaga tttcccaggc caaggcaaac acacttcctc ctcatctcct tgtgctagtg 10800
ggcagaatat ttgattgatg cctttttcac tgagagtata agcttccatg tgtcccacct 10860
ttatggcagg ggtggaagga ggtacattta attcccactg cctgcctttg gcaagccctg 10920
ggttctttgc tccccatata gatgtctaag ctaaaagccg tgggttaatg agactggcaa 10980
attgttccag gacagctaca gcatcagctc acatattcac ctctctggtt tttcattccc 11040
ctcatttttt tctgagacag agtcttgctc tgtcacccag gctggagtgc agtggcatga 11100
tctcagctca ctgaaacctc tgcctcctgg gttcaagcaa ttctcctgcc tcagcctccc 11160
gagtagctgg gactacaggc gtgtgccaac acgcccggct aattttttgt atttttatta 11220
gagacggagt ttcaccgtgt tagccaggat ggtctcgatc gcttgacctc gtgatccacc 11280
ctcctcggcc tcccaaagtg ctgggattac aggtgtgagc caccgcgccc ggcctcattc 11340
ccctcatttt tgaccgtaag gatttcccct ttcttgtaag ttctgctatg tatttaaaag 11400
aatgttttct acattttatc cagcatttct ctgtgttctg ttggaaggga agggcttagg 11460
tatctagttt gatacatagg tagaagtgga acatttctct gtcccccagc tgtcatcata 11520
taagataaac atcagataaa aagccacctg aaagtaaaac tactgactcg tgtattagtg 11580
agtataatct cttctccatc cttaggaaaa tgttcatccc agctgcggag attaacaaat 11640
gggtgattga gctttctcct cgtatttgga ccttgaaggt tatataaatt tttttcttat 11700
gaagagttgg catttctttt tattgccaat ggcaggcact cattcatatt tgatctcctc 11760
accttcccct cccctaaaac caatctccag aactttttgg actataaatt tcttggtttg 11820

acttctggag aactgttcag aatattactt tgcatttcaa attacaaact taccttggtg 11880

tatctttttc ttacaagctg cctaaatgaa tatttggtat atattggtag ttttattact 11940

atagtaaatc aaggaaatgc agtaaactta aaatgtcttt aagaaagccc tgaaatcttc 12000

atgggtgaaa ttagaaatta tcaactagat aatagtatag ataaatgaat ttgtagctaa 12060

ttcttgctag ttgttgcatc cagagagctt tgaataacat cattaatcta ctctttagcc 12120

ttgcatggta tgctatgagg ctcctgttct gttcaagtat tctaatcaat ggctttgaaa 12180

agtttatcaa atttacatac agatcacaag cctaggagaa ataactaatt cacagatgac 12240

agaattaaga ttataaaaga tttttttttt gtaattttag tagagacagg gttgccattg 12300

tattccagcc ttggcgacag agcaagactc tgcctcaaaa aaaaaaaaaa aaaggttttg 12360

gcaagctgga actctttctg caaatgacta agatagaaaa ctgccaagga caaatgagga 12420

gtagttagat tttgaaaata ttaatcatag aatagttgtt gtatgctaag tcactgaccc 12480

atattatgta cagcatttct gatctttact ttgcaagatt agtgatacta tcccaataca 12540

ctgctggaga aatcagaatt tggagaaata agttgtccaa ggcaagaaga tagtaaatta 12600

taagtacaag tgtaatatgg acagtatcta acttgaaaag atttcaggcg aaaagaatct 12660

ggggtttgcc agtcagttgc tcaaaaggtc aatgaaaacc aaatagtgaa gctatcagag 12720

aagctaataa attatagact gcttgaacag ttgtgtccag attaagggag ataatagctt 12780

tcccacccta ctttgtgcag gtcatacctc cccaaagtgt ttacctaatc agtaggttca 12840

caaactcttg gtcattatag tatatgccta aaatgtatgc acttaggaat gctaaaaatt 12900

taaatatggt ctaaagcaaa taaaagcaaa gaggaaaaac tttggacagc gtaaagacta 12960

gaatagtctt ttaaaaagaa agccagtata ttggtttgaa atatagagat gtgtcccaat 13020

ttcaagtatt ttaattgcac cttaatgaaa ttatctattt tctatagatt ttagtactat 13080

tgaatgtatt actttactgt tacctgaatt tattataaag tgtttttgaa taaataattc 13140

taaaagc 13147

<210> 6
<211> 2644
<212> PRT
<213> Homo sapiens

<400> 6

Met Gly Glu His Gly Leu Glu Leu Ala Ser Met Ile Pro Ala Leu Arg
1               5                   10                  15

Glu Leu Gly Ser Ala Thr Pro Glu Glu Tyr Asn Thr Val Val Gln Lys
            20                  25                  30

Pro Arg Gln Ile Leu Cys Gln Phe Ile Asp Arg Ile Leu Thr Asp Val
        35                  40                  45

Asn Val Val Ala Val Glu Leu Val Lys Lys Thr Asp Ser Gln Pro Thr
    50                  55                  60

Ser Val Met Leu Leu Asp Phe Ile Gln His Ile Met Lys Ser Ser Pro
65                  70                  75                  80

Leu Met Phe Val Asn Val Ser Gly Ser His Glu Arg Lys Gly Ser Cys
                85                  90                  95

Ile Glu Phe Ser Asn Trp Ile Ile Thr Arg Leu Leu Arg Ile Ala Ala
            100                 105                 110

Thr Pro Ser Cys His Leu Leu His Lys Lys Ile Cys Glu Val Ile Cys
        115                 120                 125

Ser Leu Leu Phe Leu Phe Lys Ser Lys Ser Pro Ala Ile Phe Gly Val
    130                 135                 140

Leu Thr Lys Glu Leu Leu Gln Leu Phe Glu Asp Leu Val Tyr Leu His
145                 150                 155                 160

Arg Arg Asn Val Met Gly His Ala Val Glu Trp Pro Val Val Met Ser
                165                 170                 175

Arg Phe Leu Ser Gln Leu Asp Glu His Met Gly Tyr Leu Gln Ser Ala
            180                 185                 190

Pro Leu Gln Leu Met Ser Met Gln Asn Leu Glu Phe Ile Glu Val Thr
        195                 200                 205

Leu Leu Met Val Leu Thr Arg Ile Ile Ala Ile Val Phe Phe Arg Arg
    210                 215                 220

Gln Glu Leu Leu Leu Trp Gln Ile Gly Cys Val Leu Leu Glu Tyr Gly
225                 230                 235                 240

Ser Pro Lys Ile Lys Ser Leu Ala Ile Ser Phe Leu Thr Glu Leu Phe
                245                 250                 255

Gln Leu Gly Gly Leu Pro Ala Gln Pro Ala Ser Thr Phe Phe Ser Ser
            260                 265                 270

Phe Leu Glu Leu Leu Lys His Leu Val Glu Met Asp Thr Asp Gln Leu
        275                 280                 285

Lys Leu Tyr Glu Glu Pro Leu Ser Lys Leu Ile Lys Thr Leu Phe Pro
    290                 295                 300

Phe Glu Ala Glu Ala Tyr Arg Asn Ile Glu Pro Val Tyr Leu Asn Met
305                 310                 315                 320

Leu Leu Glu Lys Leu Cys Val Met Phe Glu Asp Gly Val Leu Met Arg
                325                 330                 335

Leu Lys Ser Asp Leu Leu Lys Ala Ala Leu Cys His Leu Leu Gln Tyr
            340                 345                 350

Phe Leu Lys Phe Val Pro Ala Gly Tyr Glu Ser Ala Leu Gln Val Arg
        355                 360                 365

Lys Val Tyr Val Arg Asn Ile Cys Lys Ala Leu Leu Asp Val Leu Gly
    370                 375                 380

Ile Glu Val Asp Ala Glu Tyr Leu Leu Gly Pro Leu Tyr Ala Ala Leu
385                 390                 395                 400

Lys Met Glu Ser Met Glu Ile Ile Glu Glu Ile Gln Cys Gln Thr Gln
                405                 410                 415

Gln Glu Asn Leu Ser Ser Asn Ser Asp Gly Ile Ser Pro Lys Arg Arg
            420                 425                 430

Arg Leu Ser Ser Ser Leu Asn Pro Ser Lys Arg Ala Pro Lys Gln Thr
        435                 440                 445

Glu Glu Ile Lys His Val Asp Met Asn Gln Lys Ser Ile Leu Trp Ser
    450                 455                 460

Ala Leu Lys Gln Lys Ala Glu Ser Leu Gln Ile Ser Leu Glu Tyr Ser
465                 470                 475                 480

Gly Leu Lys Asn Pro Val Ile Glu Met Leu Glu Gly Ile Ala Val Val
                485                 490                 495

Leu Gln Leu Thr Ala Leu Cys Thr Val His Cys Ser His Gln Asn Met
            500                 505                 510

Asn Cys Arg Thr Phe Lys Asp Cys Gln His Lys Ser Lys Lys Lys Pro
        515                 520                 525

Ser Val Val Ile Thr Trp Met Ser Leu Asp Phe Tyr Thr Lys Val Leu
    530                 535                 540

Lys Ser Cys Arg Ser Leu Leu Glu Ser Val Gln Lys Leu Asp Leu Glu
545                 550                 555                 560

Ala Thr Ile Asp Lys Val Val Lys Ile Tyr Asp Ala Leu Ile Tyr Met
                565                 570                 575

Gln Val Asn Ser Ser Phe Glu Asp His Ile Leu Glu Asp Leu Cys Gly
            580                 585                 590

Met Leu Ser Leu Pro Trp Ile Tyr Ser His Ser Asp Asp Gly Cys Leu
        595                 600                 605

Lys Leu Thr Thr Phe Ala Ala Asn Leu Leu Thr Leu Ser Cys Arg Ile
    610                 615                 620

Ser Asp Ser Tyr Ser Pro Gln Ala Gln Ser Arg Cys Val Phe Leu Leu
625                 630                 635                 640

Thr Leu Phe Pro Arg Arg Ile Phe Leu Glu Trp Arg Thr Ala Val Tyr
                645                 650                 655

Asn Trp Ala Leu Gln Ser Ser His Glu Val Ile Arg Ala Ser Cys Val
            660                 665                 670

Ser Gly Phe Phe Ile Leu Leu Gln Gln Gln Asn Ser Cys Asn Arg Val
        675                 680                 685

Pro Lys Ile Leu Ile Asp Lys Val Lys Asp Asp Ser Asp Ile Val Lys
    690                 695                 700

Lys Glu Phe Ala Ser Ile Leu Gly Gln Leu Val Cys Thr Leu His Gly
705                 710                 715                 720

Met Phe Tyr Leu Thr Ser Ser Leu Thr Glu Pro Phe Ser Glu His Gly
                725                 730                 735

His Val Asp Leu Phe Cys Arg Asn Leu Lys Ala Thr Ser Gln His Glu
            740                 745                 750

Cys Ser Ser Ser Gln Leu Lys Ala Ser Val Cys Lys Pro Phe Leu Phe

755                 760                 765

Leu Leu Lys Lys Lys Ile Pro Ser Pro Val Lys Leu Ala Phe Ile Asp
    770                 775                 780

Asn Leu His His Leu Cys Lys His Leu Asp Phe Arg Glu Asp Glu Thr
785                 790                 795                 800

Asp Val Lys Ala Val Leu Gly Thr Leu Leu Asn Leu Met Glu Asp Pro
                805                 810                 815

Asp Lys Asp Val Arg Val Ala Phe Ser Gly Asn Ile Lys His Ile Leu
            820                 825                 830

Glu Ser Leu Asp Ser Glu Asp Gly Phe Ile Lys Glu Leu Phe Val Leu
        835                 840                 845

Arg Met Lys Glu Ala Tyr Thr His Ala Gln Ile Ser Arg Asn Asn Glu
    850                 855                 860

Leu Lys Asp Thr Leu Ile Leu Thr Thr Gly Asp Ile Gly Arg Ala Ala
865                 870                 875                 880

Lys Gly Asp Leu Val Pro Phe Ala Leu Leu His Leu Leu His Cys Leu
                885                 890                 895

Leu Ser Lys Ser Ala Ser Val Ser Gly Ala Ala Tyr Thr Glu Ile Arg
            900                 905                 910

Ala Leu Val Ala Ala Lys Ser Val Lys Leu Gln Ser Phe Phe Ser Gln
        915                 920                 925

Tyr Lys Lys Pro Ile Cys Gln Phe Leu Val Glu Ser Leu His Ser Ser
    930                 935                 940

Gln Met Thr Ala Leu Pro Asn Thr Pro Cys Gln Asn Ala Asp Val Arg
945                 950                 955                 960

Lys Gln Asp Val Ala His Gln Arg Glu Met Ala Leu Asn Thr Leu Ser
                965                 970                 975

Glu Ile Ala Asn Val Phe Asp Phe Pro Asp Leu Asn Arg Phe Leu Thr
            980                 985                 990

Arg Thr Leu Gln Val Leu Leu Pro  Asp Leu Ala Ala Lys  Ala Ser Pro
        995                 1000                1005

Ala Ala  Ser Ala Leu Ile Arg  Thr Leu Gly Lys Gln  Leu Asn Val
    1010                 1015                 1020

Asn Arg  Arg Glu Ile Leu Ile  Asn Asn Phe Lys Tyr  Ile Phe Ser
    1025                 1030                 1035

His Leu  Val Cys Ser Cys Ser  Lys Asp Glu Leu Glu  Arg Ala Leu
    1040                 1045                 1050

His Tyr  Leu Lys Asn Glu Thr  Glu Ile Glu Leu Gly  Ser Leu Leu
    1055                 1060                 1065

Arg Gln  Asp Phe Gln Gly Leu  His Asn Glu Leu Leu  Leu Arg Ile
    1070                 1075                 1080

Gly Glu  His Tyr Gln Gln Val  Phe Asn Gly Leu Ser  Ile Leu Ala
    1085                 1090                 1095

Ser Phe  Ala Ser Ser Asp Asp  Pro Tyr Gln Gly Pro  Arg Asp Ile
    1100                 1105                 1110

Ile Ser  Pro Glu Leu Met Ala  Asp Tyr Leu Gln Pro  Lys Leu Leu
    1115                 1120                 1125

Gly Ile  Leu Ala Phe Phe Asn  Met Gln Leu Leu Ser  Ser Ser Val
    1130                 1135                 1140

Gly Ile  Glu Asp Lys Lys Met  Ala Leu Asn Ser Leu  Met Ser Leu
    1145                 1150                 1155

Met Lys  Leu Met Gly Pro Lys  His Val Ser Ser Val  Arg Val Lys
    1160                 1165                 1170

Met Met  Thr Thr Leu Arg Thr  Gly Leu Arg Phe Lys  Asp Asp Phe
    1175                 1180                 1185

Pro Glu  Leu Cys Cys Arg Ala  Trp Asp Cys Phe Val  Arg Cys Leu
    1190                 1195                 1200

Asp His  Ala Cys Leu Gly Ser  Leu Leu Ser His Val  Ile Val Ala
    1205                 1210                 1215

Leu Leu  Pro Leu Ile His Ile  Gln Pro Lys Glu Thr  Ala Ala Ile
    1220                 1225                 1230

Phe His Tyr Leu Ile Ile Glu Asn Arg Asp Ala Val Gln Asp Phe
    1235                1240                1245

Leu His Glu Ile Tyr Phe Leu Pro Asp His Pro Glu Leu Lys Lys
    1250                1255                1260

Ile Lys Ala Val Leu Gln Glu Tyr Arg Lys Glu Thr Ser Glu Ser
    1265                1270                1275

Thr Asp Leu Gln Thr Thr Leu Gln Leu Ser Met Lys Ala Ile Gln
    1280                1285                1290

His Glu Asn Val Asp Val Arg Ile His Ala Leu Thr Ser Leu Lys
    1295                1300                1305

Glu Thr Leu Tyr Lys Asn Gln Glu Lys Leu Ile Lys Tyr Ala Thr
    1310                1315                1320

Asp Ser Glu Thr Val Glu Pro Ile Ile Ser Gln Leu Val Thr Val
    1325                1330                1335

Leu Leu Lys Gly Cys Gln Asp Ala Asn Ser Gln Ala Arg Leu Leu
    1340                1345                1350

Cys Gly Glu Cys Leu Gly Glu Leu Gly Ala Ile Asp Pro Gly Arg
    1355                1360                1365

Leu Asp Phe Ser Thr Thr Glu Thr Gln Gly Lys Asp Phe Thr Phe
    1370                1375                1380

Val Thr Gly Val Glu Asp Ser Ser Phe Ala Tyr Gly Leu Leu Met
    1385                1390                1395

Glu Leu Thr Arg Ala Tyr Leu Ala Tyr Ala Asp Asn Ser Arg Ala
    1400                1405                1410

Gln Asp Ser Ala Ala Tyr Ala Ile Gln Glu Leu Leu Ser Ile Tyr
    1415                1420                1425

Asp Cys Arg Glu Met Glu Thr Asn Gly Pro Gly His Gln Leu Trp
    1430                1435                1440

Arg Arg Phe Pro Glu His Val Arg Glu Ile Leu Glu Pro His Leu
    1445                1450                1455

Asn Thr Arg Tyr Lys Ser Ser Gln Lys Ser Thr Asp Trp Ser Gly
    1460                1465                1470

Val Lys Lys Pro Ile Tyr Leu Ser Lys Leu Gly Ser Asn Phe Ala
    1475                1480                1485

Glu Trp Ser Ala Ser Trp Ala Gly Tyr Leu Ile Thr Lys Val Arg
    1490                1495                1500

His Asp Leu Ala Ser Lys Ile Phe Thr Cys Cys Ser Ile Met Met
    1505                1510                1515

Lys His Asp Phe Lys Val Thr Ile Tyr Leu Leu Pro His Ile Leu
    1520                1525                1530

Val Tyr Val Leu Leu Gly Cys Asn Gln Glu Asp Gln Gln Glu Val
    1535                1540                1545

Tyr Ala Glu Ile Met Ala Val Leu Lys His Asp Asp Gln His Thr
    1550                1555                1560

Ile Asn Thr Gln Asp Ile Ala Ser Asp Leu Cys Gln Leu Ser Thr
    1565                1570                1575

Gln Thr Val Phe Ser Met Leu Asp His Leu Thr Gln Trp Ala Arg
    1580                1585                1590

His Lys Phe Gln Ala Leu Lys Ala Glu Lys Cys Pro His Ser Lys
    1595                1600                1605

Ser Asn Arg Asn Lys Val Asp Ser Met Val Ser Thr Val Asp Tyr
    1610                1615                1620

Glu Asp Tyr Gln Ser Val Thr Arg Phe Leu Asp Leu Ile Pro Gln
    1625                1630                1635

Asp Thr Leu Ala Val Ala Ser Phe Arg Ser Lys Ala Tyr Thr Arg
    1640                1645                1650

Ala Val Met His Phe Glu Ser Phe Ile Thr Glu Lys Lys Gln Asn
    1655                1660                1665

Ile Gln Glu His Leu Gly Phe Leu Gln Lys Leu Tyr Ala Ala Met
    1670                1675                1680

His Glu Pro Asp Gly Val Ala Gly Val Ser Ala Ile Arg Lys Ala

1685                 1690                 1695

Glu Pro Ser Leu Lys Glu Gln Ile Leu Glu His Glu Ser Leu Gly
    1700                 1705                 1710

Leu Leu Arg Asp Ala Thr Ala Cys Tyr Asp Arg Ala Ile Gln Leu
    1715                 1720                 1725

Glu Pro Asp Gln Ile Ile His Tyr His Gly Val Val Lys Ser Met
    1730                 1735                 1740

Leu Gly Leu Gly Gln Leu Ser Thr Val Ile Thr Gln Val Asn Gly
    1745                 1750                 1755

Val His Ala Asn Arg Ser Glu Trp Thr Asp Glu Leu Asn Thr Tyr
    1760                 1765                 1770

Arg Val Glu Ala Ala Trp Lys Leu Ser Gln Trp Asp Leu Val Glu
    1775                 1780                 1785

Asn Tyr Leu Ala Ala Asp Gly Lys Ser Thr Thr Trp Ser Val Arg
    1790                 1795                 1800

Leu Gly Gln Leu Leu Leu Ser Ala Lys Lys Arg Asp Ile Thr Ala
    1805                 1810                 1815

Phe Tyr Asp Ser Leu Lys Leu Val Arg Ala Glu Gln Ile Val Pro
    1820                 1825                 1830

Leu Ser Ala Ala Ser Phe Glu Arg Gly Ser Tyr Gln Arg Gly Tyr
    1835                 1840                 1845

Glu Tyr Ile Val Arg Leu His Met Leu Cys Glu Leu Glu His Ser
    1850                 1855                 1860

Ile Lys Pro Leu Phe Gln His Ser Pro Gly Asp Ser Ser Gln Glu
    1865                 1870                 1875

Asp Ser Leu Asn Trp Val Ala Arg Leu Glu Met Thr Gln Asn Ser
    1880                 1885                 1890

Tyr Arg Ala Lys Glu Pro Ile Leu Ala Leu Arg Arg Ala Leu Leu
    1895                 1900                 1905

Ser Leu Asn Lys Arg Pro Asp Tyr Asn Glu Met Val Gly Glu Cys
    1910                 1915                 1920

Trp Leu Gln Ser Ala Arg Val Ala Arg Lys Ala Gly His His Gln
    1925                1930                1935

Thr Ala Tyr Asn Ala Leu Leu Asn Ala Gly Glu Ser Arg Leu Ala
    1940                1945                1950

Glu Leu Tyr Val Glu Arg Ala Lys Trp Leu Trp Ser Lys Gly Asp
    1955                1960                1965

Val His Gln Ala Leu Ile Val Leu Gln Lys Gly Val Glu Leu Cys
    1970                1975                1980

Phe Pro Glu Asn Glu Thr Pro Pro Glu Gly Lys Asn Met Leu Ile
    1985                1990                1995

His Gly Arg Ala Met Leu Leu Val Gly Arg Phe Met Glu Glu Thr
    2000                2005                2010

Ala Asn Phe Glu Ser Asn Ala Ile Met Lys Lys Tyr Lys Asp Val
    2015                2020                2025

Thr Ala Cys Leu Pro Glu Trp Glu Asp Gly His Phe Tyr Leu Ala
    2030                2035                2040

Lys Tyr Tyr Asp Lys Leu Met Pro Met Val Thr Asp Asn Lys Met
    2045                2050                2055

Glu Lys Gln Gly Asp Leu Ile Arg Tyr Ile Val Leu His Phe Gly
    2060                2065                2070

Arg Ser Leu Gln Tyr Gly Asn Gln Phe Ile Tyr Gln Ser Met Pro
    2075                2080                2085

Arg Met Leu Thr Leu Trp Leu Asp Tyr Gly Thr Lys Ala Tyr Glu
    2090                2095                2100

Trp Glu Lys Ala Gly Arg Ser Asp Arg Val Gln Met Arg Asn Asp
    2105                2110                2115

Leu Gly Lys Ile Asn Lys Val Ile Thr Glu His Thr Asn Tyr Leu
    2120                2125                2130

Ala Pro Tyr Gln Phe Leu Thr Ala Phe Ser Gln Leu Ile Ser Arg
    2135                2140                2145

Ile Cys His Ser His Asp Glu Val Phe Val Val Leu Met Glu Ile
    2150                2155                2160

Ile Ala Lys Val Phe Leu Ala Tyr Pro Gln Gln Ala Met Trp Met
    2165                2170                2175

Met Thr Ala Val Ser Lys Ser Ser Tyr Pro Met Arg Val Asn Arg
    2180                2185                2190

Cys Lys Glu Ile Leu Asn Lys Ala Ile His Met Lys Lys Ser Leu
    2195                2200                2205

Glu Lys Phe Val Gly Asp Ala Thr Arg Leu Thr Asp Lys Leu Leu
    2210                2215                2220

Glu Leu Cys Asn Lys Pro Val Asp Gly Ser Ser Ser Thr Leu Ser
    2225                2230                2235

Met Ser Thr His Phe Lys Met Leu Lys Lys Leu Val Glu Glu Ala
    2240                2245                2250

Thr Phe Ser Glu Ile Leu Ile Pro Leu Gln Ser Val Met Ile Pro
    2255                2260                2265

Thr Leu Pro Ser Ile Leu Gly Thr His Ala Asn His Ala Ser His
    2270                2275                2280

Glu Pro Phe Pro Gly His Trp Ala Tyr Ile Ala Gly Phe Asp Asp
    2285                2290                2295

Met Val Glu Ile Leu Ala Ser Leu Gln Lys Pro Lys Lys Ile Ser
    2300                2305                2310

Leu Lys Gly Ser Asp Gly Lys Phe Tyr Ile Met Met Cys Lys Pro
    2315                2320                2325

Lys Asp Asp Leu Arg Lys Asp Cys Arg Leu Met Glu Phe Asn Ser
    2330                2335                2340

Leu Ile Asn Lys Cys Leu Arg Lys Asp Ala Glu Ser Arg Arg Arg
    2345                2350                2355

Glu Leu His Ile Arg Thr Tyr Ala Val Ile Pro Leu Asn Asp Glu
    2360                2365                2370

Cys Gly Ile Ile Glu Trp Val Asn Asn Thr Ala Gly Leu Arg Pro
    2375                2380                2385

Ile Leu Thr Lys Leu Tyr Lys Glu Lys Gly Val Tyr Met Thr Gly
    2390                2395                2400

Lys Glu Leu Arg Gln Cys Met Leu Pro Lys Ser Ala Ala Leu Ser
    2405                2410                2415

Glu Lys Leu Lys Val Phe Arg Glu Phe Leu Leu Pro Arg His Pro
    2420                2425                2430

Pro Ile Phe His Glu Trp Phe Leu Arg Thr Phe Pro Asp Pro Thr
    2435                2440                2445

Ser Trp Tyr Ser Ser Arg Ser Ala Tyr Cys Arg Ser Thr Ala Val
    2450                2455                2460

Met Ser Met Val Gly Tyr Ile Leu Gly Leu Gly Asp Arg His Gly
    2465                2470                2475

Glu Asn Ile Leu Phe Asp Ser Leu Thr Gly Glu Cys Val His Val
    2480                2485                2490

Asp Phe Asn Cys Leu Phe Asn Lys Gly Glu Thr Phe Glu Val Pro
    2495                2500                2505

Glu Ile Val Pro Phe Arg Leu Thr His Asn Met Val Asn Gly Met
    2510                2515                2520

Gly Pro Met Gly Thr Glu Gly Leu Phe Arg Arg Ala Cys Glu Val
    2525                2530                2535

Thr Met Arg Leu Met Arg Asp Gln Arg Glu Pro Leu Met Ser Val
    2540                2545                2550

Leu Lys Thr Phe Leu His Asp Pro Leu Val Glu Trp Ser Lys Pro
    2555                2560                2565

Val Lys Gly His Ser Lys Ala Pro Leu Asn Glu Thr Gly Glu Val
    2570                2575                2580

Val Asn Glu Lys Ala Lys Thr His Val Leu Asp Ile Glu Gln Arg
    2585                2590                2595

Leu Gln Gly Val Ile Lys Thr Arg Asn Arg Val Thr Gly Leu Pro

2600                 2605                 2610

Leu Ser Ile Glu Gly His Val His Tyr Leu Ile Gln Glu Ala Thr
    2615                 2620                 2625

Asp Glu Asn Leu Leu Cys Gln Met Tyr Leu Gly Trp Thr Pro Tyr
    2630                 2635                 2640

Met

<210> 7
<211> 3056
<212> PRT
<213> Homo sapiens

<400> 7

Met Ser Leu Val Leu Asn Asp Leu Leu Ile Cys Cys Arg Gln Leu Glu
1               5                   10                  15

His Asp Arg Ala Thr Glu Arg Lys Lys Glu Val Glu Lys Phe Lys Arg
            20                  25                  30

Leu Ile Arg Asp Pro Glu Thr Ile Lys His Leu Asp Arg His Ser Asp
        35                  40                  45

Ser Lys Gln Gly Lys Tyr Leu Asn Trp Asp Ala Val Phe Arg Phe Leu
    50                  55                  60

Gln Lys Tyr Ile Gln Lys Glu Thr Glu Cys Leu Arg Ile Ala Lys Pro
65                  70                  75                  80

Asn Val Ser Ala Ser Thr Gln Ala Ser Arg Gln Lys Lys Met Gln Glu
                85                  90                  95

Ile Ser Ser Leu Val Lys Tyr Phe Ile Lys Cys Ala Asn Arg Arg Ala
            100                 105                 110

Pro Arg Leu Lys Cys Gln Glu Leu Leu Asn Tyr Ile Met Asp Thr Val
        115                 120                 125

Lys Asp Ser Ser Asn Gly Ala Ile Tyr Gly Ala Asp Cys Ser Asn Ile
    130                 135                 140

Leu Leu Lys Asp Ile Leu Ser Val Arg Lys Tyr Trp Cys Glu Ile Ser
145                 150                 155                 160

Gln Gln Gln Trp Leu Glu Leu Phe Ser Val Tyr Phe Arg Leu Tyr Leu
                165                 170                 175

Lys Pro Ser Gln Asp Val His Arg Val Leu Val Ala Arg Ile Ile His
            180                 185                 190

Ala Val Thr Lys Gly Cys Cys Ser Gln Thr Asp Gly Leu Asn Ser Lys
        195                 200                 205

Phe Leu Asp Phe Phe Ser Lys Ala Ile Gln Cys Ala Arg Gln Glu Lys
    210                 215                 220

Ser Ser Ser Gly Leu Asn His Ile Leu Ala Ala Leu Thr Ile Phe Leu
225                 230                 235                 240

Lys Thr Leu Ala Val Asn Phe Arg Ile Arg Val Cys Glu Leu Gly Asp
                245                 250                 255

Glu Ile Leu Pro Thr Leu Leu Tyr Ile Trp Thr Gln His Arg Leu Asn
            260                 265                 270

Asp Ser Leu Lys Glu Val Ile Ile Glu Leu Phe Gln Leu Gln Ile Tyr
        275                 280                 285

Ile His His Pro Lys Gly Ala Lys Thr Gln Glu Lys Gly Ala Tyr Glu
    290                 295                 300

Ser Thr Lys Trp Arg Ser Ile Leu Tyr Asn Leu Tyr Asp Leu Leu Val
305                 310                 315                 320

Asn Glu Ile Ser His Ile Gly Ser Arg Gly Lys Tyr Ser Ser Gly Phe
                325                 330                 335

Arg Asn Ile Ala Val Lys Glu Asn Leu Ile Glu Leu Met Ala Asp Ile
            340                 345                 350

Cys His Gln Val Phe Asn Glu Asp Thr Arg Ser Leu Glu Ile Ser Gln
        355                 360                 365

Ser Tyr Thr Thr Thr Gln Arg Glu Ser Ser Asp Tyr Ser Val Pro Cys
    370                 375                 380

Lys Arg Lys Lys Ile Glu Leu Gly Trp Glu Val Ile Lys Asp His Leu
385                 390                 395                 400

Gln Lys Ser Gln Asn Asp Phe Asp Leu Val Pro Trp Leu Gln Ile Ala
                405                 410                 415

Thr Gln Leu Ile Ser Lys Tyr Pro Ala Ser Leu Pro Asn Cys Glu Leu
            420                 425                 430

Ser Pro Leu Leu Met Ile Leu Ser Gln Leu Leu Pro Gln Gln Arg His
        435                 440                 445

Gly Glu Arg Thr Pro Tyr Val Leu Arg Cys Leu Thr Glu Val Ala Leu
    450                 455                 460

Cys Gln Asp Lys Arg Ser Asn Leu Glu Ser Ser Gln Lys Ser Asp Leu
465                 470                 475                 480

Leu Lys Leu Trp Asn Lys Ile Trp Cys Ile Thr Phe Arg Gly Ile Ser
                485                 490                 495

Ser Glu Gln Ile Gln Ala Glu Asn Phe Gly Leu Leu Gly Ala Ile Ile
            500                 505                 510

Gln Gly Ser Leu Val Glu Val Asp Arg Glu Phe Trp Lys Leu Phe Thr
        515                 520                 525

Gly Ser Ala Cys Arg Pro Ser Cys Pro Ala Val Cys Cys Leu Thr Leu
    530                 535                 540

Ala Leu Thr Thr Ser Ile Val Pro Gly Thr Val Lys Met Gly Ile Glu
545                 550                 555                 560

Gln Asn Met Cys Glu Val Asn Arg Ser Phe Ser Leu Lys Glu Ser Ile
                565                 570                 575

Met Lys Trp Leu Leu Phe Tyr Gln Leu Glu Gly Asp Leu Glu Asn Ser
            580                 585                 590

Thr Glu Val Pro Pro Ile Leu His Ser Asn Phe Pro His Leu Val Leu
        595                 600                 605

Glu Lys Ile Leu Val Ser Leu Thr Met Lys Asn Cys Lys Ala Ala Met
    610                 615                 620

Asn Phe Phe Gln Ser Val Pro Glu Cys Glu His His Gln Lys Asp Lys
625                 630                 635                 640

Glu Glu Leu Ser Phe Ser Glu Val Glu Glu Leu Phe Leu Gln Thr Thr

                645                 650                 655

Phe Asp Lys Met Asp Phe Leu Thr Ile Val Arg Glu Cys Gly Ile Glu
            660                 665                 670

Lys His Gln Ser Ser Ile Gly Phe Ser Val His Gln Asn Leu Lys Glu
        675                 680                 685

Ser Leu Asp Arg Cys Leu Leu Gly Leu Ser Glu Gln Leu Leu Asn Asn
    690                 695                 700

Tyr Ser Ser Glu Ile Thr Asn Ser Glu Thr Leu Val Arg Cys Ser Arg
705                 710                 715                 720

Leu Leu Val Gly Val Leu Gly Cys Tyr Cys Tyr Met Gly Val Ile Ala
                725                 730                 735

Glu Glu Glu Ala Tyr Lys Ser Glu Leu Phe Gln Lys Ala Lys Ser Leu
            740                 745                 750

Met Gln Cys Ala Gly Glu Ser Ile Thr Leu Phe Lys Asn Lys Thr Asn
        755                 760                 765

Glu Glu Phe Arg Ile Gly Ser Leu Arg Asn Met Met Gln Leu Cys Thr
    770                 775                 780

Arg Cys Leu Ser Asn Cys Thr Lys Lys Ser Pro Asn Lys Ile Ala Ser
785                 790                 795                 800

Gly Phe Phe Leu Arg Leu Leu Thr Ser Lys Leu Met Asn Asp Ile Ala
                805                 810                 815

Asp Ile Cys Lys Ser Leu Ala Ser Phe Ile Lys Lys Pro Phe Asp Arg
            820                 825                 830

Gly Glu Val Glu Ser Met Glu Asp Asp Thr Asn Gly Asn Leu Met Glu
        835                 840                 845

Val Glu Asp Gln Ser Ser Met Asn Leu Phe Asn Asp Tyr Pro Asp Ser
    850                 855                 860

Ser Val Ser Asp Ala Asn Glu Pro Gly Glu Ser Gln Ser Thr Ile Gly
865                 870                 875                 880

Ala Ile Asn Pro Leu Ala Glu Glu Tyr Leu Ser Lys Gln Asp Leu Leu
                885                 890                 895

Phe Leu Asp Met Leu Lys Phe Leu Cys Leu Cys Val Thr Thr Ala Gln
            900                 905                 910

Thr Asn Thr Val Ser Phe Arg Ala Ala Asp Ile Arg Arg Lys Leu Leu
        915                 920                 925

Met Leu Ile Asp Ser Ser Thr Leu Glu Pro Thr Lys Ser Leu His Leu
    930                 935                 940

His Met Tyr Leu Met Leu Leu Lys Glu Leu Pro Gly Glu Glu Tyr Pro
945                 950                 955                 960

Leu Pro Met Glu Asp Val Leu Glu Leu Leu Lys Pro Leu Ser Asn Val
                965                 970                 975

Cys Ser Leu Tyr Arg Arg Asp Gln Asp Val Cys Lys Thr Ile Leu Asn
            980                 985                 990

His Val Leu His Val Val Lys Asn Leu Gly Gln Ser Asn Met Asp Ser
        995                 1000                1005

Glu Asn Thr Arg Asp Ala Gln Gly Gln Phe Leu Thr Val Ile Gly
    1010                1015                1020

Ala Phe Trp His Leu Thr Lys Glu Arg Lys Tyr Ile Phe Ser Val
    1025                1030                1035

Arg Met Ala Leu Val Asn Cys Leu Lys Thr Leu Leu Glu Ala Asp
    1040                1045                1050

Pro Tyr Ser Lys Trp Ala Ile Leu Asn Val Met Gly Lys Asp Phe
    1055                1060                1065

Pro Val Asn Glu Val Phe Thr Gln Phe Leu Ala Asp Asn His His
    1070                1075                1080

Gln Val Arg Met Leu Ala Ala Glu Ser Ile Asn Arg Leu Phe Gln
    1085                1090                1095

Asp Thr Lys Gly Asp Ser Ser Arg Leu Leu Lys Ala Leu Pro Leu
    1100                1105                1110

Lys Leu Gln Gln Thr Ala Phe Glu Asn Ala Tyr Leu Lys Ala Gln
    1115                1120                1125

Glu Gly Met Arg Glu Met Ser His Ser Ala Glu Asn Pro Glu Thr
    1130                1135                1140

Leu Asp Glu Ile Tyr Asn Arg Lys Ser Val Leu Leu Thr Leu Ile
    1145                1150                1155

Ala Val Val Leu Ser Cys Ser Pro Ile Cys Glu Lys Gln Ala Leu
    1160                1165                1170

Phe Ala Leu Cys Lys Ser Val Lys Glu Asn Gly Leu Glu Pro His
    1175                1180                1185

Leu Val Lys Lys Val Leu Glu Lys Val Ser Glu Thr Phe Gly Tyr
    1190                1195                1200

Arg Arg Leu Glu Asp Phe Met Ala Ser His Leu Asp Tyr Leu Val
    1205                1210                1215

Leu Glu Trp Leu Asn Leu Gln Asp Thr Glu Tyr Asn Leu Ser Ser
    1220                1225                1230

Phe Pro Phe Ile Leu Leu Asn Tyr Thr Asn Ile Glu Asp Phe Tyr
    1235                1240                1245

Arg Ser Cys Tyr Lys Val Leu Ile Pro His Leu Val Ile Arg Ser
    1250                1255                1260

His Phe Asp Glu Val Lys Ser Ile Ala Asn Gln Ile Gln Glu Asp
    1265                1270                1275

Trp Lys Ser Leu Leu Thr Asp Cys Phe Pro Lys Ile Leu Val Asn
    1280                1285                1290

Ile Leu Pro Tyr Phe Ala Tyr Glu Gly Thr Arg Asp Ser Gly Met
    1295                1300                1305

Ala Gln Gln Arg Glu Thr Ala Thr Lys Val Tyr Asp Met Leu Lys
    1310                1315                1320

Ser Glu Asn Leu Leu Gly Lys Gln Ile Asp His Leu Phe Ile Ser
    1325                1330                1335

Asn Leu Pro Glu Ile Val Val Glu Leu Leu Met Thr Leu His Glu
    1340                1345                1350

Pro Ala Asn Ser Ser Ala Ser Gln Ser Thr Asp Leu Cys Asp Phe
    1355                1360                1365

Ser Gly Asp Leu Asp Pro Ala Pro Asn Pro Pro His Phe Pro Ser
    1370                1375                1380

His Val Ile Lys Ala Thr Phe Ala Tyr Ile Ser Asn Cys His Lys
    1385                1390                1395

Thr Lys Leu Lys Ser Ile Leu Glu Ile Leu Ser Lys Ser Pro Asp
    1400                1405                1410

Ser Tyr Gln Lys Ile Leu Leu Ala Ile Cys Glu Gln Ala Ala Glu
    1415                1420                1425

Thr Asn Asn Val Tyr Lys Lys His Arg Ile Leu Lys Ile Tyr His
    1430                1435                1440

Leu Phe Val Ser Leu Leu Leu Lys Asp Ile Lys Ser Gly Leu Gly
    1445                1450                1455

Gly Ala Trp Ala Phe Val Leu Arg Asp Val Ile Tyr Thr Leu Ile
    1460                1465                1470

His Tyr Ile Asn Gln Arg Pro Ser Cys Ile Met Asp Val Ser Leu
    1475                1480                1485

Arg Ser Phe Ser Leu Cys Cys Asp Leu Leu Ser Gln Val Cys Gln
    1490                1495                1500

Thr Ala Val Thr Tyr Cys Lys Asp Ala Leu Glu Asn His Leu His
    1505                1510                1515

Val Ile Val Gly Thr Leu Ile Pro Leu Val Tyr Glu Gln Val Glu
    1520                1525                1530

Val Gln Lys Gln Val Leu Asp Leu Leu Lys Tyr Leu Val Ile Asp
    1535                1540                1545

Asn Lys Asp Asn Glu Asn Leu Tyr Ile Thr Ile Lys Leu Leu Asp
    1550                1555                1560

Pro Phe Pro Asp His Val Val Phe Lys Asp Leu Arg Ile Thr Gln
    1565                1570                1575

Gln Lys Ile Lys Tyr Ser Arg Gly Pro Phe Ser Leu Leu Glu Glu

1580                1585                1590

Ile Asn His Phe Leu Ser Val Ser Val Tyr Asp Ala Leu Pro Leu
    1595                1600                1605

Thr Arg Leu Glu Gly Leu Lys Asp Leu Arg Arg Gln Leu Glu Leu
    1610                1615                1620

His Lys Asp Gln Met Val Asp Ile Met Arg Ala Ser Gln Asp Asn
    1625                1630                1635

Pro Gln Asp Gly Ile Met Val Lys Leu Val Val Asn Leu Leu Gln
    1640                1645                1650

Leu Ser Lys Met Ala Ile Asn His Thr Gly Glu Lys Glu Val Leu
    1655                1660                1665

Glu Ala Val Gly Ser Cys Leu Gly Glu Val Gly Pro Ile Asp Phe
    1670                1675                1680

Ser Thr Ile Ala Ile Gln His Ser Lys Asp Ala Ser Tyr Thr Lys
    1685                1690                1695

Ala Leu Lys Leu Phe Glu Asp Lys Glu Leu Gln Trp Thr Phe Ile
    1700                1705                1710

Met Leu Thr Tyr Leu Asn Asn Thr Leu Val Glu Asp Cys Val Lys
    1715                1720                1725

Val Arg Ser Ala Ala Val Thr Cys Leu Lys Asn Ile Leu Ala Thr
    1730                1735                1740

Lys Thr Gly His Ser Phe Trp Glu Ile Tyr Lys Met Thr Thr Asp
    1745                1750                1755

Pro Met Leu Ala Tyr Leu Gln Pro Phe Arg Thr Ser Arg Lys Lys
    1760                1765                1770

Phe Leu Glu Val Pro Arg Phe Asp Lys Glu Asn Pro Phe Glu Gly
    1775                1780                1785

Leu Asp Asp Ile Asn Leu Trp Ile Pro Leu Ser Glu Asn His Asp
    1790                1795                1800

Ile Trp Ile Lys Thr Leu Thr Cys Ala Phe Leu Asp Ser Gly Gly
    1805                1810                1815

Thr Lys Cys Glu Ile Leu Gln Leu Leu Lys Pro Met Cys Glu Val
    1820                1825                1830

Lys Thr Asp Phe Cys Gln Thr Val Leu Pro Tyr Leu Ile His Asp
    1835                1840                1845

Ile Leu Leu Gln Asp Thr Asn Glu Ser Trp Arg Asn Leu Leu Ser
    1850                1855                1860

Thr His Val Gln Gly Phe Phe Thr Ser Cys Leu Arg His Phe Ser
    1865                1870                1875

Gln Thr Ser Arg Ser Thr Thr Pro Ala Asn Leu Asp Ser Glu Ser
    1880                1885                1890

Glu His Phe Phe Arg Cys Cys Leu Asp Lys Lys Ser Gln Arg Thr
    1895                1900                1905

Met Leu Ala Val Val Asp Tyr Met Arg Arg Gln Lys Arg Pro Ser
    1910                1915                1920

Ser Gly Thr Ile Phe Asn Asp Ala Phe Trp Leu Asp Leu Asn Tyr
    1925                1930                1935

Leu Glu Val Ala Lys Val Ala Gln Ser Cys Ala Ala His Phe Thr
    1940                1945                1950

Ala Leu Leu Tyr Ala Glu Ile Tyr Ala Asp Lys Lys Ser Met Asp
    1955                1960                1965

Asp Gln Glu Lys Arg Ser Leu Ala Phe Glu Glu Gly Ser Gln Ser
    1970                1975                1980

Thr Thr Ile Ser Ser Leu Ser Glu Lys Ser Lys Glu Glu Thr Gly
    1985                1990                1995

Ile Ser Leu Gln Asp Leu Leu Leu Glu Ile Tyr Arg Ser Ile Gly
    2000                2005                2010

Glu Pro Asp Ser Leu Tyr Gly Cys Gly Gly Gly Lys Met Leu Gln
    2015                2020                2025

Pro Ile Thr Arg Leu Arg Thr Tyr Glu His Glu Ala Met Trp Gly
    2030                2035                2040

Lys Ala Leu Val Thr Tyr Asp Leu Glu Thr Ala Ile Pro Ser Ser
    2045                2050                2055

Thr Arg Gln Ala Gly Ile Ile Gln Ala Leu Gln Asn Leu Gly Leu
    2060                2065                2070

Cys His Ile Leu Ser Val Tyr Leu Lys Gly Leu Asp Tyr Glu Asn
    2075                2080                2085

Lys Asp Trp Cys Pro Glu Leu Glu Glu Leu His Tyr Gln Ala Ala
    2090                2095                2100

Trp Arg Asn Met Gln Trp Asp His Cys Thr Ser Val Ser Lys Glu
    2105                2110                2115

Val Glu Gly Thr Ser Tyr His Glu Ser Leu Tyr Asn Ala Leu Gln
    2120                2125                2130

Ser Leu Arg Asp Arg Glu Phe Ser Thr Phe Tyr Glu Ser Leu Lys
    2135                2140                2145

Tyr Ala Arg Val Lys Glu Val Glu Glu Met Cys Lys Arg Ser Leu
    2150                2155                2160

Glu Ser Val Tyr Ser Leu Tyr Pro Thr Leu Ser Arg Leu Gln Ala
    2165                2170                2175

Ile Gly Glu Leu Glu Ser Ile Gly Glu Leu Phe Ser Arg Ser Val
    2180                2185                2190

Thr His Arg Gln Leu Ser Glu Val Tyr Ile Lys Trp Gln Lys His
    2195                2200                2205

Ser Gln Leu Leu Lys Asp Ser Asp Phe Ser Phe Gln Glu Pro Ile
    2210                2215                2220

Met Ala Leu Arg Thr Val Ile Leu Glu Ile Leu Met Glu Lys Glu
    2225                2230                2235

Met Asp Asn Ser Gln Arg Glu Cys Ile Lys Asp Ile Leu Thr Lys
    2240                2245                2250

His Leu Val Glu Leu Ser Ile Leu Ala Arg Thr Phe Lys Asn Thr
    2255                2260                2265

Gln Leu Pro Glu Arg Ala Ile Phe Gln Ile Lys Gln Tyr Asn Ser
    2270                2275                2280

Val Ser Cys Gly Val Ser Glu Trp Gln Leu Glu Glu Ala Gln Val
    2285                2290                2295

Phe Trp Ala Lys Lys Glu Gln Ser Leu Ala Leu Ser Ile Leu Lys
    2300                2305                2310

Gln Met Ile Lys Lys Leu Asp Ala Ser Cys Ala Ala Asn Asn Pro
    2315                2320                2325

Ser Leu Lys Leu Thr Tyr Thr Glu Cys Leu Arg Val Cys Gly Asn
    2330                2335                2340

Trp Leu Ala Glu Thr Cys Leu Glu Asn Pro Ala Val Ile Met Gln
    2345                2350                2355

Thr Tyr Leu Glu Lys Ala Val Glu Val Ala Gly Asn Tyr Asp Gly
    2360                2365                2370

Glu Ser Ser Asp Glu Leu Arg Asn Gly Lys Met Lys Ala Phe Leu
    2375                2380                2385

Ser Leu Ala Arg Phe Ser Asp Thr Gln Tyr Gln Arg Ile Glu Asn
    2390                2395                2400

Tyr Met Lys Ser Ser Glu Phe Glu Asn Lys Gln Ala Leu Leu Lys
    2405                2410                2415

Arg Ala Lys Glu Glu Val Gly Leu Leu Arg Glu His Lys Ile Gln
    2420                2425                2430

Thr Asn Arg Tyr Thr Val Lys Val Gln Arg Glu Leu Glu Leu Asp
    2435                2440                2445

Glu Leu Ala Leu Arg Ala Leu Lys Glu Asp Arg Lys Arg Phe Leu
    2450                2455                2460

Cys Lys Ala Val Glu Asn Tyr Ile Asn Cys Leu Leu Ser Gly Glu
    2465                2470                2475

Glu His Asp Met Trp Val Phe Arg Leu Cys Ser Leu Trp Leu Glu
    2480                2485                2490

Asn Ser Gly Val Ser Glu Val Asn Gly Met Met Lys Arg Asp Gly

2495                    2500                    2505

Met Lys Ile Pro Thr Tyr Lys Phe Leu Pro Leu Met Tyr Gln Leu
    2510                    2515                    2520

Ala Ala Arg Met Gly Thr Lys Met Met Gly Gly Leu Gly Phe His
    2525                    2530                    2535

Glu Val Leu Asn Asn Leu Ile Ser Arg Ile Ser Met Asp His Pro
    2540                    2545                    2550

His His Thr Leu Phe Ile Ile Leu Ala Leu Ala Asn Ala Asn Arg
    2555                    2560                    2565

Asp Glu Phe Leu Thr Lys Pro Glu Val Ala Arg Arg Ser Arg Ile
    2570                    2575                    2580

Thr Lys Asn Val Pro Lys Gln Ser Ser Gln Leu Asp Glu Asp Arg
    2585                    2590                    2595

Thr Glu Ala Ala Asn Arg Ile Ile Cys Thr Ile Arg Ser Arg Arg
    2600                    2605                    2610

Pro Gln Met Val Arg Ser Val Glu Ala Leu Cys Asp Ala Tyr Ile
    2615                    2620                    2625

Ile Leu Ala Asn Leu Asp Ala Thr Gln Trp Lys Thr Gln Arg Lys
    2630                    2635                    2640

Gly Ile Asn Ile Pro Ala Asp Gln Pro Ile Thr Lys Leu Lys Asn
    2645                    2650                    2655

Leu Glu Asp Val Val Val Pro Thr Met Glu Ile Lys Val Asp His
    2660                    2665                    2670

Thr Gly Glu Tyr Gly Asn Leu Val Thr Ile Gln Ser Phe Lys Ala
    2675                    2680                    2685

Glu Phe Arg Leu Ala Gly Gly Val Asn Leu Pro Lys Ile Ile Asp
    2690                    2695                    2700

Cys Val Gly Ser Asp Gly Lys Glu Arg Arg Gln Leu Val Lys Gly
    2705                    2710                    2715

Arg Asp Asp Leu Arg Gln Asp Ala Val Met Gln Gln Val Phe Gln
    2720                    2725                    2730

Met Cys Asn Thr Leu Leu Gln Arg Asn Thr Glu Thr Arg Lys Arg
    2735                2740                2745

Lys Leu Thr Ile Cys Thr Tyr Lys Val Val Pro Leu Ser Gln Arg
    2750                2755                2760

Ser Gly Val Leu Glu Trp Cys Thr Gly Thr Val Pro Ile Gly Glu
    2765                2770                2775

Phe Leu Val Asn Asn Glu Asp Gly Ala His Lys Arg Tyr Arg Pro
    2780                2785                2790

Asn Asp Phe Ser Ala Phe Gln Cys Gln Lys Lys Met Met Glu Val
    2795                2800                2805

Gln Lys Lys Ser Phe Glu Glu Lys Tyr Glu Val Phe Met Asp Val
    2810                2815                2820

Cys Gln Asn Phe Gln Pro Val Phe Arg Tyr Phe Cys Met Glu Lys
    2825                2830                2835

Phe Leu Asp Pro Ala Ile Trp Phe Glu Lys Arg Leu Ala Tyr Thr
    2840                2845                2850

Arg Ser Val Ala Thr Ser Ser Ile Val Gly Tyr Ile Leu Gly Leu
    2855                2860                2865

Gly Asp Arg His Val Gln Asn Ile Leu Ile Asn Glu Gln Ser Ala
    2870                2875                2880

Glu Leu Val His Ile Asp Leu Gly Val Ala Phe Glu Gln Gly Lys
    2885                2890                2895

Ile Leu Pro Thr Pro Glu Thr Val Pro Phe Arg Leu Thr Arg Asp
    2900                2905                2910

Ile Val Asp Gly Met Gly Ile Thr Gly Val Glu Gly Val Phe Arg
    2915                2920                2925

Arg Cys Cys Glu Lys Thr Met Glu Val Met Arg Asn Ser Gln Glu
    2930                2935                2940

Thr Leu Leu Thr Ile Val Glu Val Leu Leu Tyr Asp Pro Leu Phe
    2945                2950                2955

Asp Trp Thr Met Asn Pro Leu Lys Ala Leu Tyr Leu Gln Gln Arg
    2960                2965                2970

Pro Glu Asp Glu Thr Glu Leu His Pro Thr Leu Asn Ala Asp Asp
    2975                2980                2985

Gln Glu Cys Lys Arg Asn Leu Ser Asp Ile Asp Gln Ser Phe Asn
    2990                2995                3000

Lys Val Ala Glu Arg Val Leu Met Arg Leu Gln Glu Lys Leu Lys
    3005                3010                3015

Gly Val Glu Glu Gly Thr Val Leu Ser Val Gly Gly Gln Val Asn
    3020                3025                3030

Leu Leu Ile Gln Gln Ala Ile Asp Pro Lys Asn Leu Ser Arg Leu
    3035                3040                3045

Phe Pro Gly Trp Lys Ala Trp Val
    3050                3055

SEQUENCE LISTING

<110>  Niigata TLO Corporation

<120>  ATR inhibitor

<130>  IPY-259

<150>  JP2005-307534
<151>  2005-10-21

<160>  7

<170>  PatentIn version 3.4

<210>  1
<211>  21
<212>  DNA
<213>  Synthetic

<400>  1
aagcgcctga ttcgagatcc t                                                21

<210>  2
<211>  21
<212>  DNA
<213>  Synthetic

<400>  2
aagacggtgt gctcatgcgg c                                                21

<210>  3
<211>  24
<212>  DNA

<213>  Synthetic

<400>  3
cggcaagctg accctgaagt tcat                                              24



<210>  4
<211>  8265
<212>  DNA
<213>  Synthetic

<400>  4
gcctccacac ggctccgtcg ggcgccgcgc tcttccggca gcggtacgtt tggagacgcc
60

gggaacccgc gttggcgtgg ttgactagtg cctcgcagcc tcagcatggg ggaacatggc
120

ctggagctgg cttccatgat ccccgccctg cgggagctgg gcagtgccac accagaggaa
180

tataatacag ttgtacagaa gccaagacaa attctgtgtc aattcattga ccggatactt
240

acagatgtaa atgttgttgc tgtagaactt gtaaagaaaa ctgactctca gccaacctcc
300

gtgatgttgc ttgatttcat ccagcatatc atgaaatcct ccccacttat gtttgtaaat
360

gtgagtggaa gccatgagcg caaaggcagt tgtattgaat tcagtaattg gatcataacg
420

agacttctgc ggattgcagc aactccctcc tgtcatttgt tacacaagaa aatctgtgaa
480

gtcatctgtt cattattatt tctttttaaa agcaagagtc ctgctatttt tggggtactc
540

acaaaagaat tattacaact ttttgaagac ttggtttacc tccatagaag aaatgtgatg
600

ggtcatgctg tggaatggcc agtggtcatg agccgatttt taagtcaatt agatgaacac
660

atgggatatt tacaatcagc tcctttgcag ttgatgagta tgcaaaattt agaatttatt
720

gaagtcactt tattaatggt tcttactcgt attattgcaa ttgtgttttt tagaaggcaa
780

gaactcttac tttggcagat aggttgtgtt ctgctagagt atggtagtcc aaaaattaaa
840

tccctagcaa ttagcttttt aacagaactt tttcagcttg gaggactacc agcacaacca
900

gctagcactt ttttcagctc atttttggaa ttattaaaac accttgtaga aatggatact
960

gaccaattga aactctatga agagccatta tcaaagctga taaagacact atttcccttt
1020

gaagcagaag cttatagaaa tattgaacct gtctatttaa atatgctgct ggaaaaactc
1080

tgtgtcatgt ttgaagacgg tgtgctcatg cggcttaagt ctgatttgct aaaagcagct
1140

ttgtgccatt tactgcagta tttccttaaa tttgtgccag ctgggtatga atctgcttta
1200

caagtcagga aggtctatgt gagaaatatt tgtaaagctc ttttggatgt gcttggaatt
1260
gaggtagatg cagagtactt gttgggccca ctttatgcag ctttgaaaat ggaaagtatg
1320
gaaatcattg aggagattca atgccaaact caacaggaaa acctcagcag taatagtgat
1380
ggaatatcac ccaaaaggcg tcgtctcagc tcgtctctaa acccttctaa aagagcacca
1440
aaacagactg aggaaattaa acatgtggac atgaaccaaa agagcatatt atggagtgca
1500
ctgaaacaga aagctgaatc ccttcagatt tcccttgaat acagtggcct aaagaatcct
1560
gttattgaga tgttagaagg aattgctgtt gtcttacaac tgactgctct gtgtactgtt
1620
cattgttctc atcaaaacat gaactgccgt actttcaagg actgtcaaca taaatccaag
1680
aagaaacctt ctgtagtgat aacttggatg tcattggatt tttacacaaa agtgcttaag
1740
agctgtagaa gtttgttaga atctgttcag aaactggacc tggaggcaac cattgataag
1800
gtggtgaaaa tttatgatgc tttgatttat atgcaagtaa acagttcatt tgaagatcat
1860
atcctggaag atttatgtgg tatgctctca cttccatgga tttattccca ttctgatgat
1920

ggctgtttaa agttgaccac atttgccgct aatcttctaa cattaagctg taggatttca
1980

gatagctatt caccacaggc acaatcacga tgtgtgtttc ttctgactct gtttccaaga
2040

agaatattcc ttgagtggag aacagcagtt tacaactggg ccctgcagag ctcccatgaa
2100

gtaatccggg ctagttgtgt tagtggattt tttatcttat tgcagcagca gaattcttgt
2160

aacagagttc ccaagattct tatagataaa gtcaaagatg attctgacat tgtcaagaaa
2220

gaatttgctt ctatacttgg tcaacttgtc tgtactcttc acggcatgtt ttatctgaca
2280

agttctttaa cagaaccttt ctctgaacac ggacatgtgg acctcttctg taggaacttg
2340

aaagccactt ctcaacatga atgttcatct tctcaactaa aagcttctgt ctgcaagcca
2400

ttccttttcc tactgaaaaa aaaaatacct agtccagtaa aacttgcttt catagataat
2460

ctacatcatc tttgtaagca tcttgatttt agagaagatg aaacagatgt aaaagcagtt
2520

cttggaactt tattaaattt aatggaagat ccagacaaag atgttagagt ggcttttagt
2580

ggaaatatca agcacatatt ggaatccttg gactctgaag atggatttat aaaggagctt
2640

tttgtcttaa gaatgaagga agcatataca catgcccaaa tatcaagaaa taatgagctg
2700

aaggatacct tgattcttac aacaggggat attggaaggg ccgcaaaagg agatttggta
2760

ccatttgcac tcttacactt attgcattgt ttgttatcca agtcagcatc tgtctctgga
2820

gcagcataca cagaaattag agctctggtt gcagctaaaa gtgttaaact gcaaagtttt
2880

ttcagccagt ataagaaacc catctgtcag tttttggtag aatcccttca ctctagtcag
2940

atgacagcac ttccgaatac tccatgccag aatgctgacg tgcgaaaaca agatgtggct
3000

caccagagag aaatggcttt aaatacgttg tctgaaattg ccaacgtttt cgactttcct
3060

gatcttaatc gttttcttac taggacatta caagttctac tacctgatct tgctgccaaa
3120

gcaagccctg cagcttctgc tctcattcga actttaggaa aacaattaaa tgtcaatcgt
3180

agagagattt taataaacaa cttcaaatat attttttctc atttggtctg ttcttgttcc
3240

aaagatgaat tagaacgtgc ccttcattat ctgaagaatg aaacagaaat tgaactgggg
3300

agcctgttga gacaagattt ccaaggattg cataatgaat tattgctgcg tattggagaa
3360

cactatcaac aggtttttaa tggtttgtca atacttgcct catttgcatc cagtgatgat
3420

ccatatcagg gcccgagaga tatcatatca cctgaactga tggctgatta tttacaaccc
3480

aaattgttgg gcattttggc tttttttaac atgcagttac tgagctctag tgttggcatt
3540

gaagataaga aaatggcctt gaacagtttg atgtctttga tgaagttaat gggacccaaa
3600

catgtcagtt ctgtgagggt gaagatgatg accacactga gaactggcct tcgattcaag
3660

gatgattttc ctgaattgtg ttgcagagct tgggactgct ttgttcgctg cctggatcat
3720

gcttgtctgg gctcccttct cagtcatgta atagtagctt tgttacctct tatacacatc
3780

cagcctaaag aaactgcagc tatcttccac tacctcataa ttgaaaacag ggatgctgtg
3840

caagattttc ttcatgaaat atatttttta cctgatcatc cagaattaaa aaagataaaa
3900

gccgttctcc aggaatacag aaaggagacc tctgagagca ctgatcttca gacaactctt
3960

cagctctcta tgaaggccat tcaacatgaa aatgtcgatg ttcgtattca tgctcttaca
4020

agcttgaagg aaaccttgta taaaaatcag gaaaaactga taaagtatgc aacagacagt
4080

gaaacagtag aacctattat ctcacagttg gtgacagtgc ttttgaaagg ttgccaagat
4140

gcaaactctc aagctcggtt gctctgtggg gaatgtttag gggaattggg ggcgatagat
4200

ccaggtcgat tagatttctc aacaactgaa actcaaggaa aagattttac atttgtgact
4260

ggagtagaag attcaagctt tgcctatgga ttattgatgg agctaacaag agcttacctt
4320

gcgtatgctg ataatagccg agctcaagat tcagctgcct atgccattca ggagttgctt
4380

tctatttatg actgtagaga gatggagacc aacggcccag gtcaccaatt gtggaggaga
4440

tttcctgagc atgttcggga aatactagaa cctcatctaa ataccagata caagagttct
4500

cagaagtcaa ccgattggtc tggagtaaag aagccaattt acttaagtaa attgggtagt
4560

aactttgcag aatggtcagc atcttgggca ggttatctta ttacaaaggt tcgacatgat
4620

cttgccagta aaattttcac ctgctgtagc attatgatga agcatgattt caaagtgacc
4680

atctatcttc ttccacatat tctggtgtat gtcttactgg gttgtaatca agaagatcag
4740

caggaggttt atgcagaaat tatggcagtt ctaaagcatg acgatcagca taccataaat
4800

acccaagaca ttgcatctga tctgtgtcaa ctcagtacac agactgtgtt ctccatgctt
4860

gaccatctca cacagtgggc aaggcacaaa tttcaggcac tgaaagctga gaaatgtcca
4920

cacagcaaat caaacagaaa taaggtagac tcaatggtat ctactgtgga ttatgaagac
4980

tatcagagtg taacccgttt tctagacctc ataccccagg atactctggc agtagcttcc
5040

tttcgctcca aagcatacac acgagctgta atgcactttg aatcatttat tacagaaaag
5100

aagcaaaata ttcaggaaca tcttggattt ttacagaaat tgtatgctgc tatgcatgaa
5160

cctgatggag tggccggagt cagtgcaatt agaaaggcag aaccatctct aaaagaacag
5220

atccttgaac atgaaagcct tggcttgctg agggatgcca ctgcttgtta tgacagggct
5280

attcagctag aaccagacca gatcattcat tatcatggtg tagtaaagtc catgttaggt
5340

cttggtcagc tgtctactgt tatcactcag gtgaatggag tgcatgctaa caggtccgag
5400

tggacagatg aattaaacac gtacagagtg gaagcagctt ggaaattgtc acagtgggat
5460

ttggtggaaa actatttggc agcagatgga aaatctacaa catggagtgt cagactggga
5520

cagctattat tatcagccaa aaaaagagat atcacagctt tttatgactc actgaaacta
5580
gtgagagcag aacaaattgt acctctttca gctgcaagct ttgaaagagg ctcctaccaa
5640
cgaggatatg aatatattgt gagattgcac atgttatgtg agttggagca tagcatcaaa
5700
ccacttttcc agcattctcc aggtgacagt tctcaagaag attctctaaa ctgggtagct
5760
cgactagaaa tgacccagaa ttcctacaga gccaaggagc ctatcctggc tctccggagg
5820
gctttactaa gcctcaacaa aagaccagat tacaatgaaa tggttggaga atgctggctg
5880
cagagtgcca gggtagctag aaaggctggt caccaccaga cagcctacaa tgctctcctt
5940
aatgcagggg aatcacgact cgctgaactg tacgtggaaa gggcaaagtg gctctggtcc
6000
aagggtgatg ttcaccaggc actaattgtt cttcaaaaag gtgttgaatt atgttttcct
6060
gaaaatgaaa ccccacctga gggtaagaac atgttaatcc atggtcgagc tatgctacta
6120
gtgggccgat ttatggaaga aacagctaac tttgaaagca atgcaattat gaaaaaatat
6180
aaggatgtga ccgcgtgcct gccagaatgg gaggatgggc atttttacct tgccaagtac
6240

tatgacaaat tgatgcccat ggtcacagac aacaaaatgg aaaagcaagg tgatctcatc
6300

cggtatatag ttcttcattt tggcagatct ctacaatatg gaaatcagtt catatatcag
6360

tcaatgccac gaatgttaac tctatggctt gattatggta caaaggcata tgaatgggaa
6420

aaagctggcc gctccgatcg tgtacaaatg aggaatgatt tgggtaaaat aaacaaggtt
6480

atcacagagc atacaaacta tttagctcca tatcaatttt tgactgcttt ttcacaattg
6540

atctctcgaa tttgtcattc tcacgatgaa gtttttgttg tcttgatgga aataatagcc
6600

aaagtatttc tagcctatcc tcaacaagca atgtggatga tgacagctgt gtcaaagtca
6660

tcttatccca tgcgtgtgaa cagatgcaag gaaatcctca ataaagctat tcatatgaaa
6720

aaatccttag agaagtttgt tggagatgca actcgcctaa cagataagct tctagaattg
6780

tgcaataaac cggttgatgg aagtagttcc acattaagca tgagcactca ttttaaaatg
6840

cttaaaaagc tggtagaaga agcaacattt agtgaaatcc tcattcctct acaatcagtc
6900

atgataccta cacttccatc aattctgggt acccatgcta accatgctag ccatgaacca
6960

tttcctggac attgggccta tattgcaggg tttgatgata tggtggaaat tcttgcttct
7020

cttcagaaac caaagaagat ttctttaaaa ggctcagatg gaaagttcta catcatgatg
7080

tgtaagccaa aagatgacct gagaaaggat tgtagactaa tggaattcaa ttccttgatt
7140

aataagtgct taagaaaaga tgcagagtct cgtagaagag aacttcatat tcgaacatat
7200

gcagttattc cactaaatga tgaatgtggg attattgaat gggtgaacaa cactgctggt
7260

ttgagaccta ttctgaccaa actatataaa gaaaagggag tgtatatgac aggaaaagaa
7320

cttcgccagt gtatgctacc aaagtcagca gctttatctg aaaaactcaa agtattccga
7380

gaatttctcc tgcccaggca tcctcctatt tttcatgagt ggtttctgag aacattccct
7440

gatcctacat catggtacag tagtagatca gcttactgcc gttccactgc agtaatgtca
7500

atggttggtt atattctggg gcttggagac cgtcatggtg aaaatattct ctttgattct
7560

ttgactggtg aatgcgtaca tgtagatttc aattgtcttt tcaataaggg agaaaccttt
7620

gaagttccag aaattgtgcc atttcgcctg actcataata tggttaatgg aatgggtcct
7680

atgggaacag agggtctttt tcgaagagca tgtgaagtta caatgaggct gatgcgtgat
7740

cagcgagagc ctttaatgag tgtcttaaag acttttctac atgatcctct tgtggaatgg
7800

agtaaaccag tgaaagggca ttccaaagcg ccactgaatg aaactggaga agttgtcaat
7860

gaaaaggcca agacccatgt tcttgacatt gagcagcgac tacaaggtgt aatcaagact
7920

cgaaatagag tgacaggact gccgttatct attgaaggac atgtgcatta ccttatacaa
7980

gaagctactg atgaaaactt actatgccag atgtatcttg gttggactcc atatatgtga
8040

aatgaaatta tgtaaaagaa tatgttaata atctaaaagt aatgcatttg gtatgaatct
8100

gtggttgtat ctgttcaatt ctaaagtaca acataaattt acgttctcag caactgttat
8160

ttctctctga tcattaatta tatgtaaaat aatatacatt cagttattaa gaaataaact
8220

gctttcttaa taaaaaaaaa aaaaaaaaaa aaaaaaaaaa aaaaa
8265

<210> 5
<211> 13147
<212> DNA
<213> Homo sapiens

<400> 5
   ccggagcccg agccgaaggg cgagccgcaa acgctaagtc gctggccatt ggtggacatg
   60
   gcgcaggcgc gtttgctccg acgggccgaa tgttttgggg cagtgttttg agcgcggaga
   120
   ccgcgtgata ctggatgcgc atgggcatac cgtgctctgc ggctgcttgg cgttgcttct
   180
   tcctccagaa gtgggcgctg ggcagtcacg cagggtttga accggaagcg ggagtaggta
   240
   gctgcgtggc taacggagaa aagaagccgt ggccgcggga ggaggcgaga ggagtcggga
   300
   tctgcgctgc agccaccgcc gcggttgata ctactttgac cttccgagtg cagtgacagt
   360
   gatgtgtgtt ctgaaattgt gaaccatgag tctagtactt aatgatctgc ttatctgctg
   420
   ccgtcaacta gaacatgata gagctacaga acgaaagaaa gaagttgaga aatttaagcg
   480
   cctgattcga gatcctgaaa caattaaaca tctagatcgg cattcagatt ccaaacaagg
   540
   aaaatatttg aattgggatg ctgtttttag atttttacag aaatatattc agaaagaaac
   600
   agaatgtctg agaatagcaa aaccaaatgt atcagcctca acacaagcct ccaggcagaa
   660
   aaagatgcag gaaatcagta gtttggtcaa atacttcatc aaatgtgcaa acagaagagc
   720
   acctaggcta aaatgtcaag aactcttaaa ttatatcatg gatacagtga aagattcatc
   780
   taatggtgct atttacggag ctgattgtag caacatacta ctcaaagaca ttctttctgt
   840
   gagaaaatac tggtgtgaaa tatctcagca acagtggtta gaattgttct ctgtgtactt
   900
   caggctctat ctgaaacctt cacaagatgt tcatagagtt ttagtggcta gaataattca
   960
   tgctgttacc aaaggatgct gttctcagac tgacggatta aattccaaat ttttggactt
   1020
   tttttccaag gctattcagt gtgcgagaca agaaaagagc tcttcaggtc taaatcatat
   1080
   cttagcagct cttactatct tcctcaagac tttggctgtc aactttcgaa ttcgagtgtg
   1140
   tgaattagga gatgaaattc ttcccacttt gctttatatt tggactcaac ataggcttaa
   1200
   tgattcttta aaagaagtca ttattgaatt atttcaactg caaatttata tccatcatcc
   1260
   gaaaggagcc aaaacccaag aaaaaggtgc ttatgaatca acaaaatgga gaagtatttt
   1320
   atacaactta tatgatctgc tagtgaatga gataagtcat ataggaagta gaggaaagta
   1380
   ttcttcagga tttcgtaata ttgccgtcaa agaaaatttg attgaattga tggcagatat
   1440
   ctgtcaccag gtttttaatg aagataccag atccttggag atttctcaat cttacactac
   1500
   tacacaaaga gaatctagtg attacagtgt cccttgcaaa aggaagaaaa tagaactagg
   1560
   ctgggaagta ataaaagatc accttcagaa gtcacagaat gattttgatc ttgtgccttg
   1620
   gctacagatt gcaacccaat taatatcaaa gtatcctgca agtttaccta actgtgagct
   1680
   gtctccatta ctgatgatac tatctcagct tctaccccaa cagcgacatg gggaacgtac
   1740
   accatatgtg ttacgatgcc ttacggaagt tgcattgtgt caagacaaga ggtcaaacct
   1800
   agaaagctca caaaagtcag atttattaaa actctggaat aaaatttggt gtattacctt
   1860
   tcgtggtata agttctgagc aaatacaagc tgaaaacttt ggcttacttg gagccataat
   1920
   tcagggtagt ttagttgagg ttgacagaga attctggaag ttatttactg ggtcagcctg
   1980
   cagaccttca tgtcctgcag tatgctgttt gactttggca ctgaccacca gtatagttcc
   2040
   aggaacggta aaaatgggaa tagagcaaaa tatgtgtgaa gtaaatagaa gcttttcttt
   2100
   aaaggaatca ataatgaaat ggctcttatt ctatcagtta gagggtgact tagaaaatag
   2160
   cacagaagtg cctccaattc ttcacagtaa ttttcctcat cttgtactgg agaaaattct
   2220
   tgtgagtctc actatgaaaa actgtaaagc tgcaatgaat tttttccaaa gcgtgccaga
   2280
   atgtgaacac caccaaaaag ataaagaaga actttcattc tcagaagtag aagaactatt
   2340
   tcttcagaca acttttgaca agatggactt tttaaccatt gtgagagaat gtggtataga
   2400
   aaagcaccag tccagtattg gcttctctgt ccaccagaat ctcaaggaat cactggatcg
   2460
   ctgtcttctg ggattatcag aacagcttct gaataattac tcatctgaga ttacaaattc
   2520
   agaaactctt gtccggtgtt cacgtctttt ggtgggtgtc cttggctgct actgttacat
   2580
   gggtgtaata gctgaagagg aagcatataa gtcagaatta ttccagaaag ccaagtctct
   2640
   aatgcaatgt gcaggagaaa gtatcactct gtttaaaaat aagacaaatg aggaattcag
   2700
   aattggttcc ttgagaaata tgatgcagct atgtacacgt tgcttgagca actgtaccaa
   2760
   gaagagtcca aataagattg catctggctt tttcctgcga ttgttaacat caaagctaat
   2820
   gaatgacatt gcagatattt gtaaaagttt agcatccttc atcaaaaagc catttgaccg
   2880
   tggagaagta gaatcaatgg aagatgatac taatggaaat ctaatggagg tggaggatca
   2940
   gtcatccatg aatctattta acgattaccc tgatagtagt gttagtgatg caaacgaacc
   3000
   tggagagagc caaagtacca taggtgccat taatccttta gctgaagaat atctgtcaaa
   3060
   gcaagatcta cttttcttag acatgctcaa gttcttgtgt ttgtgtgtaa ctactgctca
   3120
   gaccaatact gtgtccttta gggcagctga tattcggagg aaattgttaa tgttaattga
   3180
   ttctagcacg ctagaaccta ccaaatccct ccacctgcat atgtatctaa tgcttttaaa
   3240
   ggagcttcct ggagaagagt accccttgcc aatggaagat gttcttgaac ttctgaaacc
   3300
   actatccaat gtgtgttctt tgtatcgtcg tgaccaagat gtttgtaaaa ctattttaaa
   3360
   ccatgtcctt catgtagtga aaaacctagg tcaaagcaat atggactctg agaacacaag
   3420
   ggatgctcaa ggacagtttc ttacagtaat tggagcattt tggcatctaa caaaggagag
   3480
   gaaatatata ttctctgtaa gaatggccct agtaaattgc cttaaaactt tgcttgaggc
   3540
   tgatccttat tcaaaatggg ccattcttaa tgtaatggga aaagactttc ctgtaaatga
   3600
   agtatttaca caatttcttg ctgacaatca tcaccaagtt cgcatgttgg ctgcagagtc
   3660
   aatcaataga ttgttccagg acacgaaggg agattcttcc aggttactga aagcacttcc
   3720
   tttgaagctt cagcaaacag cttttgaaaa tgcatacttg aaagctcagg aaggaatgag
   3780
   agaaatgtcc catagtgctg agaaccctga aactttggat gaaatttata atagaaaatc
   3840
   tgttttactg acgttgatag ctgtggtttt atcctgtagc cctatctgcg aaaaacaggc
   3900
   tttgtttgcc ctgtgtaaat ctgtgaaaga gaatggatta gaacctcacc ttgtgaaaaa
   3960
   ggttttagag aaagtttctg aaacttttgg atatagacgt ttagaagact ttatggcatc
   4020
   tcatttagat tatctggttt tggaatggct aaatcttcaa gatactgaat acaacttatc
   4080
   ttcttttcct tttattttat taaactacac aaatattgag gatttctata gatcttgtta
   4140
   taaggttttg attccacatc tggtgattag aagtcatttt gatgaggtga agtccattgc
   4200
   taatcagatt caagaggact ggaaaagtct tctaacagac tgctttccaa agattcttgt
   4260
   aaatattctt ccttattttg cctatgaggg taccagagac agtgggatgg cacagcaaag
   4320
   agagactgct accaaggtct atgatatgct taaaagtgaa aacttattgg gaaaacagat
   4380
   tgatcactta ttcattagta atttaccaga gattgtggtg gagttattga tgacgttaca
   4440
   tgagccagca aattctagtg ccagtcagag cactgacctc tgtgactttt caggggattt
   4500
   ggatcctgct cctaatccac ctcattttcc atcgcatgtg attaaagcaa catttgccta
   4560
   tatcagcaat tgtcataaaa ccaagttaaa aagcatttta gaaattcttt ccaaaagccc
   4620
   tgattcctat cagaaaattc ttcttgccat atgtgagcaa gcagctgaaa caaataatgt
   4680
   ttataagaag cacagaattc ttaaaatata tcacctgttt gttagtttat tactgaaaga
   4740
   tataaaaagt ggcttaggag gagcttgggc ctttgttctt cgagacgtta tttatacttt
   4800
   gattcactat atcaaccaaa ggccttcttg tatcatggat gtgtcattac gtagcttctc
   4860
   cctttgttgt gacttattaa gtcaggtttg ccagacagcc gtgacttact gtaaggatgc
   4920
   tctagaaaac catcttcatg ttattgttgg tacacttata ccccttgtgt atgagcaggt
   4980
   ggaggttcag aaacaggtat tggacttgtt gaaatactta gtgatagata acaaggataa
   5040
   tgaaaacctc tatatcacga ttaagctttt agatcctttt cctgaccatg ttgtttttaa
   5100
   ggatttgcgt attactcagc aaaaaatcaa atacagtaga ggaccctttt cactcttgga
   5160
   ggaaattaac cattttctct cagtaagtgt ttatgatgca cttccattga caagacttga
   5220
   aggactaaag gatcttcgaa gacaactgga actacataaa gatcagatgg tggacattat
   5280
   gagagcttct caggataatc cgcaagatgg gattatggtg aaactagttg tcaatttgtt
   5340
   gcagttatcc aagatggcaa taaaccacac tggtgaaaaa gaagttctag aggctgttgg
   5400
   aagctgcttg ggagaagtgg gtcctataga tttctctacc atagctatac aacatagtaa
   5460
   agatgcatct tataccaagg cccttaagtt atttgaagat aaagaacttc agtggacctt
   5520
   cataatgctg acctacctga ataacacact ggtagaagat tgtgtcaaag ttcgatcagc
   5580
   agctgttacc tgtttgaaaa acattttagc cacaaagact ggacatagtt tctgggagat
   5640
   ttataagatg acaacagatc caatgctggc ctatctacag ccttttagaa catcaagaaa
   5700
   aaagttttta gaagtaccca gatttgacaa agaaaaccct tttgaaggcc tggatgatat
   5760
   aaatctgtgg attcctctaa gtgaaaatca tgacatttgg ataaagacac tgacttgtgc
   5820
   ttttttggac agtggaggca caaaatgtga aattcttcaa ttattaaagc caatgtgtga
   5880
   agtgaaaact gacttttgtc agactgtact tccatacttg attcatgata ttttactcca
   5940
   agatacaaat gaatcatgga gaaatctgct ttctacacat gttcagggat ttttcaccag
   6000
   ctgtcttcga cacttctcgc aaacgagccg atccacaacc cctgcaaact tggattcaga
   6060
   gtcagagcac tttttccgat gctgtttgga taaaaaatca caaagaacaa tgcttgctgt
   6120
   tgtggactac atgagaagac aaaagagacc ttcttcagga acaattttta atgatgcttt
   6180
   ctggctggat ttaaattatc tagaagttgc caaggtagct cagtcttgtg ctgctcactt
   6240
   tacagcttta ctctatgcag aaatctatgc agataagaaa agtatggatg atcaagagaa
   6300
   aagaagtctt gcatttgaag aaggaagcca gagtacaact atttctagct tgagtgaaaa
   6360
   aagtaaagaa gaaactggaa taagtttaca ggatcttctc ttagaaatct acagaagtat
   6420
   aggggagcca gatagtttgt atggctgtgg tggagggaag atgttacaac ccattactag
   6480
   actacgaaca tatgaacacg aagcaatgtg gggcaaagcc ctagtaacat atgacctcga
   6540
   aacagcaatc ccctcatcaa cacgccaggc aggaatcatt caggccttgc agaatttggg
   6600
   actctgccat attctttccg tctatttaaa aggattggat tatgaaaata aagactggtg
   6660
   tcctgaacta gaagaacttc attaccaagc agcatggagg aatatgcagt gggaccattg
   6720
   cacttccgtc agcaaagaag tagaaggaac cagttaccat gaatcattgt acaatgctct
   6780
   acaatctcta agagacagag aattctctac attttatgaa agtctcaaat atgccagagt
   6840
   aaaagaagtg gaagagatgt gtaagcgcag ccttgagtct gtgtattcgc tctatcccac
   6900
   acttagcagg ttgcaggcca ttggagagct ggaaagcatt ggggagcttt tctcaagatc
   6960
   agtcacacat agacaactct ctgaagtata tattaagtgg cagaaacact cccagcttct
   7020
   caaggacagt gattttagtt ttcaggagcc tatcatggct ctacgcacag tcattttgga
   7080
   gatcctgatg gaaaaggaaa tggacaactc acaaagagaa tgtattaagg acattctcac
   7140
   caaacacctt gtagaactct ctatactggc cagaactttc aagaacactc agctccctga
   7200
   aagggcaata tttcaaatta aacagtacaa ttcagttagc tgtggagtct ctgagtggca
   7260
   gctggaagaa gcacaagtat tctgggcaaa aaaggagcag agtcttgccc tgagtattct
   7320
   caagcaaatg atcaagaagt tggatgccag ctgtgcagcg aacaatccca gcctaaaact
   7380
   tacatacaca gaatgtctga gggtttgtgg caactggtta gcagaaacgt gcttagaaaa
   7440
   tcctgcggtc atcatgcaga cctatctaga aaaggcagta gaagttgctg gaaattatga
   7500
   tggagaaagt agtgatgagc taagaaatgg aaaaatgaag gcatttctct cattagcccg
   7560
   gttttcagat actcaatacc aaagaattga aaactacatg aaatcatcgg aatttgaaaa
   7620
   caagcaagct ctcctgaaaa gagccaaaga ggaagtaggt ctccttaggg aacataaaat
   7680
   tcagacaaac agatacacag taaaggttca gcgagagctg gagttggatg aattagccct
   7740
   gcgtgcactg aaagaggatc gtaaacgctt cttatgtaaa gcagttgaaa attatatcaa
   7800
   ctgcttatta agtggagaag aacatgatat gtgggtattc cgactttgtt ccctctggct
   7860
   tgaaaattct ggagtttctg aagtcaatgg catgatgaag agagacggaa tgaagattcc
   7920
   aacatataaa tttttgcctc ttatgtacca attggctgct agaatgggga ccaagatgat
   7980
   gggaggccta ggatttcatg aagtcctcaa taatctaatc tctagaattt caatggatca
   8040
   cccccatcac actttgttta ttatactggc cttagcaaat gcaaacagag atgaatttct
   8100
   gactaaacca gaggtagcca gaagaagcag aataactaaa aatgtgccta aacaaagctc
   8160
   tcagcttgat gaggatcgaa cagaggctgc aaatagaata atatgtacta tcagaagtag
   8220
   gagacctcag atggtcagaa gtgttgaggc actttgtgat gcttatatta tattagcaaa
   8280
   cttagatgcc actcagtgga agactcagag aaaaggcata aatattccag cagaccagcc
   8340
   aattactaaa cttaagaatt tagaagatgt tgttgtccct actatggaaa ttaaggtgga
   8400
   ccacacagga gaatatggaa atctggtgac tatacagtca tttaaagcag aatttcgctt
   8460
   agcaggaggt gtaaatttac caaaaataat agattgtgta ggttccgatg gcaaggagag
   8520
   gagacagctt gttaagggcc gtgatgacct gagacaagat gctgtcatgc aacaggtctt
   8580
   ccagatgtgt aatacattac tgcagagaaa cacggaaact aggaagagga aattaactat
   8640
   ctgtacttat aaggtggttc ccctctctca gcgaagtggt gttcttgaat ggtgcacagg
   8700
   aactgtcccc attggtgaat ttcttgttaa caatgaagat ggtgctcata aaagatacag
   8760
   gccaaatgat ttcagtgcct ttcagtgcca aaagaaaatg atggaggtgc aaaaaaagtc
   8820
   ttttgaagag aaatatgaag tcttcatgga tgtttgccaa aattttcaac cagttttccg
   8880
   ttacttctgc atggaaaaat tcttggatcc agctatttgg tttgagaagc gattggctta
   8940
   tacgcgcagt gtagctactt cttctattgt tggttacata cttggacttg gtgatagaca
   9000
   tgtacagaat atcttgataa atgagcagtc agcagaactt gtacatatag atctaggtgt
   9060
   tgcttttgaa cagggcaaaa tccttcctac tcctgagaca gttcctttta gactcaccag
   9120
   agatattgtg gatggcatgg gcattacggg tgttgaaggt gtcttcagaa gatgctgtga
   9180
   gaaaaccatg gaagtgatga gaaactctca ggaaactctg ttaaccattg tagaggtcct
   9240
   tctatatgat ccactctttg actggaccat gaatcctttg aaagctttgt atttacagca
   9300
   gaggccggaa gatgaaactg agcttcaccc tactctgaat gcagatgacc aagaatgcaa
   9360
   acgaaatctc agtgatattg accagagttt caacaaagta gctgaacgtg tcttaatgag
   9420
   actacaagag aaactgaaag gagtggaaga aggcactgtg ctcagtgttg gtggacaagt
   9480
   gaatttgctc atacagcagg ccatagaccc caaaaatctc agccgacttt tcccaggatg
   9540
   gaaagcttgg gtgtgatctt cagtatatga attacccttt cattcagcct ttagaaatta
   9600
   tattttagcc tttattttta acctgccaac atactttaag tagggattaa tatttaagtg
   9660
   aactattgtg ggtttttttg aatgttggtt ttaatacttg atttaatcac cactcaaaaa
   9720
   tgttttgatg gtcttaagga acatctctgc tttcactctt tagaaataat ggtcattcgg
   9780
   gctgggcgca gcggctcacg cctgtaatcc cagcactttg ggaggccgag gtgagcggat
   9840
   cacaaggtca ggagttcgag accagcctgg ccaagagacc agcctggcca gtatggtgaa
   9900
   accctgtctc tactaaaaat acaaaaatta gccgagcatg gtggcgggca cctgtaatcc
   9960
   cagctactcg agaggctgag gcaggagaat ctcttgaacc tgggaggtga aggttgctgt
   10020
   gggccaaaat catgccattg cactccagcc tgggtgacaa gagcgaaact ccatctcaaa
   10080
   aaaaaaaaaa aaaaaacaga aacgtatttg gatttttcct agtaagatca ctcagtgtta
   10140
   ctaaataatg aagttgttat ggagaacaaa tttcaaagac acagttagtg tagttactat
   10200
   ttttttaagt gtgtattaaa acttctcatt ctattctctt tatcttttaa gcccttctgt
   10260
   actgtccatg tatgttatct ttctgtgata acttcataga ttgccttcta gttcatgaat
   10320
   tctcttgtca gatgtatata atctctttta ccctatccat tgggcttctt ctttcagaaa
   10380
   ttgtttttca tttctaatta tgcatcattt ttcagatctc tgtttcttga tgtcattttt
   10440
   aatgtttttt taatgttttt tatgtcacta attattttaa atgtctgtac ttgatagaca
   10500
   ctgtaatagt tctattaaat ttagttcctg ctgtttatat ctgttgattt ttgtatttga
   10560
   taggctgttc atccagtttt gtctttttga aaagtgagtt tattttcagc aaggctttat
   10620
   ctatgggaat cttgagtgtc tgtttatgtc atattcccag ggctgttgct gcacacaagc
   10680
   ccattcttat tttaatttct tggctttagg gtttccatac ctgaagtgta gcataaatac
   10740
   tgataggaga tttcccaggc caaggcaaac acacttcctc ctcatctcct tgtgctagtg
   10800
   ggcagaatat ttgattgatg cctttttcac tgagagtata agcttccatg tgtcccacct
   10860
   ttatggcagg ggtggaagga ggtacattta attcccactg cctgcctttg gcaagccctg
   10920
   ggttctttgc tccccatata gatgtctaag ctaaaagccg tgggttaatg agactggcaa
   10980
   attgttccag gacagctaca gcatcagctc acatattcac ctctctggtt tttcattccc
   11040
   ctcatttttt tctgagacag agtcttgctc tgtcacccag gctggagtgc agtggcatga
   11100
   tctcagctca ctgaaacctc tgcctcctgg gttcaagcaa ttctcctgcc tcagcctccc
   11160
   gagtagctgg gactacaggc gtgtgccaac acgcccggct aattttttgt atttttatta
   11220
   gagacggagt ttcaccgtgt tagccaggat ggtctcgatc gcttgacctc gtgatccacc
   11280
   ctcctcggcc tcccaaagtg ctgggattac aggtgtgagc caccgcgccc ggcctcattc
   11340
   ccctcatttt tgaccgtaag gatttcccct ttcttgtaag ttctgctatg tatttaaaag
   11400
   aatgttttct acattttatc cagcatttct ctgtgttctg ttggaaggga agggcttagg
   11460
   tatctagttt gatacatagg tagaagtgga acatttctct gtcccccagc tgtcatcata
   11520
   taagataaac atcagataaa aagccacctg aaagtaaaac tactgactcg tgtattagtg
   11580
   agtataatct cttctccatc cttaggaaaa tgttcatccc agctgcggag attaacaaat
   11640
   gggtgattga gctttctcct cgtatttgga ccttgaaggt tatataaatt tttttcttat
   11700
   gaagagttgg catttctttt tattgccaat ggcaggcact cattcatatt tgatctcctc
   11760
   accttcccct cccctaaaac caatctccag aactttttgg actataaatt tcttggtttg
   11820
   acttctggag aactgttcag aatattactt tgcatttcaa attacaaact taccttggtg
   11880
   tatctttttc ttacaagctg cctaaatgaa tatttggtat atattggtag ttttattact
   11940
   atagtaaatc aaggaaatgc agtaaactta aaatgtcttt aagaaagccc tgaaatcttc
   12000
   atgggtgaaa ttagaaatta tcaactagat aatagtatag ataaatgaat ttgtagctaa
   12060
   ttcttgctag ttgttgcatc cagagagctt tgaataacat cattaatcta ctctttagcc
   12120
   ttgcatggta tgctatgagg ctcctgttct gttcaagtat tctaatcaat ggctttgaaa
   12180
   agtttatcaa atttacatac agatcacaag cctaggagaa ataactaatt cacagatgac
   12240
   agaattaaga ttataaaaga tttttttttt gtaattttag tagagacagg gttgccattg
   12300
   tattccagcc ttggcgacag agcaagactc tgcctcaaaa aaaaaaaaaa aaaggttttg
   12360
   gcaagctgga actctttctg caaatgacta agatagaaaa ctgccaagga caaatgagga
   12420
   gtagttagat tttgaaaata ttaatcatag aatagttgtt gtatgctaag tcactgaccc
   12480
   atattatgta cagcatttct gatctttact ttgcaagatt agtgatacta tcccaataca
   12540
   ctgctggaga aatcagaatt tggagaaata agttgtccaa ggcaagaaga tagtaaatta
   12600
   taagtacaag tgtaatatgg acagtatcta acttgaaaag atttcaggcg aaaagaatct
   12660
   ggggtttgcc agtcagttgc tcaaaaggtc aatgaaaacc aaatagtgaa gctatcagag
   12720
   aagctaataa attatagact gcttgaacag ttgtgtccag attaagggag ataatagctt
   12780
   tcccacccta ctttgtgcag gtcatacctc cccaaagtgt ttacctaatc agtaggttca
   12840
   caaactcttg gtcattatag tatatgccta aaatgtatgc acttaggaat gctaaaaatt
   12900
   taaatatggt ctaaagcaaa taaaagcaaa gaggaaaaac tttggacagc gtaaagacta
   12960
   gaatagtctt ttaaaaagaa agccagtata ttggtttgaa atatagagat gtgtcccaat
   13020
   ttcaagtatt ttaattgcac cttaatgaaa ttatctattt tctatagatt ttagtactat
   13080
   tgaatgtatt actttactgt tacctgaatt tattataaag tgtttttgaa taaataattc
   13140
   taaaagc 13147

<210> 6
<211> 2644
<212> PRT
<213> Homo sapiens

<400> 6

Met Gly Glu His Gly Leu Glu Leu Ala Ser Met Ile Pro Ala Leu Arg
1               5                   10                  15

Glu Leu Gly Ser Ala Thr Pro Glu Glu Tyr Asn Thr Val Val Gln Lys
            20                  25                  30

Pro Arg Gln Ile Leu Cys Gln Phe Ile Asp Arg Ile Leu Thr Asp Val
        35                  40                  45

Asn Val Val Ala Val Glu Leu Val Lys Lys Thr Asp Ser Gln Pro Thr
    50                  55                  60

Ser Val Met Leu Leu Asp Phe Ile Gln His Ile Met Lys Ser Ser Pro
65                  70                  75                  80

Leu Met Phe Val Asn Val Ser Gly Ser His Glu Arg Lys Gly Ser Cys
                85                  90                  95

Ile Glu Phe Ser Asn Trp Ile Ile Thr Arg Leu Leu Arg Ile Ala Ala
            100                 105                 110

Thr Pro Ser Cys His Leu Leu His Lys Lys Ile Cys Glu Val Ile Cys
        115                 120                 125

Ser Leu Leu Phe Leu Phe Lys Ser Lys Ser Pro Ala Ile Phe Gly Val
    130                 135                 140

Leu Thr Lys Glu Leu Leu Gln Leu Phe Glu Asp Leu Val Tyr Leu His
145                 150                 155                 160

Arg Arg Asn Val Met Gly His Ala Val Glu Trp Pro Val Val Met Ser
                165                 170                 175

Arg Phe Leu Ser Gln Leu Asp Glu His Met Gly Tyr Leu Gln Ser Ala
            180                 185                 190

Pro Leu Gln Leu Met Ser Met Gln Asn Leu Glu Phe Ile Glu Val Thr
        195                 200                 205

Leu Leu Met Val Leu Thr Arg Ile Ile Ala Ile Val Phe Phe Arg Arg
    210                 215                 220

Gln Glu Leu Leu Leu Trp Gln Ile Gly Cys Val Leu Leu Glu Tyr Gly
225                 230                 235                 240

Ser Pro Lys Ile Lys Ser Leu Ala Ile Ser Phe Leu Thr Glu Leu Phe
                245                 250                 255

Gln Leu Gly Gly Leu Pro Ala Gln Pro Ala Ser Thr Phe Phe Ser Ser
            260                 265                 270

Phe Leu Glu Leu Leu Lys His Leu Val Glu Met Asp Thr Asp Gln Leu
        275                 280                 285

Lys Leu Tyr Glu Glu Pro Leu Ser Lys Leu Ile Lys Thr Leu Phe Pro
    290                 295                 300

Phe Glu Ala Glu Ala Tyr Arg Asn Ile Glu Pro Val Tyr Leu Asn Met
305                 310                 315                 320

Leu Leu Glu Lys Leu Cys Val Met Phe Glu Asp Gly Val Leu Met Arg
                325                 330                 335

Leu Lys Ser Asp Leu Leu Lys Ala Ala Leu Cys His Leu Leu Gln Tyr
            340                 345                 350

Phe Leu Lys Phe Val Pro Ala Gly Tyr Glu Ser Ala Leu Gln Val Arg
        355                 360                 365

Lys Val Tyr Val Arg Asn Ile Cys Lys Ala Leu Leu Asp Val Leu Gly
    370                 375                 380

Ile Glu Val Asp Ala Glu Tyr Leu Leu Gly Pro Leu Tyr Ala Ala Leu
385                 390                 395                 400

Lys Met Glu Ser Met Glu Ile Ile Glu Glu Ile Gln Cys Gln Thr Gln
                405                 410                 415

Gln Glu Asn Leu Ser Ser Asn Ser Asp Gly Ile Ser Pro Lys Arg Arg
            420                 425                 430

Arg Leu Ser Ser Ser Leu Asn Pro Ser Lys Arg Ala Pro Lys Gln Thr
        435                 440                 445

Glu Glu Ile Lys His Val Asp Met Asn Gln Lys Ser Ile Leu Trp Ser
    450                 455                 460

Ala Leu Lys Gln Lys Ala Glu Ser Leu Gln Ile Ser Leu Glu Tyr Ser
465                 470                 475                 480

Gly Leu Lys Asn Pro Val Ile Glu Met Leu Glu Gly Ile Ala Val Val
                485                 490                 495

Leu Gln Leu Thr Ala Leu Cys Thr Val His Cys Ser His Gln Asn Met
            500                 505                 510

Asn Cys Arg Thr Phe Lys Asp Cys Gln His Lys Ser Lys Lys Lys Pro
        515                 520                 525

Ser Val Val Ile Thr Trp Met Ser Leu Asp Phe Tyr Thr Lys Val Leu
    530                 535                 540

Lys Ser Cys Arg Ser Leu Leu Glu Ser Val Gln Lys Leu Asp Leu Glu
545                 550                 555                 560

Ala Thr Ile Asp Lys Val Val Lys Ile Tyr Asp Ala Leu Ile Tyr Met
                565                 570                 575

Gln Val Asn Ser Ser Phe Glu Asp His Ile Leu Glu Asp Leu Cys Gly
            580                 585                 590

Met Leu Ser Leu Pro Trp Ile Tyr Ser His Ser Asp Asp Gly Cys Leu
        595                 600                 605

Lys Leu Thr Thr Phe Ala Ala Asn Leu Leu Thr Leu Ser Cys Arg Ile
    610                 615                 620

Ser Asp Ser Tyr Ser Pro Gln Ala Gln Ser Arg Cys Val Phe Leu Leu
625                 630                 635                 640

Thr Leu Phe Pro Arg Arg Ile Phe Leu Glu Trp Arg Thr Ala Val Tyr
                645                 650                 655

Asn Trp Ala Leu Gln Ser Ser His Glu Val Ile Arg Ala Ser Cys Val
            660                 665                 670

Ser Gly Phe Phe Ile Leu Leu Gln Gln Gln Asn Ser Cys Asn Arg Val
        675                 680                 685

Pro Lys Ile Leu Ile Asp Lys Val Lys Asp Asp Ser Asp Ile Val Lys
    690                 695                 700

Lys Glu Phe Ala Ser Ile Leu Gly Gln Leu Val Cys Thr Leu His Gly
705                 710                 715                 720

Met Phe Tyr Leu Thr Ser Ser Leu Thr Glu Pro Phe Ser Glu His Gly
                725                 730                 735

His Val Asp Leu Phe Cys Arg Asn Leu Lys Ala Thr Ser Gln His Glu
            740                 745                 750

Cys Ser Ser Ser Gln Leu Lys Ala Ser Val Cys Lys Pro Phe Leu Phe
        755                 760                 765

Leu Leu Lys Lys Lys Ile Pro Ser Pro Val Lys Leu Ala Phe Ile Asp
    770                 775                 780

Asn Leu His His Leu Cys Lys His Leu Asp Phe Arg Glu Asp Glu Thr
785                 790                 795                 800

Asp Val Lys Ala Val Leu Gly Thr Leu Leu Asn Leu Met Glu Asp Pro
                805                 810                 815

Asp Lys Asp Val Arg Val Ala Phe Ser Gly Asn Ile Lys His Ile Leu
            820                 825                 830

Glu Ser Leu Asp Ser Glu Asp Gly Phe Ile Lys Glu Leu Phe Val Leu
        835                 840                 845

Arg Met Lys Glu Ala Tyr Thr His Ala Gln Ile Ser Arg Asn Asn Glu
    850                 855                 860

Leu Lys Asp Thr Leu Ile Leu Thr Thr Gly Asp Ile Gly Arg Ala Ala
865                 870                 875                 880

Lys Gly Asp Leu Val Pro Phe Ala Leu Leu His Leu Leu His Cys Leu
                885                 890                 895

Leu Ser Lys Ser Ala Ser Val Ser Gly Ala Ala Tyr Thr Glu Ile Arg
            900                 905                 910

Ala Leu Val Ala Ala Lys Ser Val Lys Leu Gln Ser Phe Phe Ser Gln
        915                 920                 925

Tyr Lys Lys Pro Ile Cys Gln Phe Leu Val Glu Ser Leu His Ser Ser
    930                 935                 940

Gln Met Thr Ala Leu Pro Asn Thr Pro Cys Gln Asn Ala Asp Val Arg
945                 950                 955                 960

Lys Gln Asp Val Ala His Gln Arg Glu Met Ala Leu Asn Thr Leu Ser
                965                 970                 975

Glu Ile Ala Asn Val Phe Asp Phe Pro Asp Leu Asn Arg Phe Leu Thr
            980                 985                 990

Arg Thr Leu Gln Val Leu Leu Pro Asp Leu Ala Ala Lys Ala Ser Pro
        995                 1000                1005

Ala Ala Ser Ala Leu Ile Arg Thr Leu Gly Lys Gln Leu Asn Val
    1010                1015                1020

Asn Arg Arg Glu Ile Leu Ile Asn Asn Phe Lys Tyr Ile Phe Ser
    1025                1030                1035

His Leu Val Cys Ser Cys Ser Lys Asp Glu Leu Glu Arg Ala Leu
    1040                1045                1050

His Tyr Leu Lys Asn Glu Thr Glu Ile Glu Leu Gly Ser Leu Leu
    1055                1060                1065

Arg Gln Asp Phe Gln Gly Leu His Asn Glu Leu Leu Leu Arg Ile
    1070                1075                1080

Gly Glu His Tyr Gln Gln Val Phe Asn Gly Leu Ser Ile Leu Ala
    1085                1090                1095

Ser Phe Ala Ser Ser Asp Asp Pro Tyr Gln Gly Pro Arg Asp Ile
    1100                1105                1110

Ile Ser Pro Glu Leu Met Ala Asp Tyr Leu Gln Pro Lys Leu Leu
    1115                1120                1125

Gly Ile Leu Ala Phe Phe Asn Met Gln Leu Leu Ser Ser Ser Val
    1130                1135                1140

Gly Ile Glu Asp Lys Lys Met Ala Leu Asn Ser Leu Met Ser Leu
    1145                1150                1155

Met Lys Leu Met Gly Pro Lys His Val Ser Ser Val Arg Val Lys
    1160                1165                1170

Met Met Thr Thr Leu Arg Thr Gly Leu Arg Phe Lys Asp Asp Phe
    1175                1180                1185

Pro Glu Leu Cys Cys Arg Ala Trp Asp Cys Phe Val Arg Cys Leu
    1190                1195                1200

Asp His Ala Cys Leu Gly Ser Leu Leu Ser His Val Ile Val Ala
    1205                1210                1215

Leu Leu Pro Leu Ile His Ile Gln Pro Lys Glu Thr Ala Ala Ile
    1220                1225                1230

Phe His Tyr Leu Ile Ile Glu Asn Arg Asp Ala Val Gln Asp Phe
    1235                1240                1245

Leu His Glu Ile Tyr Phe Leu Pro Asp His Pro Glu Leu Lys Lys
    1250                1255                1260

Ile Lys Ala Val Leu Gln Glu Tyr Arg Lys Glu Thr Ser Glu Ser
    1265                1270                1275

Thr Asp Leu Gln Thr Thr Leu Gln Leu Ser Met Lys Ala Ile Gln
    1280                1285                1290

His Glu Asn Val Asp Val Arg Ile His Ala Leu Thr Ser Leu Lys
    1295                1300                1305

Glu Thr Leu Tyr Lys Asn Gln Glu Lys Leu Ile Lys Tyr Ala Thr
    1310                1315                1320

Asp Ser Glu Thr Val Glu Pro Ile Ile Ser Gln Leu Val Thr Val
    1325                1330                1335

Leu Leu Lys Gly Cys Gln Asp Ala Asn Ser Gln Ala Arg Leu Leu
    1340                1345                1350

Cys Gly Glu Cys Leu Gly Glu Leu Gly Ala Ile Asp Pro Gly Arg
    1355                1360                1365

Leu Asp Phe Ser Thr Thr Glu Thr Gln Gly Lys Asp Phe Thr Phe
    1370                1375                1380

Val Thr Gly Val Glu Asp Ser Ser Phe Ala Tyr Gly Leu Leu Met
    1385                1390                1395

Glu Leu Thr Arg Ala Tyr Leu Ala Tyr Ala Asp Asn Ser Arg Ala
    1400                1405                1410

Gln Asp Ser Ala Ala Tyr Ala Ile Gln Glu Leu Leu Ser Ile Tyr
    1415                1420                1425

Asp Cys Arg Glu Met Glu Thr Asn Gly Pro Gly His Gln Leu Trp
    1430                1435                1440

Arg Arg Phe Pro Glu His Val Arg Glu Ile Leu Glu Pro His Leu
    1445                1450                1455

Asn Thr Arg Tyr Lys Ser Ser Gln Lys Ser Thr Asp Trp Ser Gly
    1460                1465                1470

Val Lys Lys Pro Ile Tyr Leu Ser Lys Leu Gly Ser Asn Phe Ala
    1475                1480                1485

Glu Trp Ser Ala Ser Trp Ala Gly Tyr Leu Ile Thr Lys Val Arg
    1490                1495                1500

His Asp Leu Ala Ser Lys Ile Phe Thr Cys Cys Ser Ile Met Met
    1505                1510                1515

Lys His Asp Phe Lys Val Thr Ile Tyr Leu Leu Pro His Ile Leu
    1520                1525                1530

Val Tyr Val Leu Leu Gly Cys Asn Gln Glu Asp Gln Gln Glu Val
    1535                1540                1545

Tyr Ala Glu Ile Met Ala Val Leu Lys His Asp Asp Gln His Thr
    1550                1555                1560

Ile Asn Thr Gln Asp Ile Ala Ser Asp Leu Cys Gln Leu Ser Thr
    1565                1570                1575

Gln Thr Val Phe Ser Met Leu Asp His Leu Thr Gln Trp Ala Arg
    1580                1585                1590

His Lys Phe Gln Ala Leu Lys Ala Glu Lys Cys Pro His Ser Lys
    1595                1600                1605

Ser Asn Arg Asn Lys Val Asp Ser Met Val Ser Thr Val Asp Tyr
    1610                1615                1620

Glu Asp Tyr Gln Ser Val Thr Arg Phe Leu Asp Leu Ile Pro Gln
    1625                1630                1635

Asp Thr Leu Ala Val Ala Ser Phe Arg Ser Lys Ala Tyr Thr Arg
    1640                1645                1650

Ala Val Met His Phe Glu Ser Phe Ile Thr Glu Lys Lys Gln Asn
    1655                1660                1665

Ile Gln Glu His Leu Gly Phe Leu Gln Lys Leu Tyr Ala Ala Met
    1670                1675                1680

His Glu Pro Asp Gly Val Ala Gly Val Ser Ala Ile Arg Lys Ala
    1685                1690                1695

Glu Pro Ser Leu Lys Glu Gln Ile Leu Glu His Glu Ser Leu Gly
    1700                1705                1710

Leu Leu Arg Asp Ala Thr Ala Cys Tyr Asp Arg Ala Ile Gln Leu
    1715                1720                1725

Glu Pro Asp Gln Ile Ile His Tyr His Gly Val Val Lys Ser Met
    1730                1735                1740

Leu Gly Leu Gly Gln Leu Ser Thr Val Ile Thr Gln Val Asn Gly
    1745                1750                1755

Val His Ala Asn Arg Ser Glu Trp Thr Asp Glu Leu Asn Thr Tyr
    1760                1765                1770

Arg Val Glu Ala Ala Trp Lys Leu Ser Gln Trp Asp Leu Val Glu
    1775                1780                1785

Asn Tyr Leu Ala Ala Asp Gly Lys Ser Thr Thr Trp Ser Val Arg
    1790                1795                1800

Leu Gly Gln Leu Leu Leu Ser Ala Lys Lys Arg Asp Ile Thr Ala
    1805                1810                1815

Phe Tyr Asp Ser Leu Lys Leu Val Arg Ala Glu Gln Ile Val Pro
    1820                1825                1830

Leu Ser Ala Ala Ser Phe Glu Arg Gly Ser Tyr Gln Arg Gly Tyr
    1835                1840                1845

Glu Tyr Ile Val Arg Leu His Met Leu Cys Glu Leu Glu His Ser
    1850                1855                1860

Ile Lys Pro Leu Phe Gln His Ser Pro Gly Asp Ser Ser Gln Glu
    1865                1870                1875

Asp Ser Leu Asn Trp Val Ala Arg Leu Glu Met Thr Gln Asn Ser
    1880                1885                1890

Tyr Arg Ala Lys Glu Pro Ile Leu Ala Leu Arg Arg Ala Leu Leu
    1895                1900                1905

Ser Leu Asn Lys Arg Pro Asp Tyr Asn Glu Met Val Gly Glu Cys
    1910                1915                1920

Trp Leu Gln Ser Ala Arg Val Ala Arg Lys Ala Gly His His Gln
    1925                1930                1935

Thr Ala Tyr Asn Ala Leu Leu Asn Ala Gly Glu Ser Arg Leu Ala
    1940                1945                1950

Glu Leu Tyr Val Glu Arg Ala Lys Trp Leu Trp Ser Lys Gly Asp
    1955                1960                1965

Val His Gln Ala Leu Ile Val Leu Gln Lys Gly Val Glu Leu Cys
    1970                1975                1980

Phe Pro Glu Asn Glu Thr Pro Pro Glu Gly Lys Asn Met Leu Ile
    1985                1990                1995

His Gly Arg Ala Met Leu Leu Val Gly Arg Phe Met Glu Glu Thr
    2000                2005                2010

Ala Asn Phe Glu Ser Asn Ala Ile Met Lys Lys Tyr Lys Asp Val
    2015                2020                2025

Thr Ala Cys Leu Pro Glu Trp Glu Asp Gly His Phe Tyr Leu Ala
    2030                2035                2040

Lys Tyr Tyr Asp Lys Leu Met Pro Met Val Thr Asp Asn Lys Met
    2045                2050                2055

Glu Lys Gln Gly Asp Leu Ile Arg Tyr Ile Val Leu His Phe Gly
    2060                2065                2070

Arg Ser Leu Gln Tyr Gly Asn Gln Phe Ile Tyr Gln Ser Met Pro
    2075                2080                2085

Arg Met Leu Thr Leu Trp Leu Asp Tyr Gly Thr Lys Ala Tyr Glu
    2090                2095                2100

Trp Glu Lys Ala Gly Arg Ser Asp Arg Val Gln Met Arg Asn Asp
    2105                2110                2115

Leu Gly Lys Ile Asn Lys Val Ile Thr Glu His Thr Asn Tyr Leu
    2120                2125                2130

Ala Pro Tyr Gln Phe Leu Thr Ala Phe Ser Gln Leu Ile Ser Arg
    2135                2140                2145

Ile Cys His Ser His Asp Glu Val Phe Val Val Leu Met Glu Ile
    2150                2155                2160

Ile Ala Lys Val Phe Leu Ala Tyr Pro Gln Gln Ala Met Trp Met
    2165                2170                2175

Met Thr Ala Val Ser Lys Ser Ser Tyr Pro Met Arg Val Asn Arg
    2180                2185                2190

Cys Lys Glu Ile Leu Asn Lys Ala Ile His Met Lys Lys Ser Leu
    2195                2200                2205

Glu Lys Phe Val Gly Asp Ala Thr Arg Leu Thr Asp Lys Leu Leu
    2210                2215                2220

Glu Leu Cys Asn Lys Pro Val Asp Gly Ser Ser Ser Thr Leu Ser
    2225                2230                2235

Met Ser Thr His Phe Lys Met Leu Lys Lys Leu Val Glu Glu Ala
    2240                2245                2250

Thr Phe Ser Glu Ile Leu Ile Pro Leu Gln Ser Val Met Ile Pro
    2255                2260                2265

Thr Leu Pro Ser Ile Leu Gly Thr His Ala Asn His Ala Ser His
    2270                2275                2280

Glu Pro Phe Pro Gly His Trp Ala Tyr Ile Ala Gly Phe Asp Asp
    2285                2290                2295

Met Val Glu Ile Leu Ala Ser Leu Gln Lys Pro Lys Lys Ile Ser
    2300                2305                2310

Leu Lys Gly Ser Asp Gly Lys Phe Tyr Ile Met Met Cys Lys Pro
    2315                2320                2325

Lys Asp Asp Leu Arg Lys Asp Cys Arg Leu Met Glu Phe Asn Ser
    2330                2335                2340

Leu Ile Asn Lys Cys Leu Arg Lys Asp Ala Glu Ser Arg Arg Arg
    2345                2350                2355

Glu Leu His Ile Arg Thr Tyr Ala Val Ile Pro Leu Asn Asp Glu
    2360                2365                2370

Cys Gly Ile Ile Glu Trp Val Asn Asn Thr Ala Gly Leu Arg Pro
    2375                2380                2385

Ile Leu Thr Lys Leu Tyr Lys Glu Lys Gly Val Tyr Met Thr Gly
    2390                2395                2400

Lys Glu Leu Arg Gln Cys Met Leu Pro Lys Ser Ala Ala Leu Ser
    2405                2410                2415

Glu Lys Leu Lys Val Phe Arg Glu Phe Leu Leu Pro Arg His Pro
    2420                2425                2430

Pro Ile Phe His Glu Trp Phe Leu Arg Thr Phe Pro Asp Pro Thr
    2435                2440                2445

Ser Trp Tyr Ser Ser Arg Ser Ala Tyr Cys Arg Ser Thr Ala Val
    2450                2455                2460

Met Ser Met Val Gly Tyr Ile Leu Gly Leu Gly Asp Arg His Gly
    2465                2470                2475

Glu Asn Ile Leu Phe Asp Ser Leu Thr Gly Glu Cys Val His Val
    2480                2485                2490

Asp Phe Asn Cys Leu Phe Asn Lys Gly Glu Thr Phe Glu Val Pro
    2495                2500                2505

Glu Ile Val Pro Phe Arg Leu Thr His Asn Met Val Asn Gly Met
    2510                2515                2520

Gly Pro Met Gly Thr Glu Gly Leu Phe Arg Arg Ala Cys Glu Val
    2525                2530                2535

Thr Met Arg Leu Met Arg Asp Gln Arg Glu Pro Leu Met Ser Val
    2540                2545                2550

Leu Lys Thr Phe Leu His Asp Pro Leu Val Glu Trp Ser Lys Pro
    2555                2560                2565

Val Lys Gly His Ser Lys Ala Pro Leu Asn Glu Thr Gly Glu Val
    2570                2575                2580

Val Asn Glu Lys Ala Lys Thr His Val Leu Asp Ile Glu Gln Arg
    2585                2590                2595

Leu Gln Gly Val Ile Lys Thr Arg Asn Arg Val Thr Gly Leu Pro
    2600                2605                2610

Leu Ser Ile Glu Gly His Val His Tyr Leu Ile Gln Glu Ala Thr
    2615                2620                2625

Asp Glu Asn Leu Leu Cys Gln Met Tyr Leu Gly Trp Thr Pro Tyr
    2630                2635                2640

Met

<210> 7
<211> 3056
<212> PRT
<213> Homo sapiens

<400> 7

Met Ser Leu Val Leu Asn Asp Leu Leu Ile Cys Cys Arg Gln Leu Glu

1               5                   10                  15

His Asp Arg Ala Thr Glu Arg Lys Lys Glu Val Glu Lys Phe Lys Arg
            20                  25                  30

Leu Ile Arg Asp Pro Glu Thr Ile Lys His Leu Asp Arg His Ser Asp
        35                  40                  45

Ser Lys Gln Gly Lys Tyr Leu Asn Trp Asp Ala Val Phe Arg Phe Leu
    50                  55                  60

Gln Lys Tyr Ile Gln Lys Glu Thr Glu Cys Leu Arg Ile Ala Lys Pro
65                  70                  75                  80

Asn Val Ser Ala Ser Thr Gln Ala Ser Arg Gln Lys Lys Met Gln Glu
                85                  90                  95

Ile Ser Ser Leu Val Lys Tyr Phe Ile Lys Cys Ala Asn Arg Arg Ala
            100                 105                 110

Pro Arg Leu Lys Cys Gln Glu Leu Leu Asn Tyr Ile Met Asp Thr Val
        115                 120                 125

Lys Asp Ser Ser Asn Gly Ala Ile Tyr Gly Ala Asp Cys Ser Asn Ile
    130                 135                 140

Leu Leu Lys Asp Ile Leu Ser Val Arg Lys Tyr Trp Cys Glu Ile Ser

145                 150                 155                 160

Gln Gln Gln Trp Leu Glu Leu Phe Ser Val Tyr Phe Arg Leu Tyr Leu
                165                 170                 175

Lys Pro Ser Gln Asp Val His Arg Val Leu Val Ala Arg Ile Ile His
            180                 185                 190

Ala Val Thr Lys Gly Cys Cys Ser Gln Thr Asp Gly Leu Asn Ser Lys
        195                 200                 205

Phe Leu Asp Phe Phe Ser Lys Ala Ile Gln Cys Ala Arg Gln Glu Lys
    210                 215                 220

Ser Ser Ser Gly Leu Asn His Ile Leu Ala Ala Leu Thr Ile Phe Leu
225                 230                 235                 240

Lys Thr Leu Ala Val Asn Phe Arg Ile Arg Val Cys Glu Leu Gly Asp
                245                 250                 255

Glu Ile Leu Pro Thr Leu Leu Tyr Ile Trp Thr Gln His Arg Leu Asn
            260                 265                 270

Asp Ser Leu Lys Glu Val Ile Ile Glu Leu Phe Gln Leu Gln Ile Tyr
        275                 280                 285

Ile His His Pro Lys Gly Ala Lys Thr Gln Glu Lys Gly Ala Tyr Glu

290                 295                 300

Ser Thr Lys Trp Arg Ser Ile Leu Tyr Asn Leu Tyr Asp Leu Leu Val
305                 310                 315                 320

Asn Glu Ile Ser His Ile Gly Ser Arg Gly Lys Tyr Ser Ser Gly Phe
                325                 330                 335

Arg Asn Ile Ala Val Lys Glu Asn Leu Ile Glu Leu Met Ala Asp Ile
            340                 345                 350

Cys His Gln Val Phe Asn Glu Asp Thr Arg Ser Leu Glu Ile Ser Gln
        355                 360                 365

Ser Tyr Thr Thr Thr Gln Arg Glu Ser Ser Asp Tyr Ser Val Pro Cys
    370                 375                 380

Lys Arg Lys Lys Ile Glu Leu Gly Trp Glu Val Ile Lys Asp His Leu
385                 390                 395                 400

Gln Lys Ser Gln Asn Asp Phe Asp Leu Val Pro Trp Leu Gln Ile Ala
                405                 410                 415

Thr Gln Leu Ile Ser Lys Tyr Pro Ala Ser Leu Pro Asn Cys Glu Leu
            420                 425                 430

Ser Pro Leu Leu Met Ile Leu Ser Gln Leu Leu Pro Gln Gln Arg His

        435                 440                 445

Gly Glu Arg Thr Pro Tyr Val Leu Arg Cys Leu Thr Glu Val Ala Leu
    450                 455                 460

Cys Gln Asp Lys Arg Ser Asn Leu Glu Ser Ser Gln Lys Ser Asp Leu
465                 470                 475                 480

Leu Lys Leu Trp Asn Lys Ile Trp Cys Ile Thr Phe Arg Gly Ile Ser
                485                 490                 495

Ser Glu Gln Ile Gln Ala Glu Asn Phe Gly Leu Leu Gly Ala Ile Ile
            500                 505                 510

Gln Gly Ser Leu Val Glu Val Asp Arg Glu Phe Trp Lys Leu Phe Thr
        515                 520                 525

Gly Ser Ala Cys Arg Pro Ser Cys Pro Ala Val Cys Cys Leu Thr Leu
    530                 535                 540

Ala Leu Thr Thr Ser Ile Val Pro Gly Thr Val Lys Met Gly Ile Glu
545                 550                 555                 560

Gln Asn Met Cys Glu Val Asn Arg Ser Phe Ser Leu Lys Glu Ser Ile
                565                 570                 575

Met Lys Trp Leu Leu Phe Tyr Gln Leu Glu Gly Asp Leu Glu Asn Ser

            580                 585                 590

Thr Glu Val Pro Pro Ile Leu His Ser Asn Phe Pro His Leu Val Leu
        595                 600                 605

Glu Lys Ile Leu Val Ser Leu Thr Met Lys Asn Cys Lys Ala Ala Met
    610                 615                 620

Asn Phe Phe Gln Ser Val Pro Glu Cys Glu His His Gln Lys Asp Lys
625                 630                 635                 640

Glu Glu Leu Ser Phe Ser Glu Val Glu Glu Leu Phe Leu Gln Thr Thr
                645                 650                 655

Phe Asp Lys Met Asp Phe Leu Thr Ile Val Arg Glu Cys Gly Ile Glu
            660                 665                 670

Lys His Gln Ser Ser Ile Gly Phe Ser Val His Gln Asn Leu Lys Glu
        675                 680                 685

Ser Leu Asp Arg Cys Leu Leu Gly Leu Ser Glu Gln Leu Leu Asn Asn
    690                 695                 700

Tyr Ser Ser Glu Ile Thr Asn Ser Glu Thr Leu Val Arg Cys Ser Arg
705                 710                 715                 720

Leu Leu Val Gly Val Leu Gly Cys Tyr Cys Tyr Met Gly Val Ile Ala

                725                 730                 735

Glu Glu Glu Ala Tyr Lys Ser Glu Leu Phe Gln Lys Ala Lys Ser Leu
            740                 745                 750

Met Gln Cys Ala Gly Glu Ser Ile Thr Leu Phe Lys Asn Lys Thr Asn
        755                 760                 765

Glu Glu Phe Arg Ile Gly Ser Leu Arg Asn Met Met Gln Leu Cys Thr
    770                 775                 780

Arg Cys Leu Ser Asn Cys Thr Lys Lys Ser Pro Asn Lys Ile Ala Ser
785                 790                 795                 800

Gly Phe Phe Leu Arg Leu Leu Thr Ser Lys Leu Met Asn Asp Ile Ala
                805                 810                 815

Asp Ile Cys Lys Ser Leu Ala Ser Phe Ile Lys Lys Pro Phe Asp Arg
            820                 825                 830

Gly Glu Val Glu Ser Met Glu Asp Asp Thr Asn Gly Asn Leu Met Glu
        835                 840                 845

Val Glu Asp Gln Ser Ser Met Asn Leu Phe Asn Asp Tyr Pro Asp Ser
    850                 855                 860

Ser Val Ser Asp Ala Asn Glu Pro Gly Glu Ser Gln Ser Thr Ile Gly

865                 870                 875                 880

Ala Ile Asn Pro Leu Ala Glu Glu Tyr Leu Ser Lys Gln Asp Leu Leu
                885                 890                 895

Phe Leu Asp Met Leu Lys Phe Leu Cys Leu Cys Val Thr Thr Ala Gln
            900                 905                 910

Thr Asn Thr Val Ser Phe Arg Ala Ala Asp Ile Arg Arg Lys Leu Leu
        915                 920                 925

Met Leu Ile Asp Ser Ser Thr Leu Glu Pro Thr Lys Ser Leu His Leu
    930                 935                 940

His Met Tyr Leu Met Leu Leu Lys Glu Leu Pro Gly Glu Glu Tyr Pro
945                 950                 955                 960

Leu Pro Met Glu Asp Val Leu Glu Leu Leu Lys Pro Leu Ser Asn Val
                965                 970                 975

Cys Ser Leu Tyr Arg Arg Asp Gln Asp Val Cys Lys Thr Ile Leu Asn
            980                 985                 990

His Val Leu His Val Val Lys Asn Leu Gly Gln Ser Asn Met Asp Ser
        995                 1000                1005

Glu Asn Thr Arg Asp Ala Gln Gly Gln Phe Leu Thr Val Ile Gly

1010                1015                1020

Ala Phe Trp His Leu Thr Lys Glu Arg Lys Tyr Ile Phe Ser Val
    1025                1030                1035

Arg Met Ala Leu Val Asn Cys Leu Lys Thr Leu Leu Glu Ala Asp
    1040                1045                1050

Pro Tyr Ser Lys Trp Ala Ile Leu Asn Val Met Gly Lys Asp Phe
    1055                1060                1065

Pro Val Asn Glu Val Phe Thr Gln Phe Leu Ala Asp Asn His His
    1070                1075                1080

Gln Val Arg Met Leu Ala Ala Glu Ser Ile Asn Arg Leu Phe Gln
    1085                1090                1095

Asp Thr Lys Gly Asp Ser Ser Arg Leu Leu Lys Ala Leu Pro Leu
    1100                1105                1110

Lys Leu Gln Gln Thr Ala Phe Glu Asn Ala Tyr Leu Lys Ala Gln
    1115                1120                1125

Glu Gly Met Arg Glu Met Ser His Ser Ala Glu Asn Pro Glu Thr
    1130                1135                1140

Leu Asp Glu Ile Tyr Asn Arg Lys Ser Val Leu Leu Thr Leu Ile

1145                1150                1155

Ala Val  Val Leu Ser Cys Ser  Pro Ile Cys Glu Lys  Gln Ala Leu
    1160                1165                1170

Phe Ala  Leu Cys Lys Ser Val  Lys Glu Asn Gly Leu  Glu Pro His
    1175                1180                1185

Leu Val  Lys Lys Val Leu Glu  Lys Val Ser Glu Thr  Phe Gly Tyr
    1190                1195                1200

Arg Arg  Leu Glu Asp Phe Met  Ala Ser His Leu Asp  Tyr Leu Val
    1205                1210                1215

Leu Glu  Trp Leu Asn Leu Gln  Asp Thr Glu Tyr Asn  Leu Ser Ser
    1220                1225                1230

Phe Pro  Phe Ile Leu Leu Asn  Tyr Thr Asn Ile Glu  Asp Phe Tyr
    1235                1240                1245

Arg Ser  Cys Tyr Lys Val Leu  Ile Pro His Leu Val  Ile Arg Ser
    1250                1255                1260

His Phe  Asp Glu Val Lys Ser  Ile Ala Asn Gln Ile  Gln Glu Asp
    1265                1270                1275

Trp Lys  Ser Leu Leu Thr Asp  Cys Phe Pro Lys Ile  Leu Val Asn

1280                1285                1290

Ile Leu Pro Tyr Phe Ala Tyr Glu Gly Thr Arg Asp Ser Gly Met
1295                1300                1305

Ala Gln Gln Arg Glu Thr Ala Thr Lys Val Tyr Asp Met Leu Lys
1310                1315                1320

Ser Glu Asn Leu Leu Gly Lys Gln Ile Asp His Leu Phe Ile Ser
1325                1330                1335

Asn Leu Pro Glu Ile Val Val Glu Leu Leu Met Thr Leu His Glu
1340                1345                1350

Pro Ala Asn Ser Ser Ala Ser Gln Ser Thr Asp Leu Cys Asp Phe
1355                1360                1365

Ser Gly Asp Leu Asp Pro Ala Pro Asn Pro Pro His Phe Pro Ser
1370                1375                1380

His Val Ile Lys Ala Thr Phe Ala Tyr Ile Ser Asn Cys His Lys
1385                1390                1395

Thr Lys Leu Lys Ser Ile Leu Glu Ile Leu Ser Lys Ser Pro Asp
1400                1405                1410

Ser Tyr Gln Lys Ile Leu Leu Ala Ile Cys Glu Gln Ala Ala Glu

1415                1420                1425

Thr Asn Asn Val Tyr Lys Lys His Arg Ile Leu Lys Ile Tyr His
    1430                1435                1440

Leu Phe Val Ser Leu Leu Leu Lys Asp Ile Lys Ser Gly Leu Gly
    1445                1450                1455

Gly Ala Trp Ala Phe Val Leu Arg Asp Val Ile Tyr Thr Leu Ile
    1460                1465                1470

His Tyr Ile Asn Gln Arg Pro Ser Cys Ile Met Asp Val Ser Leu
    1475                1480                1485

Arg Ser Phe Ser Leu Cys Cys Asp Leu Leu Ser Gln Val Cys Gln
    1490                1495                1500

Thr Ala Val Thr Tyr Cys Lys Asp Ala Leu Glu Asn His Leu His
    1505                1510                1515

Val Ile Val Gly Thr Leu Ile Pro Leu Val Tyr Glu Gln Val Glu
    1520                1525                1530

Val Gln Lys Gln Val Leu Asp Leu Leu Lys Tyr Leu Val Ile Asp
    1535                1540                1545

Asn Lys Asp Asn Glu Asn Leu Tyr Ile Thr Ile Lys Leu Leu Asp

1550                1555                1560

Pro Phe  Pro Asp His Val Val  Phe Lys Asp Leu Arg  Ile Thr Gln
    1565                1570                1575

Gln Lys  Ile Lys Tyr Ser Arg  Gly Pro Phe Ser Leu  Leu Glu Glu
    1580                1585                1590

Ile Asn  His Phe Leu Ser Val  Ser Val Tyr Asp Ala  Leu Pro Leu
    1595                1600                1605

Thr Arg  Leu Glu Gly Leu Lys  Asp Leu Arg Arg Gln  Leu Glu Leu
    1610                1615                1620

His Lys  Asp Gln Met Val Asp  Ile Met Arg Ala Ser  Gln Asp Asn
    1625                1630                1635

Pro Gln  Asp Gly Ile Met Val  Lys Leu Val Val Asn  Leu Leu Gln
    1640                1645                1650

Leu Ser  Lys Met Ala Ile Asn  His Thr Gly Glu Lys  Glu Val Leu
    1655                1660                1665

Glu Ala  Val Gly Ser Cys Leu  Gly Glu Val Gly Pro  Ile Asp Phe
    1670                1675                1680

Ser Thr  Ile Ala Ile Gln His  Ser Lys Asp Ala Ser  Tyr Thr Lys

1685                1690                1695

Ala Leu Lys Leu Phe Glu Asp Lys Glu Leu Gln Trp Thr Phe Ile
    1700                1705                1710

Met Leu Thr Tyr Leu Asn Asn Thr Leu Val Glu Asp Cys Val Lys
    1715                1720                1725

Val Arg Ser Ala Ala Val Thr Cys Leu Lys Asn Ile Leu Ala Thr
    1730                1735                1740

Lys Thr Gly His Ser Phe Trp Glu Ile Tyr Lys Met Thr Thr Asp
    1745                1750                1755

Pro Met Leu Ala Tyr Leu Gln Pro Phe Arg Thr Ser Arg Lys Lys
    1760                1765                1770

Phe Leu Glu Val Pro Arg Phe Asp Lys Glu Asn Pro Phe Glu Gly
    1775                1780                1785

Leu Asp Asp Ile Asn Leu Trp Ile Pro Leu Ser Glu Asn His Asp
    1790                1795                1800

Ile Trp Ile Lys Thr Leu Thr Cys Ala Phe Leu Asp Ser Gly Gly
    1805                1810                1815

Thr Lys Cys Glu Ile Leu Gln Leu Leu Lys Pro Met Cys Glu Val

1820                1825                1830

Lys Thr  Asp Phe Cys Gln Thr  Val Leu Pro Tyr Leu  Ile His Asp
    1835                1840                1845

Ile Leu  Leu Gln Asp Thr Asn  Glu Ser Trp Arg Asn  Leu Leu Ser
    1850                1855                1860

Thr His  Val Gln Gly Phe Phe  Thr Ser Cys Leu Arg  His Phe Ser
    1865                1870                1875

Gln Thr  Ser Arg Ser Thr Thr  Pro Ala Asn Leu Asp  Ser Glu Ser
    1880                1885                1890

Glu His  Phe Phe Arg Cys Cys  Leu Asp Lys Lys Ser  Gln Arg Thr
    1895                1900                1905

Met Leu  Ala Val Val Asp Tyr  Met Arg Arg Gln Lys  Arg Pro Ser
    1910                1915                1920

Ser Gly  Thr Ile Phe Asn Asp  Ala Phe Trp Leu Asp  Leu Asn Tyr
    1925                1930                1935

Leu Glu  Val Ala Lys Val Ala  Gln Ser Cys Ala Ala  His Phe Thr
    1940                1945                1950

Ala Leu  Leu Tyr Ala Glu Ile  Tyr Ala Asp Lys Lys  Ser Met Asp

1955                    1960                    1965

Asp Gln  Glu Lys Arg Ser Leu  Ala Phe Glu Glu Gly  Ser Gln Ser
    1970                    1975                    1980

Thr Thr  Ile Ser Ser Leu Ser  Glu Lys Ser Lys Glu  Glu Thr Gly
    1985                    1990                    1995

Ile Ser  Leu Gln Asp Leu Leu  Leu Glu Ile Tyr Arg  Ser Ile Gly
    2000                    2005                    2010

Glu Pro  Asp Ser Leu Tyr Gly  Cys Gly Gly Gly Lys  Met Leu Gln
    2015                    2020                    2025

Pro Ile  Thr Arg Leu Arg Thr  Tyr Glu His Glu Ala  Met Trp Gly
    2030                    2035                    2040

Lys Ala  Leu Val Thr Tyr Asp  Leu Glu Thr Ala Ile  Pro Ser Ser
    2045                    2050                    2055

Thr Arg  Gln Ala Gly Ile Ile  Gln Ala Leu Gln Asn  Leu Gly Leu
    2060                    2065                    2070

Cys His  Ile Leu Ser Val Tyr  Leu Lys Gly Leu Asp  Tyr Glu Asn
    2075                    2080                    2085

Lys Asp  Trp Cys Pro Glu Leu  Glu Glu Leu His Tyr  Gln Ala Ala

2090                2095                2100

Trp Arg  Asn Met Gln Trp Asp  His Cys Thr Ser Val  Ser Lys Glu
    2105                2110                2115

Val Glu  Gly Thr Ser Tyr His  Glu Ser Leu Tyr Asn  Ala Leu Gln
    2120                2125                2130

Ser Leu  Arg Asp Arg Glu Phe  Ser Thr Phe Tyr Glu  Ser Leu Lys
    2135                2140                2145

Tyr Ala  Arg Val Lys Glu Val  Glu Glu Met Cys Lys  Arg Ser Leu
    2150                2155                2160

Glu Ser  Val Tyr Ser Leu Tyr  Pro Thr Leu Ser Arg  Leu Gln Ala
    2165                2170                2175

Ile Gly  Glu Leu Glu Ser Ile  Gly Glu Leu Phe Ser  Arg Ser Val
    2180                2185                2190

Thr His  Arg Gln Leu Ser Glu  Val Tyr Ile Lys Trp  Gln Lys His
    2195                2200                2205

Ser Gln  Leu Leu Lys Asp Ser  Asp Phe Ser Phe Gln  Glu Pro Ile
    2210                2215                2220

Met Ala  Leu Arg Thr Val Ile  Leu Glu Ile Leu Met  Glu Lys Glu

2225                2230                2235

Met Asp Asn Ser Gln Arg Glu Cys Ile Lys Asp Ile Leu Thr Lys
    2240                2245                2250

His Leu Val Glu Leu Ser Ile Leu Ala Arg Thr Phe Lys Asn Thr
    2255                2260                2265

Gln Leu Pro Glu Arg Ala Ile Phe Gln Ile Lys Gln Tyr Asn Ser
    2270                2275                2280

Val Ser Cys Gly Val Ser Glu Trp Gln Leu Glu Glu Ala Gln Val
    2285                2290                2295

Phe Trp Ala Lys Lys Glu Gln Ser Leu Ala Leu Ser Ile Leu Lys
    2300                2305                2310

Gln Met Ile Lys Lys Leu Asp Ala Ser Cys Ala Ala Asn Asn Pro
    2315                2320                2325

Ser Leu Lys Leu Thr Tyr Thr Glu Cys Leu Arg Val Cys Gly Asn
    2330                2335                2340

Trp Leu Ala Glu Thr Cys Leu Glu Asn Pro Ala Val Ile Met Gln
    2345                2350                2355

Thr Tyr Leu Glu Lys Ala Val Glu Val Ala Gly Asn Tyr Asp Gly

2360                2365                2370

Glu Ser Ser Asp Glu Leu Arg Asn Gly Lys Met Lys Ala Phe Leu
    2375                2380                2385

Ser Leu Ala Arg Phe Ser Asp Thr Gln Tyr Gln Arg Ile Glu Asn
    2390                2395                2400

Tyr Met Lys Ser Ser Glu Phe Glu Asn Lys Gln Ala Leu Leu Lys
    2405                2410                2415

Arg Ala Lys Glu Glu Val Gly Leu Leu Arg Glu His Lys Ile Gln
    2420                2425                2430

Thr Asn Arg Tyr Thr Val Lys Val Gln Arg Glu Leu Glu Leu Asp
    2435                2440                2445

Glu Leu Ala Leu Arg Ala Leu Lys Glu Asp Arg Lys Arg Phe Leu
    2450                2455                2460

Cys Lys Ala Val Glu Asn Tyr Ile Asn Cys Leu Leu Ser Gly Glu
    2465                2470                2475

Glu His Asp Met Trp Val Phe Arg Leu Cys Ser Leu Trp Leu Glu
    2480                2485                2490

Asn Ser Gly Val Ser Glu Val Asn Gly Met Met Lys Arg Asp Gly

2495                2500                2505

Met Lys Ile Pro Thr Tyr Lys Phe Leu Pro Leu Met Tyr Gln Leu
    2510                2515                2520

Ala Ala Arg Met Gly Thr Lys Met Met Gly Gly Leu Gly Phe His
    2525                2530                2535

Glu Val Leu Asn Asn Leu Ile Ser Arg Ile Ser Met Asp His Pro
    2540                2545                2550

His His Thr Leu Phe Ile Ile Leu Ala Leu Ala Asn Ala Asn Arg
    2555                2560                2565

Asp Glu Phe Leu Thr Lys Pro Glu Val Ala Arg Arg Ser Arg Ile
    2570                2575                2580

Thr Lys Asn Val Pro Lys Gln Ser Ser Gln Leu Asp Glu Asp Arg
    2585                2590                2595

Thr Glu Ala Ala Asn Arg Ile Ile Cys Thr Ile Arg Ser Arg Arg
    2600                2605                2610

Pro Gln Met Val Arg Ser Val Glu Ala Leu Cys Asp Ala Tyr Ile
    2615                2620                2625

Ile Leu Ala Asn Leu Asp Ala Thr Gln Trp Lys Thr Gln Arg Lys

2630                2635                2640

Gly Ile Asn Ile Pro Ala Asp Gln Pro Ile Thr Lys Leu Lys Asn
    2645                2650                2655

Leu Glu Asp Val Val Pro Thr Met Glu Ile Lys Val Asp His
    2660                2665                2670

Thr Gly Glu Tyr Gly Asn Leu Val Thr Ile Gln Ser Phe Lys Ala
    2675                2680                2685

Glu Phe Arg Leu Ala Gly Gly Val Asn Leu Pro Lys Ile Ile Asp
    2690                2695                2700

Cys Val Gly Ser Asp Gly Lys Glu Arg Arg Gln Leu Val Lys Gly
    2705                2710                2715

Arg Asp Asp Leu Arg Gln Asp Ala Val Met Gln Gln Val Phe Gln
    2720                2725                2730

Met Cys Asn Thr Leu Leu Gln Arg Asn Thr Glu Thr Arg Lys Arg
    2735                2740                2745

Lys Leu Thr Ile Cys Thr Tyr Lys Val Val Pro Leu Ser Gln Arg
    2750                2755                2760

Ser Gly Val Leu Glu Trp Cys Thr Gly Thr Val Pro Ile Gly Glu

2765                2770                2775

Phe Leu Val Asn Asn Glu Asp Gly Ala His Lys Arg Tyr Arg Pro
    2780                2785                2790

Asn Asp Phe Ser Ala Phe Gln Cys Gln Lys Lys Met Met Glu Val
    2795                2800                2805

Gln Lys Lys Ser Phe Glu Glu Lys Tyr Glu Val Phe Met Asp Val
    2810                2815                2820

Cys Gln Asn Phe Gln Pro Val Phe Arg Tyr Phe Cys Met Glu Lys
    2825                2830                2835

Phe Leu Asp Pro Ala Ile Trp Phe Glu Lys Arg Leu Ala Tyr Thr
    2840                2845                2850

Arg Ser Val Ala Thr Ser Ser Ile Val Gly Tyr Ile Leu Gly Leu
    2855                2860                2865

Gly Asp Arg His Val Gln Asn Ile Leu Ile Asn Glu Gln Ser Ala
    2870                2875                2880

Glu Leu Val His Ile Asp Leu Gly Val Ala Phe Glu Gln Gly Lys
    2885                2890                2895

Ile Leu Pro Thr Pro Glu Thr Val Pro Phe Arg Leu Thr Arg Asp

2900                    2905                    2910

Ile Val  Asp Gly Met Gly Ile  Thr Gly Val Glu Gly  Val Phe Arg
    2915                    2920                    2925

Arg Cys  Cys Glu Lys Thr Met  Glu Val Met Arg Asn  Ser Gln Glu
    2930                    2935                    2940

Thr Leu  Leu Thr Ile Val Glu  Val Leu Leu Tyr Asp  Pro Leu Phe
    2945                    2950                    2955

Asp Trp  Thr Met Asn Pro Leu  Lys Ala Leu Tyr Leu  Gln Gln Arg
    2960                    2965                    2970

Pro Glu  Asp Glu Thr Glu Leu  His Pro Thr Leu Asn  Ala Asp Asp
    2975                    2980                    2985

Gln Glu  Cys Lys Arg Asn Leu  Ser Asp Ile Asp Gln  Ser Phe Asn
    2990                    2995                    3000

Lys Val  Ala Glu Arg Val Leu  Met Arg Leu Gln Glu  Lys Leu Lys
    3005                    3010                    3015

Gly Val  Glu Glu Gly Thr Val  Leu Ser Val Gly Gly  Gln Val Asn
    3020                    3025                    3030

Leu Leu  Ile Gln Gln Ala Ile  Asp Pro Lys Asn Leu  Ser Arg Leu

         3035                 3040                 3045

Phe Pro Gly Trp Lys Ala Trp Val
        3050                3055